(19) Europäisches Patentamt
European Patent Office
Office européen des brevets

(11) EP 1 485 410 B1

(12) EUROPEAN PATENT SPECIFICATION

(45) Date of publication and mention of the grant of the patent:
**20.06.2007 Bulletin 2007/25**

(21) Application number: **03744814.9**

(22) Date of filing: **19.03.2003**

(51) Int Cl.:
*C07K 14/47* (2006.01) *C12N 15/12* (2006.01)
*C12N 5/10* (2006.01) *C07K 16/18* (2006.01)
*G01N 33/53* (2006.01) *C12Q 1/68* (2006.01)
*A61K 38/16* (2006.01) *A61K 48/00* (2006.01)
*A01K 67/027* (2006.01)

(86) International application number:
**PCT/EP2003/002857**

(87) International publication number:
**WO 2003/080661 (02.10.2003 Gazette 2003/40)**

(54) **DIAGNOSTIC AND THERAPEUTIC USE OF HUMAN MAGUIN PROTEINS AND NUCLEIC ACIDS FOR NEURODEGENERATIVE DISEASES**

DIAGNOSTISCHE UND THERAPEUTISCHE VERWENDUNG VON HUMANEN MAGUINPROTEINEN UND NUKLEINSÄUREN BEI NEURODEGENERATIVEN KRANKHEITEN

UTILISATION DE PROTEINES MAGUIN HUMAINES ET D'ACIDES NUCLEIQUES DANS LE DIAGNOSTIC ET LE TRAITEMENT DE PATHOLOGIES NEURODEGENERATIVES

(84) Designated Contracting States:
**AT BE BG CH CY CZ DE DK EE ES FI FR GB GR HU IE IT LI LU MC NL PT RO SE SI SK TR**

(30) Priority: **21.03.2002 EP 02006353**
**21.03.2002 US 365815 P**

(43) Date of publication of application:
**15.12.2004 Bulletin 2004/51**

(73) Proprietor: **EVOTEC Neurosciences GmbH**
**22525 Hamburg (DE)**

(72) Inventors:
- **VON DER KAMMER, Heinz**
**22607 Hamburg (DE)**
- **HANES, Jozef**
**84107 Bratislava (SK)**
- **POHLNER, Johannes**
**22175 Hamburg (DE)**

(74) Representative: **von Kreisler Selting Werner Patentanwälte**
**P.O. Box 10 22 41**
**50462 Köln (DE)**

(56) References cited:
- **DATABASE EMBL [Online] 16 January 2002 (2002-01-16) LANIGAN,T.M. AND GUAN, K.L.: "Homo sapiens connector enhancer of KSR2A mRNA, complete cds." Database accession no. AF418269 XP002210679**
- **DATABASE GENSEQ [Online] 8 November 2000 (2000-11-08) TAKAI,Y. ET AL.: "Rat MAGUIN 1 protein" Database accession no. AAY92942 XP002210736 -& DATABASE WPI Section Ch, Week 200033 Derwent Publications Ltd., London, GB; Class B04, AN 2000-387785 XP002210680 & WO 00 29572 A (KAGAKU GIJUTSU SHINKO JIGYODAN), 25 May 2000 (2000-05-25)**
- **DATABASE GENSEQ [Online] 8 November 2000 (2000-11-08) TAKAI, Y. ET AL.: "Rat MAGUIN 2 protein" Database accession no. AAY92943 XP002210737 -& DATABASE WPI Section Ch, Week 200033 Derwent Publications Ltd., London, GB; Class B04, AN 2000-387785 XP002210680 & WO 00 29572 A (KAGAKU GIJUTSU SHINKO JIGYODAN), 25 May 2000 (2000-05-25)**
- **MULDER, C.: "CSF markers relate to pathogenetic mechanisms in Alzheimer's disease" JOURNAL OF NEURAL TRANSMISSION, vol. 109, 2002, pages 1491-1498,**

EP 1 485 410 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

SEQUENCE LISTING

<110> Evotec NeuroSciences GmbH

<120> DIAGNOSTIC AND THERAPEUTIC USE OF HUMAN MAGUIN PROTEINS AND NUCLEIC ACIDS FOR NEURODEGENERATIVE DISEASES

<130> 030640wo ME/BM

<140> 02006353.3
<141> 2002-03-21

<160> 24

<170> PatentIn Ver. 2.1

<210> 1
<211> 1034
<212> PRT
<213> Homo sapiens

<400> 1

```
Met Ala Leu Ile Met Glu Pro Val Ser Lys Trp Ser Pro Ser Gln Val
  1               5                  10                  15

Val Asp Trp Met Lys Gly Leu Asp Asp Cys Leu Gln Gln Tyr Ile Lys
             20                  25                  30

Asn Phe Glu Arg Glu Lys Ile Ser Gly Asp Gln Leu Leu Arg Ile Thr
         35                  40                  45

His Gln Glu Leu Glu Asp Leu Gly Val Ser Arg Ile Gly His Gln Glu
     50                  55                  60

Leu Ile Leu Glu Ala Val Asp Leu Leu Cys Ala Leu Asn Tyr Gly Leu
 65                  70                  75                  80

Glu Thr Glu Asn Leu Lys Thr Leu Ser His Lys Leu Asn Ala Ser Ala
                 85                  90                  95

Lys Asn Leu Gln Asn Phe Ile Thr Gly Arg Arg Arg Ser Gly His Tyr
            100                 105                 110

Asp Gly Arg Thr Ser Arg Lys Leu Pro Asn Asp Phe Leu Thr Ser Val
        115                 120                 125

Val Asp Leu Ile Gly Ala Ala Lys Ser Leu Leu Ala Trp Leu Asp Arg
    130                 135                 140

Ser Pro Phe Ala Ala Val Thr Asp Tyr Ser Val Thr Arg Asn Asn Val
145                 150                 155                 160

Ile Gln Leu Cys Leu Glu Leu Thr Thr Ile Val Gln Gln Asp Cys Thr
                165                 170                 175
```

Val Tyr Glu Thr Glu Asn Lys Ile Leu His Val Cys Lys Thr Leu Ser
180 185 190

Gly Val Cys Asp His Ile Ile Ser Leu Ser Ser Asp Pro Leu Val Ser
195 200 205

Gln Ser Ala His Leu Glu Val Ile Gln Leu Ala Asn Ile Lys Pro Ser
210 215 220

Glu Gly Leu Gly Met Tyr Ile Lys Ser Thr Tyr Asp Gly Leu His Val
225 230 235 240

Ile Thr Gly Thr Thr Glu Asn Ser Pro Ala Asp Arg Cys Lys Lys Ile
245 250 255

His Ala Gly Asp Glu Val Ile Gln Val Asn His Gln Thr Val Val Gly
260 265 270

Trp Gln Leu Lys Asn Leu Val Asn Ala Leu Arg Glu Asp Pro Ser Gly
275 280 285

Val Ile Leu Thr Leu Lys Lys Arg Pro Gln Ser Met Leu Thr Ser Ala
290 295 300

Pro Ala Leu Leu Lys Asn Met Arg Trp Lys Pro Leu Ala Leu Gln Pro
305 310 315 320

Leu Ile Pro Arg Ser Pro Thr Ser Ser Val Ala Thr Pro Ser Ser Thr
325 330 335

Ile Ser Thr Pro Thr Lys Arg Asp Ser Ser Ala Leu Gln Asp Leu Tyr
340 345 350

Ile Pro Pro Pro Pro Ala Glu Pro Tyr Ile Pro Arg Asp Glu Lys Gly
355 360 365

Asn Leu Pro Cys Glu Asp Leu Arg Gly His Met Val Gly Lys Pro Val
370 375 380

His Lys Gly Ser Glu Ser Pro Asn Ser Phe Leu Asp Gln Glu Tyr Arg
385 390 395 400

Lys Arg Phe Asn Ile Val Glu Glu Asp Thr Val Leu Tyr Cys Tyr Glu
405 410 415

Tyr Glu Lys Gly Arg Ser Ser Ser Gln Gly Arg Arg Glu Ser Thr Pro
420 425 430

Thr Tyr Gly Lys Leu Arg Pro Ile Ser Met Pro Val Glu Tyr Asn Trp
435 440 445

Val Gly Asp Tyr Glu Asp Pro Asn Lys Met Lys Arg Asp Ser Arg Arg
450 455 460

Glu Asn Ser Leu Leu Arg Tyr Met Ser Asn Glu Lys Ile Ala Gln Glu
465 470 475 480

Glu Tyr Met Phe Gln Arg Asn Ser Lys Lys Asp Thr Gly Lys Lys Ser
485 490 495

Lys Lys Lys Gly Asp Lys Ser Asn Ser Pro Thr His Tyr Ser Leu Leu
500 505 510

Pro Ser Leu Gln Met Asp Ala Leu Arg Gln Asp Ile Met Gly Thr Pro
515 520 525

Val Pro Glu Thr Thr Leu Tyr His Thr Phe Gln Gln Ser Ser Leu Gln
530 535 540

His Lys Ser Lys Lys Lys Asn Lys Gly Pro Ile Ala Gly Lys Ser Lys
545 550 555 560

Arg Arg Ile Ser Cys Lys Asp Leu Gly Arg Gly Asp Cys Glu Gly Trp
565 570 575

Leu Trp Lys Lys Lys Asp Ala Lys Ser Tyr Phe Ser Gln Lys Trp Lys
580 585 590

Lys Tyr Trp Phe Val Leu Lys Asp Ala Ser Leu Tyr Trp Tyr Ile Asn
595 600 605

Glu Glu Asp Glu Lys Ala Glu Gly Phe Ile Ser Leu Pro Glu Phe Lys
610 615 620

Ile Asp Arg Ala Ser Glu Cys Arg Lys Lys Tyr Ala Phe Lys Ala Cys
625 630 635 640

His Pro Lys Ile Lys Ser Phe Tyr Phe Ala Ala Glu His Leu Asp Asp
645 650 655

Met Asn Arg Trp Leu Asn Arg Ile Asn Met Leu Thr Ala Gly Tyr Ala
660 665 670

Glu Arg Glu Arg Ile Lys Gln Glu Gln Asp Tyr Trp Ser Glu Ser Asp
675 680 685

Lys Glu Glu Ala Asp Thr Pro Ser Thr Pro Lys Gln Asp Ser Pro Pro
690 695 700

Pro Pro Tyr Asp Thr Tyr Pro Arg Pro Pro Ser Met Ser Cys Ala Ser
705 710 715 720

Pro Tyr Val Glu Ala Lys His Ser Arg Leu Ser Ser Thr Glu Thr Ser
725 730 735

Gln Ser Gln Ser Ser His Glu Glu Phe Arg Gln Glu Val Thr Gly Ser
740 745 750

Ser Ala Val Ser Pro Ile Arg Lys Thr Ala Ser Gln Arg Arg Ser Trp
755 760 765

Gln Asp Leu Ile Glu Thr Pro Leu Thr Ser Ser Gly Leu His Tyr Leu
770 775 780

Gln Thr Leu Pro Leu Glu Asp Ser Val Phe Ser Asp Ser Ala Ala Ile
785 790 795 800

Ser Pro Glu His Arg Arg Gln Ser Thr Leu Pro Thr Gln Lys Cys His
805 810 815

Leu Gln Asp His Tyr Gly Pro Tyr Pro Leu Ala Glu Ser Glu Arg Met
820 825 830

Gln Val Leu Asn Gly Asn Gly Gly Lys Pro Arg Ser Phe Thr Leu Pro
835 840 845

Arg Asp Ser Gly Phe Asn His Cys Cys Leu Asn Ala Pro Val Ser Ala
850 855 860

Cys Asp Pro Gln Asp Asp Val Gln Pro Pro Glu Val Glu Glu Glu Glu
865 870 875 880

Glu Glu Glu Glu Glu Glu Gly Glu Ala Ala Gly Glu Asn Ile Gly Glu
885 890 895

Lys Ser Glu Ser Arg Glu Glu Lys Leu Gly Asp Ser Leu Gln Asp Leu
900 905 910

Tyr Arg Ala Leu Glu Gln Ala Ser Leu Ser Pro Leu Gly Glu His Arg
915 920 925

Ile Ser Thr Lys Met Glu Tyr Lys Leu Ser Phe Ile Lys Arg Cys Asn
930 935 940

Asp Pro Val Met Asn Glu Lys Leu His Arg Leu Arg Ile Leu Lys Ser
945 950 955 960

Thr Leu Lys Ala Arg Glu Gly Glu Val Ala Ile Ile Asp Lys Val Leu
965 970 975

Asp Asn Pro Asp Leu Thr Ser Lys Glu Phe Gln Gln Trp Lys Gln Met
980 985 990

Tyr Leu Asp Leu Phe Leu Asp Ile Cys Gln Asn Thr Thr Ser Asn Asp
995 1000 1005

Pro Leu Ser Ile Ser Ser Glu Val Asp Val Ile Thr Ser Ser Leu Ala
1010 1015 1020

His Thr His Ser Tyr Ile Glu Thr His Val
1025 1030

<210> 2
<211> 948
<212> PRT
<213> Homo sapiens

<400> 2

```
Phe Ile Gly Arg Glu Ser Glu Gln Ile Asp Asn Ala Met Ile Asn Ala
 1               5                  10                  15

Cys Ile Asp Ser Glu Gln Glu Asn Cys Glu Phe His Met Ala Asn Met
            20                  25                  30

Ala Gly Ile Asn Pro Arg Thr Glu Ile Asn Leu Glu Asn Gly Thr His
        35                  40                  45

Ala Ala Met Ala Leu Ile Met Glu Pro Val Ser Lys Trp Ser Pro Ser
    50                  55                  60

Gln Val Val Asp Trp Met Lys Gly Leu Asp Asp Cys Leu Gln Gln Tyr
65                  70                  75                  80

Ile Lys Asn Phe Glu Arg Glu Lys Ile Ser Gly Asp Gln Leu Leu Arg
                85                  90                  95

Ile Thr His Gln Glu Leu Glu Asp Leu Gly Val Ser Arg Ile Gly His
            100                 105                 110

Gln Glu Leu Ile Leu Glu Ala Val Asp Leu Leu Cys Ala Leu Asn Tyr
        115                 120                 125

Gly Leu Glu Thr Glu Asn Leu Lys Thr Leu Ser His Lys Leu Asn Ala
    130                 135                 140

Ser Ala Lys Asn Leu Gln Asn Phe Ile Thr Gly Arg Arg Arg Ser Gly
145                 150                 155                 160

His Tyr Asp Gly Arg Thr Ser Arg Lys Leu Pro Asn Asp Phe Leu Thr
                165                 170                 175

Ser Val Val Asp Leu Ile Gly Ala Ala Lys Ser Leu Leu Ala Trp Leu
            180                 185                 190

Asp Arg Ser Pro Phe Ala Ala Val Thr Asp Tyr Ser Val Thr Arg Asn
        195                 200                 205

Asn Val Ile Gln Leu Cys Leu Glu Leu Thr Thr Ile Val Gln Gln Asp
    210                 215                 220

Cys Thr Val Tyr Glu Thr Glu Asn Lys Ile Leu His Val Cys Lys Thr
225                 230                 235                 240

Leu Ser Gly Val Cys Asp His Ile Ile Ser Leu Ser Ser Asp Pro Leu
```

245 250 255

Val Ser Gln Ser Ala His Leu Glu Val Ile Gln Leu Ala Asn Ile Lys
260 265 270

Pro Ser Glu Gly Leu Gly Met Tyr Ile Lys Ser Thr Tyr Asp Gly Leu
275 280 285

His Val Ile Thr Gly Thr Thr Glu Asn Ser Pro Ala Asp Arg Cys Lys
290 295 300

Lys Ile His Ala Gly Asp Glu Val Ile Gln Val Asn His Gln Thr Val
305 310 315 320

Val Gly Trp Gln Leu Lys Asn Leu Val Asn Ala Leu Arg Glu Asp Pro
325 330 335

Ser Gly Val Ile Leu Thr Leu Lys Lys Arg Pro Gln Ser Met Leu Thr
340 345 350

Ser Ala Pro Ala Leu Leu Lys Asn Met Arg Trp Lys Pro Leu Ala Leu
355 360 365

Gln Pro Leu Ile Pro Arg Ser Pro Thr Ser Ser Val Ala Thr Pro Ser
370 375 380

Ser Thr Ile Ser Thr Pro Thr Lys Arg Asp Ser Ser Ala Leu Gln Asp
385 390 395 400

Leu Tyr Ile Pro Pro Pro Pro Ala Glu Pro Tyr Ile Pro Arg Asp Glu
405 410 415

Lys Gly Asn Leu Pro Cys Glu Asp Leu Arg Gly His Met Val Gly Lys
420 425 430

Pro Val His Lys Gly Ser Glu Ser Pro Asn Ser Phe Leu Asp Gln Glu
435 440 445

Tyr Arg Lys Arg Phe Asn Ile Val Glu Glu Asp Thr Val Leu Tyr Cys
450 455 460

Tyr Glu Tyr Glu Lys Gly Arg Ser Ser Ser Gln Gly Arg Arg Glu Ser
465 470 475 480

Thr Pro Thr Tyr Gly Lys Leu Arg Pro Ile Ser Met Pro Val Glu Tyr
485 490 495

Asn Trp Val Gly Asp Tyr Glu Asp Pro Asn Lys Met Lys Arg Asp Ser
500 505 510

Arg Arg Glu Asn Ser Leu Leu Arg Tyr Met Ser Asn Glu Lys Ile Ala
515 520 525

Gln Glu Glu Tyr Met Phe Gln Arg Asn Ser Lys Lys Asp Thr Gly Lys

530 535 540

Lys Ser Lys Lys Lys Gly Asp Lys Ser Asn Ser Pro Thr His Tyr Ser
545 550 555 560

Leu Leu Pro Ser Leu Gln Met Asp Ala Leu Arg Gln Asp Ile Met Gly
565 570 575

Thr Pro Val Pro Glu Thr Thr Leu Tyr His Thr Phe Gln Gln Ser Ser
580 585 590

Leu Gln His Lys Ser Lys Lys Lys Asn Lys Gly Pro Ile Ala Gly Lys
595 600 605

Ser Lys Arg Arg Ile Ser Cys Lys Asp Leu Gly Arg Gly Asp Cys Glu
610 615 620

Gly Trp Leu Trp Lys Lys Lys Asp Ala Lys Ser Tyr Phe Ser Gln Lys
625 630 635 640

Trp Lys Lys Tyr Trp Phe Val Leu Lys Asp Ala Ser Leu Tyr Trp Tyr
645 650 655

Ile Asn Glu Glu Asp Glu Lys Ala Glu Gly Phe Ile Ser Leu Pro Glu
660 665 670

Phe Lys Ile Asp Arg Ala Ser Glu Cys Arg Lys Lys Tyr Ala Phe Lys
675 680 685

Ala Cys His Pro Lys Ile Lys Ser Phe Tyr Phe Ala Ala Glu His Leu
690 695 700

Asp Asp Met Asn Arg Trp Leu Asn Arg Ile Asn Met Leu Thr Ala Gly
705 710 715 720

Tyr Ala Glu Arg Glu Arg Ile Lys Gln Glu Gln Asp Tyr Trp Ser Glu
725 730 735

Ser Asp Lys Glu Glu Ala Asp Thr Pro Ser Thr Pro Lys Gln Asp Ser
740 745 750

Pro Pro Pro Pro Tyr Asp Thr Tyr Pro Arg Pro Pro Ser Met Ser Cys
755 760 765

Ala Ser Pro Tyr Val Glu Ala Lys His Ser Arg Leu Ser Ser Thr Glu
770 775 780

Thr Ser Gln Ser Gln Ser Ser His Glu Glu Phe Arg Gln Glu Val Thr
785 790 795 800

Gly Ser Ser Ala Val Ser Pro Ile Arg Lys Thr Ala Ser Gln Arg Arg
805 810 815

Ser Trp Gln Asp Leu Ile Glu Thr Pro Leu Thr Ser Ser Gly Leu His

```
            820                 825                 830

Tyr Leu Gln Thr Leu Pro Leu Glu Asp Ser Val Phe Ser Asp Ser Ala
        835                 840                 845

Ala Ile Ser Pro Glu His Arg Arg Gln Ser Thr Leu Pro Thr Gln Lys
    850                 855                 860

Cys His Leu Gln Asp His Tyr Gly Pro Tyr Pro Leu Ala Glu Ser Glu
865                 870                 875                 880

Arg Met Gln Val Leu Asn Gly Asn Gly Gly Lys Pro Arg Ser Phe Thr
                885                 890                 895

Leu Pro Arg Asp Ser Gly Phe Asn His Cys Cys Leu Asn Ala Pro Val
            900                 905                 910

Ser Ala Cys Asp Pro Gln Asp Asp Val Gln Pro Pro Glu Val Glu Glu
        915                 920                 925

Glu Glu Glu Glu Glu Glu Glu Glu Gly Glu Ala Ala Gly Glu Asn Ile
    930                 935                 940

Gly Glu Lys Ser
945
```

<210> 3
<211> 3105
<212> DNA
<213> Homo sapiens

<400> 3

```
atggctctga taatggaacc ggtgagcaaa tggtctccga gtcaagtagt ggactggatg 60
aaaggtcttg atgactgttt gcagcagtat attaagaact ttgagaggga gaagatcagt 120
ggggaccagc tgctgcgcat tacacatcag gagctagaag atctgggggt cagccgcatt 180
ggccatcagg aactgatctt ggaagcagtt gaccttctgt gtgcattgaa ttatggcttg 240
gaaacagaaa atctaaaaac cctttctcac aagttgaatg catctgccaa aaatctgcag 300
aattttataa caggaaggag aaggagtggc cattatgatg ggaggaccag ccgaaaattg 360
ccaaacgact ttctgacctc agttgtggat ctgattggag cagccaagag tctgcttgcc 420
tggttggaca ggtcaccatt tgctgctgtg acagactatt cagttacaag aaataatgtc 480
atacaactct gcctggagtt aacaacaatt gtgcaacagg attgtactgt atatgaaaca 540
gagaataaaa ttcttcacgt gtgtaaaact ctttctggag tctgtgacca catcatatcc 600
ctgtcgtcag atcctctggt ttcacagtct gctcacctgg aagtgattca actggcaaac 660
attaaaccaa gcgaagggct gggtatgtat attaaatcta catatgatgg cctccatgta 720
attactggaa ccacagaaaa ttcacctgca gatcggtgca agaaaatcca tgctggcgat 780
gaagtgattc aagttaatca tcagactgtg gtggggtggc agttgaaaaa tttggtgaat 840
gcactacgag aggacccgag tggtgttatc ttaactttga aaaagcgacc tcagagcatg 900
cttacctcag caccagcttt actgaaaaat atgagatgga agccccttgc tctgcagcct 960
cttataccta gaagtcccac aagcagcgtt gccacgcctt ccagcaccat cagtacaccc 1020
accaaaagag acagttctgc cctccaggat ctctacattc cccctcctcc tgcagaacca 1080
tatattccca gggatgaaaa aggaaacctt ccttgtgaag acctcagagg acatatggtg 1140
ggcaagccag tgcataaggg atctgaatca ccaaattcat ttctggatca ggaatatcga 1200
aagagattta atattgtcga agaagatact gtcttatatt gctatgaata tgaaaaagga 1260
```

```
agatcaagta gtcaaggaag acgagaaagc accccaactt atggcaagct acgacctata 1320
tctatgccag tggaatataa ttgggtgggg gactatgaag atccaaataa gatgaagaga 1380
gatagtagaa gagaaaactc tctacttcgg tatatgagca atgaaaagat tgctcaagaa 1440
gaatacatgt ttcagagaaa cagcaaaaag gacacaggga agaagtcaaa aaagaagggt 1500
gataagagta atagcccaac tcactattca ttgctaccta gtttacaaat ggatgcactg 1560
agacaagaca tcatgggcac tcctgtgcca gagaccacac tataccatac atttcagcag 1620
tcctcactgc agcacaaatc aaagaagaaa aacaaaggtc ctatagcagg caagagcaaa 1680
agacgaattt cttgcaaaga tcttggccgt ggtgactgtg agggctggct ttggaaaaag 1740
aaagatgcga agagttactt ttcacagaaa tggaaaaaat attggtttgt cctaaaggat 1800
gcatcccttt attggtatat taatgaggag gatgaaaaag cagaaggatt cattagcctg 1860
cctgaattta aaattgatag agccagtgaa tgccgcaaaa aatatgcatt caaagcctgt 1920
catcctaaaa tcaaaagctt ttattttgct gctgaacatc ttgatgatat gaacaggtgg 1980
cttaacagaa ttaatatgct gactgcagga tatgcagaaa gagagaggat taagcaggaa 2040
caagattact ggagtgagag tgacaaggaa gaagcagata ctccatcaac accaaaacaa 2100
gatagccctc cacccccata tgatacatac ccacgacctc cctcgatgag ttgcgccagt 2160
ccttatgtgg aagcaaaaca tagccgactt tcctccacgg agacttctca gtctcagtct 2220
tctcatgagg agtttcgcca ggaagtaact gggagcagtg cagtgtctcc cattcgcaag 2280
acagccagtc agcgccgctc ctggcaggat ttaattgaga cgccactgac aagttcaggc 2340
ttacactatc ttcagactct gccctggag gattctgtct tctctgactc cgcggccatc 2400
tccccagagc acaggcggca gtctaccctg ccaactcaga aatgccacct gcaggatcac 2460
tatgggccat accccttagc tgagagtgag aggatgcaag tgctaaatgg aaatgggggc 2520
aagcctcgaa gttttactct gcctcgagat agcgggttca accattgctg tctgaatgct 2580
ccagttagtg cctgtgaccc acaggatgac gtgcaacccc cagaggtgga ggaagaggag 2640
gaggaggagg aggaggaagg ggaggcagca ggggaaaaca taggagaaaa aagtgaaagc 2700
agagaagaaa agttaggaga ctcattgcaa gatttataca gggcactgga gcaggccagt 2760
ctgtcaccac taggagaaca tcgtatttca accaagatgg aatacaagct atcatttata 2820
aaaagatgta atgatcctgt aatgaatgaa aaactacacc ggctgagaat tctcaaaagc 2880
actttaaagg ccagagaagg ggaagtagcc attatcgata aagtcctaga caatccagac 2940
ttgacatcta aagaattcca acaatggaag cagatgtacc tcgacctttt cttggatatc 3000
tgtcaaaata ccacctcaaa tgacccactg agtatttctt ctgaagtaga tgtaatcact 3060
tcctctctag cacacactca ttcatacatt gaaacgcatg tctaa                 3105
```

<210> 4
<211> 2683
<212> DNA
<213> Homo sapiens

<400> 4

```
atggctctga taatggaacc ggtgagcaaa tggtctccga gtcaagtagt ggactggatg 60
aaaggtcttg atgactgttt gcagcagtat attaagaact ttgagaggga gaagatcagt 120
ggggaccagc tgctgcgcat tacacatcag gagctagaag atctgggggt cagccgcatt 180
ggccatcagg aactgatctt ggaagcagtt gaccttctgt gtgcattgaa ttatggcttg 240
gaaacagaaa atctaaaaac cctttctcac aagttgaatg catctgccaa aaatctgcag 300
aattttataa caggaaggag aaggagtggc cattatgatg ggaggaccag ccgaaaattg 360
ccaaacgact ttctgacctc agttgtggat ctgattggag cagccaagag tctgcttgcc 420
tggttggaca ggtcaccatt tgctgctgtg acagactatt cagttacaag aaataatgtc 480
atacaactct gcctggagtt aacaacaatt gtgcaacagg attgtactgt atatgaaaca 540
gagaataaaa ttcttcacgt gtgtaaaact ctttctggag tctgtgacca catcatatcc 600
ctgtcgtcag atcctctggt ttcacagtct gctcacctgg aagtgattca gctggcaaac 660
attaaaccaa gcgaagggct gggtatgtat attaaatcta catatgatgg cctccatgta 720
attactggaa ccacagaaaa ttcacctgca gatcggtgca agaaaatcca tgctggcgat 780
gaagtgattc aagttaatca tcagactgtg gtggggtggc agttgaaaaa tttggtgaat 840
gcactacgag aggacccgag tggtgttatc ttaactttga aaaagcgacc tcagagcatg 900
```

```
cttacctcag caccagcttt actgaaaaat atgagatgga agccccttgc tctgcagcct 960
cttataccta gaagtcccac aagcagcgtt gccacgcctt ccagcaccat cagtacaccc 1020
accaaaagag acagttctgc cctccaggat ctctacattc cccctcctcc tgcagaacca 1080
tatattccca gggatgaaaa aggaaacctt ccttgtgaag acctcagagg acatatggtg 1140
ggcaagccag tgcataaggg atctgaatca ccaaattcat ttctggatca ggaatatcga 1200
aagagattta atattgtcga agaagatact gtcttatatt gctatgaata tgaaaaagga 1260
agatcaagta gtcaaggaag acgagaaagc accccaactt atggcaagct acgacctata 1320
tctatgccag tggaatataa ttgggtgggg gactatgaag atccaaataa gatgaagaga 1380
gatagtagaa gagaaaactc tctacttcgg tatatgagca atgaaaagat tgctcaagaa 1440
gaatacatgt ttcagagaaa cagcaaaaag gacacaggga agaagtcaaa aaagaagggt 1500
gataagagta atagcccaac tcactattca ttgctaccta gtttacaaat ggatgcactg 1560
agacaagaca tcatgggcac tcctgtgcca gagaccacac tataccatac atttcagcag 1620
tcctcactgc agcacaaatc aaagaagaaa aacaaaggtc ctatagcagg caagagcaaa 1680
agacgaattt cttgcaaaga tcttggccgt ggtgactgtg agggctggct ttggaaaaag 1740
aaagatgcga agagttactt ttcacagaaa tggaaaaaat attggtttgt cctaaaggat 1800
gcatcccttt attggtatat taatgaggag gatgaaaaag cagaaggatt cattagcctg 1860
cctgaattta aaattgatag agccagtgaa tgccgcaaaa aatatgcatt caaagcctgt 1920
catcctaaaa tcaaaagctt ttattttgct gctgaacatc ttgatgatat gaacaggtgg 1980
cttaacagaa ttaatatgct gactgcagga tatgcagaaa gagagaggat taagcaggaa 2040
caagattact ggagtgagag tgacaaggaa gaagcagata ctccatcaac accaaaacaa 2100
gatagccctc cacccccata tgatacatac ccacgacctc cctcgatgag ttgcgccagt 2160
ccttatgtgg aagcaaaaca tagccgactt tcctccacgg agacttctca gtctcagtct 2220
tctcatgagg agtttcgcca ggaagtaact gggagcagtg cagtgtctcc cattcgcaag 2280
acagccagtc agcgccgctc ctggcaggat ttaattgaga cgccactgac aagttcaggc 2340
ttacactatc ttcagactct gcccctggag gattctgtct tctctgactc cgcggccatc 2400
tccccagagc acaggcggca gtctaccctg ccaactcaga aatgccacct gcaggatcac 2460
tatgggccat accccttagc tgagagtgag aggatgcaag tgctaaatgg aaatgggggc 2520
aagcctcgaa gttttactct gcctcgagat agcgggttca accattgctg tctgaatgct 2580
ccagttagtg cctgtgaccc acaggatgac gtgcaacccc cagaggtgga ggaagaggag 2640
gaggaggagg aggaggaagg ggaggcagca ggggaaaaca tag                   2683
```

<210> 5
<211> 5749
<212> DNA
<213> Artificial Sequence

<220>
<223> Description of Artificial Sequence: cDNA of human Maguin-1

<400> 5

```
cgggcagcta gtcgtgctcg gggcttcact cccgcgcgtg aggcgagcgg gcaagttggc 60
tgagggcgtg cggcagaggc tgcttccctc ggcgacgcga cccctcagca actcaagcta 120
tgaactgaag ctccctaggg acggagaccg gagcggagcg gcggaggcag cagcagcagc 180
agcagcagca gcagcagcag cagccgccgc cgccgccgcc ttagcgggaa ctgagcagac 240
ccggcgcgga gccacgactc ctgcacgttt acctccctgt cgccgttcct gccggcggtt 300
ggctaaaaga cgttacagcc gcgagacccg acacacaaaa gccgctttct ccgcgccgcc 360
cgcccaggga ggctgcggcc agcaagggac cccacctgag agcagctcgg gctgctgagt 420
tcgttttgtg tctgagctct gcgctctgca cggaaccgac cccgtaccca tggctctgat 480
aatggaaccg gtgagcaaat ggtctccgag tcaagtagtg gactggatga aaggtcttga 540
tgactgtttg cagcagtata ttaagaactt tgagagggag aagatcagtg gggaccagct 600
gctgcgcatt acacatcagg agctagaaga tctgggggtc agccgcattg gccatcagga 660
actgatcttg gaagcagttg accttctgtg tgcattgaat tatggcttgg aaacagaaaa 720
```

```
tctaaaaacc ctttctcaca agttgaatgc atctgccaaa aatctgcaga attttataac 780
aggaaggaga aggagtggcc attatgatgg gaggaccagc cgaaaattgc caaacgactt 840
tctgacctca gttgtggatc tgattggagc agccaagagt ctgcttgcct ggttggacag 900
gtcaccattt gctgctgtga cagactattc agttacaaga aataatgtca tacaactctg 960
cctggagtta acaacaattg tgcaacagga ttgtactgta tatgaaacag agaataaaat 1020
tcttcacgtg tgtaaaactc tttctggagt ctgtgaccac atcatatccc tgtcgtcaga 1080
tcctctggtt tcacagtctg ctcacctgga agtgattcaa ctggcaaaca ttaaaccaag 1140
cgaagggctg ggtatgtata ttaaatctac atatgatggc ctccatgtaa ttactggaac 1200
cacagaaaat tcacctgcag atcggtgcaa gaaaatccat gctggcgatg aagtgattca 1260
agttaatcat cagactgtgg tggggtggca gttgaaaaat ttggtgaatg cactacgaga 1320
ggacccgagt ggtgttatct taactttgaa aaagcgacct cagagcatgc ttacctcagc 1380
accagcttta ctgaaaaata tgagatggaa gccccttgct ctgcagcctc ttatacctag 1440
aagtcccaca agcagcgttg ccacgccttc cagcaccatc agtacaccca ccaaaagaga 1500
cagttctgcc ctccaggatc tctacattcc ccctcctcct gcagaaccat atattcccag 1560
ggatgaaaaa ggaaaccttc cttgtgaaga cctcagagga catatggtgg gcaagccagt 1620
gcataaggga tctgaatcac caaattcatt tctggatcag gaatatcgaa agagatttaa 1680
tattgtcgaa gaagatactg tcttatattg ctatgaatat gaaaaaggaa gatcaagtag 1740
tcaaggaaga cgagaaagca ccccaactta tggcaagcta cgacctatat ctatgccagt 1800
ggaatataat tgggtggggg actatgaaga tccaaataag atgaagagag atagtagaag 1860
agaaaactct ctacttcggt atatgagcaa tgaaaagatt gctcaagaag aatacatgtt 1920
tcagagaaac agcaaaaagg acacagggaa gaagtcaaaa aagaagggtg ataagagtaa 1980
tagcccaact cactattcat tgctacctag tttacaaatg gatgcactga gacaagacat 2040
catgggcact cctgtgccag agaccacact ataccataca tttcagcagt cctcactgca 2100
gcacaaatca aagaagaaaa acaaaggtcc tatagcaggc aagagcaaaa gacgaatttc 2160
ttgcaaagat cttggccgtg gtgactgtga gggctggctt tggaaaaaga aagatgcgaa 2220
gagttacttt tcacagaaat ggaaaaaata ttggtttgtc ctaaaggatg catcccttta 2280
ttggtatatt aatgaggagg atgaaaaagc agaaggattc attagcctgc ctgaatttaa 2340
aattgataga gccagtgaat gccgcaaaaa atatgcattc aaagcctgtc atcctaaaat 2400
caaaagcttt tattttgctg ctgaacatct tgatgatatg aacaggtggc ttaacagaat 2460
taatatgctg actgcaggat atgcagaaag agagaggatt aagcaggaac aagattactg 2520
gagtgagagt gacaaggaag aagcagatac tccatcaaca ccaaaacaag atagccctcc 2580
accccccatat gatacatacc cacgacctcc ctcgatgagt tgcgccagtc cttatgtgga 2640
agcaaaacat agccgacttt cctccacgga gacttctcag tctcagtctt ctcatgagga 2700
gtttcgccag gaagtaactg ggagcagtgc agtgtctccc attcgcaaga cagccagtca 2760
gcgccgctcc tggcaggatt taattgagac gccactgaca agttcaggct tacactatct 2820
tcagactctg cccctggagg attctgtctt ctctgactcc gcggccatct ccccagagca 2880
caggcggcag tctaccctgc caactcagaa atgccacctg caggatcact atgggccata 2940
ccccttagct gagagtgaga ggatgcaagt gctaaatgga aatgggggca agcctcgaag 3000
ttttactctg cctcgagata gcgggttcaa ccattgctgt ctgaatgctc cagttagtgc 3060
ctgtgaccca caggatgacg tgcaaccccc agaggtggag gaagaggagg aggaggagga 3120
ggaggaaggg gaggcagcag gggaaaacat aggagaaaaa agtgaaagca gagaagaaaa 3180
gttaggagac tcattgcaag atttatacag ggcactggag caggccagtc tgtcaccact 3240
aggagaacat cgtatttcaa ccaagatgga atacaagcta tcatttataa aaagatgtaa 3300
tgatcctgta atgaatgaaa aactacaccg gctgagaatt ctcaaaagca ctttaaaggc 3360
cagagaaggg gaagtagcca ttatcgataa agtcctagac aatccagact tgacatctaa 3420
agaattccaa caatggaagc agatgtacct cgaccttttc ttggatatct gtcaaaatac 3480
cacctcaaat gacccactga gtatttcttc tgaagtagat gtaatcactt cctctctagc 3540
acacactcat tcatacattg aaacgcatgt ctaaatgtat tctgccttca gaccatctag 3600
tacctgctgg tactctgaac aagtatataa ggtagttttt atatcaatgt gtggaacact 3660
tgacaagcta tactttaatg ttaccaaact atatgaaaca aaccatatat ggtcacaata 3720
ccactatctt taatgagcat ttgtatattt tatatgcaac agtgctcagc ttatgtttac 3780
catgtgcaaa atcaactgtc tttaatgact taaaattaac ttttgcaaac aattctaaat 3840
acaggtggtc ttcaagtagt aaaaccacaa aaggcagttt tctatctatg gtcatctttt 3900
ctccctttaa gttaatttta tataaacaag acttcaaaag taaatcacat tttttcaggt 3960
```

gcagacatcc ttgtgggtgg gaaagaattt aaaccttttt tatatttatt aaaatgttct 4020
aagaattttc ttaaacattg cacaaagttt aatgctgtag ttttattttt gtgaaatgta 4080
gatgcgcata caagagctaa gcaaaataga agagcatcga cataagaaaa gttcaggtat 4140
ctaatattcg tcttaatagt ctattaactt gtgaaagcta agttaatgga aatattattc 4200
caaatctatg agaacacttg gtgtatcagg gcaaagcttt gtaagatgtt tttgtaacta 4260
agaccaaaat tgaagataga gctgctttat tttcttggtt taaatcttcc tttattttttg 4320
tagtgatgag atgctgattg tgtacagaag aatttgagag gggattttta aaaactgact 4380
taacacaccc agaaaggcag ctaacagcta tatatatata taaatttcag cccaaactca 4440
tgtttttaaa ctccaactct taaaagacaa caaggtataa actgaaatga atcaactttc 4500
cacttagttt ccaattttcc cctagtccac taattaaact taggtaatta tacttcaggt 4560
agggaagtac aatatgttta gtttcaggct gatgtgtgtt ataaaaaaca acactgaaaa 4620
ataaaaatgt acttcccttc taaggagcaa gcaggtgatg gtcattcaaa gagatgtcac 4680
attgaattat gagagaaaca atttagaggt ttttttcctg gcttcatgaa ttgttctata 4740
gagtggatga agtctaagga aaagtcctct tcatatattt ccatttataa gcgtcttgtt 4800
tttgaaagtg atcacagcat gaaaatgact gtgctgcttt ttagtgtctg gctgcataat 4860
gtacaagtca caatttgctg tttttttcag gaggagaaag ggaacctcct ttactattct 4920
atatcctaaa atctacttct aatcagcttt atactgttgc ctgtacagct cagtgaatgt 4980
actttcatct ttaagagttc agatatatgc cagtgaatat ttttgctgta gaggagaaag 5040
taaaaactcc acagcgggga tctttttctt tgcttttgaa accaccattg aatcactatc 5100
gttttgcaga ctttgcacaa ctgtacagga gagtggcctt tctacagcac attttcagta 5160
atcctatatt tagtcaaaat ggatgagaaa tcatgtatta atgtttgtat ggaattttgg 5220
gtccagtgta atatttttat catttaaaaa gaactctatt tgtaaaaaca tttatttact 5280
gcatggatat tgacgcacat taaatttgtg ggattttgta tatgtaaaaa aaaaaaaaaa 5340
aaaaaaaaac aaaaaacctc ttgtcctaaa atgaagtgtg cttgttaaca ggtgtttaga 5400
cttattgatg tttactagac caaatgtgta tgttcactta aaaatatatg tacctgatgg 5460
atgtgtcatg tttacagtgg ccaggttgtg gcctgtaaac agcaagcagt tgacgggaag 5520
actagctctg ttgctactaa gcagctttta cttttgtaaa gtcagctctg ttgttttaaa 5580
tggtaaaaat taaactaatg aatttgacaa gactcgtggc tagcctagca tgaaagagac 5640
cttttaacac tatataatat ctgtacattt tattgcattc gtttcaaatc taggagagag 5700
gcagcactgt aaactgaagt caaataaatt cagctcttaa tgaatcctt 5749

<210> 6
<211> 4350
<212> DNA
<213> Artificial Sequence

<220>
<223> Description of Artificial Sequence: cDNA of human Maguin-2

<400> 6

gtgctcgggg cttcactccc gcgcgtgagg cgagcgggca agttggctga gggcgtgcgg 60
cagaggctgc ttccctcggc gacgcgaccc ctcagcaact caagctatga actgaagctc 120
cctagggacg gagaccggag cggagcggcg gaggcagcag cagcagcagc agcagcagca 180
gcagcagcag ccgccgccgc cgccgcctta gcgggaactg agcagacccg gcgcggagcc 240
acgactcctg cacgtttacc tccctgtcgc cgttcctgcc ggcggttggc taaaagacgt 300
tacagccgcg agacccgaca cacaaaagcc gctttctccg cgccgcccgc ccagggaggc 360
tgcggccagc aagggacccc acctgagagc agctcgggct gctgagttcg ttttgtgtct 420
gagctctgcg ctctgcacgg aaccgacccc gtacccatgg ctctgataat ggaaccggtg 480
agcaaatggt ctccgagtca agtagtggac tggatgaaag gtcttgatga ctgtttgcag 540
cagtatatta agaactttga gagggagaag atcagtgggg accagctgct gcgcattaca 600
catcaggagc tagaagatct gggggtcagc cgcattggcc atcaggaact gatcttggaa 660
gcagttgacc ttctgtgtgc attgaattat ggcttggaaa cagaaaatct aaaaaccctt 720

```
tctcacaagt tgaatgcatc tgccaaaaat ctgcagaatt ttataacagg aaggagaagg 780
agtggccatt atgatgggag gaccagccga aaattgccaa acgactttct gacctcagtt 840
gtggatctga ttggagcagc caagagtctg cttgcctggt tggacaggtc accatttgct 900
gctgtgacag actattcagt tacaagaaat aatgtcatac aactctgcct ggagttaaca 960
acaattgtgc aacaggattg tactgtatat gaaacagaga ataaaattct tcacgtgtgt 1020
aaaactcttt ctggagtctg tgaccacatc atatccctgt cgtcagatcc tctggtttca 1080
cagtctgctc acctggaagt gattcagctg gcaaacatta aaccaagcga agggctgggt 1140
atgtatatta aatctacata tgatggcctc catgtaatta ctggaaccac agaaaattca 1200
cctgcagatc ggtgcaagaa aatccatgct ggcgatgaag tgattcaagt taatcatcag 1260
actgtggtgg ggtggcagtt gaaaaatttg gtgaatgcac tacgagagga cccgagtggt 1320
gttatcttaa ctttgaaaaa gcgacctcag agcatgctta cctcagcacc agctttactg 1380
aaaaatatga gatggaagcc ccttgctctg cagcctctta tacctagaag tcccacaagc 1440
agcgttgcca cgccttccag caccatcagt acacccacca aaagagacag ttctgccctc 1500
caggatctct acattccccc tcctcctgca gaaccatata ttcccaggga tgaaaaagga 1560
aaccttcctt gtgaagacct cagaggacat atggtgggca agccagtgca taagggatct 1620
gaatcaccaa attcatttct ggatcaggaa tatcgaaaga gatttaatat tgtcgaagaa 1680
gatactgtct tatattgcta tgaatatgaa aaaggaagat caagtagtca aggaagacga 1740
gaaagcaccc caacttatgg caagctacga cctatatcta tgccagtgga atataattgg 1800
gtgggggact atgaagatcc aaataagatg aagagagata gtagaagaga aaactctcta 1860
cttcggtata tgagcaatga aaagattgct caagaagaat acatgtttca gagaaacagc 1920
aaaaaggaca cagggaagaa gtcaaaaaag aagggtgata agagtaatag cccaactcac 1980
tattcattgc tacctagttt acaaatggat gcactgagac aagacatcat gggcactcct 2040
gtgccagaga ccacactata ccatacattt cagcagtcct cactgcagca caaatcaaag 2100
aagaaaaaca aaggtcctat agcaggcaag agcaaaagac gaatttcttg caaagatctt 2160
ggccgtggtg actgtgaggg ctggctttgg aaaaagaaag atgcgaagag ttacttttca 2220
cagaaatgga aaaaatattg gtttgtccta aaggatgcat ccctttattg gtatattaat 2280
gaggaggatg aaaaagcaga aggattcatt agcctgcctg aatttaaaat tgatagagcc 2340
agtgaatgcc gcaaaaaata tgcattcaaa gcctgtcatc ctaaaatcaa aagcttttat 2400
tttgctgctg aacatcttga tgatatgaac aggtggctta acagaattaa tatgctgact 2460
gcaggatatg cagaaagaga gaggattaag caggaacaag attactggag tgagagtgac 2520
aaggaagaag cagatactcc atcaacacca aaacaagata gccctccacc cccatatgat 2580
acatacccac gacctccctc gatgagttgc gccagtcctt atgtggaagc aaaacatagc 2640
cgactttcct ccacggagac ttctcagtct cagtcttctc atgaggagtt tcgccaggaa 2700
gtaactggga gcagtgcagt gtctcccatt cgcaagacag ccagtcagcg ccgctcctgg 2760
caggatttaa ttgagacgcc actgacaagt tcaggcttac actatcttca gactctgccc 2820
ctggaggatt ctgtcttctc tgactccgcg gccatctccc cagagcacag gcggcagtct 2880
accctgccaa ctcagaaatg ccacctgcag gatcactatg ggccataccc cttagctgag 2940
agtgagagga tgcaagtgct aaatggaaat gggggcaagc ctcgaagttt tactctgcct 3000
cgagatagcg ggttcaacca ttgctgtctg aatgctccag ttagtgcctg tgacccacag 3060
gatgacgtgc aacccccaga ggtggaggaa gaggaggagg aggaggagga ggaaggggag 3120
gcagcagggg aaaacatagg agaaaaaagc taatacactg cgagagttgg tagaacctct 3180
ccatgccaaa tcggatccac ttctgttggc actcaaccca ttggactcac agattgataa 3240
gctaatgttt agagaattta gatcggagag agtcggtacg gcgcagactc aacatcaacc 3300
tcttgcaagc aactaaaatg gcctcgtcct tgctgtttat aacagaaaac agacttgtaa 3360
aaagcttaga tcatcaagtg ttttggattg ggggcctccc aaagggatat aagaggggca 3420
ggccactctt aagaagaatg cgagctttct acattgggac tagcataaga tcaaagccaa 3480
tcaagatgga gcacagtaac agaaaactgc ggtttctgtg ggagaacaga aggggaaagg 3540
gtcttaactg ggaaagggct ctgtgtggta acacctcagt tgtgttctcc tgacaccagg 3600
aaaagagagg gatcagcttc aataactaga aaattctggc tgtttaatgg actctttggt 3660
ggcctcttta aggcaaagca gagaaagcaa attatgtatt aagtgtattt tgcattttta 3720
aaacttgacg tgctgtattg tactaaatta agtgtaatct attaaggcaa ggtatacaca 3780
atttgctttg aaacttacta tgtttattct attataaagt gtattcaggt gcaacacaga 3840
gactgctttc ggtgacatta atgaagaaaa tttctcatgc caggctttat tatagaatct 3900
tcagctaaaa tcctaacttt ctccttattt cttggcactt gtatacaagt ggtgttgcct 3960
```

```
cttagggcag gcatgagcta ttcttttctg taaaatattt tgaatctata ggctgtgggt 4020
ttcatttttg aaaagtattt tgtctggatg tctttcaaac tagcttcaga tattatttaa 4080
tactatgtaa ctgggtcccc tatggctcaa tcaatattgc ttatttttct tctgtagtgg 4140
atgtgaaatt tcctttagtt ggataagata cactgtaata attttaatgc taattaatga 4200
tatttcatac tgtgcaatga acagataatt taacactgta ttttgaaatg tttttttctt 4260
cctgtcaccg cagtgtgtgg tattgcataa tgtgaatacc tgtaaaaata taaattactt 4320
aaaaataaaa atatgaccaa ttggtatcag                                  4350
```

<210> 7
<211> 50
<212> DNA
<213> Artificial Sequence

<220>
<223> Description of Artificial Sequence: cDNA fragment of Maguin-1

<400> 7
ggagagaggc agcactgtaa actgaagtca aataaattca gctcttaatg 50

<210> 8
<211> 13
<212> DNA
<213> Artificial Sequence

<220>
<223> Description of Artificial Sequence:
One-base-anchor oligonucleotides

<400> 8
httttttttt tta 13

<210> 9
<211> 13
<212> DNA
<213> Artificial Sequence

<220>
<223> Description of Artificial Sequence:
One-base-anchor oligonucleotides

<400> 9
httttttttt ttg 13

<210> 10
<211> 13
<212> DNA
<213> Artificial Sequence

<220>
<223> Description of Artificial Sequence:
One-base-anchor oligonucleotides

<400> 10
httttttttt ttc 13

<210> 11
<211> 19
<212> DNA
<213> Artificial Sequence

<220>
<223> Description of Artificial Sequence: Primer for Maguin-1

<400> 11
cagcaagcag ttgacggga 19

<210> 12
<211> 22
<212> DNA
<213> Artificial Sequence

<220>
<223> Description of Artificial Sequence: Primer for Maguin-1

<400> 12
tgaatttgac aagactcgtg gc 22

<210> 13
<211> 19
<212> DNA
<213> Artificial Sequence

<220>
<223> Description of Artificial Sequence: Primer for Maguin-2

<400> 13
gggcctccca aagggatat 19

<210> 14
<211> 22
<212> DNA
<213> Artificial Sequence

<220>
<223> Description of Artificial Sequence: Primer for Maguin-2

<400> 14
cccaatgtag aaagctcgca tt 22

<210> 15
<211> 20
<212> DNA
<213> Artificial Sequence

<220>
<223> Description of Artificial Sequence: primer for cyclophilin B

<400> 15
actgaagcac tacgggcctg 20

<210> 16
<211> 19
<212> DNA

<213> Artificial Sequence

<220>
<223> Description of Artificial Sequence: primer for cyclophilin B

<400> 16
agccgttggt gtctttgcc 19

<210> 17
<211> 20
<212> DNA
<213> Artificial Sequence

<220>
<223> Description of Artificial Sequence: Primer for ribosomal protein S9

<400> 17
ggtcaaattt accctggcca 20

<210> 18
<211> 22
<212> DNA
<213> Artificial Sequence

<220>
<223> Description of Artificial Sequence: Primer for ribosomal protein S9

<400> 18
tctcatcaag cgtcagcagt tc 22

<210> 19
<211> 19
<212> DNA
<213> Artificial Sequence

<220>
<223> Description of Artificial Sequence: primer for beta-actin

<400> 19
tggaacggtg aaggtgaca 19

<210> 20
<211> 19
<212> DNA
<213> Artificial Sequence

<220>
<223> Description of Artificial Sequence: primer for beta-actin

<400> 20
ggcaagggac ttcctgtaa 19

<210> 21
<211> 20
<212> DNA
<213> Artificial Sequence

<220>

<223> Description of Artificial Sequence: Primer for GAPDH

<400> 21
cgtcatgggt gtgaaccatg 20

<210> 22
<211> 21
<212> DNA
<213> Artificial Sequence

<220>
<223> Description of Artificial Sequence: Primer for GAPDH

<400> 22
gctaagcagt tggtggtgca g 21

<210> 23
<211> 21
<212> DNA
<213> Artificial Sequence

<220>
<223> Description of Artificial Sequence: Primer for transferrin receptor

<400> 23
gtcgctggtc agttcgtgat t 21

<210> 24
<211> 23
<212> DNA
<213> Artificial Sequence

<220>
<223> Description of Artificial Sequence: Primer for transferrin receptor

<400> 24
agcagttggc tgttgtacct ctc 23

## Description

The present invention relates to methods of diagnosing, prognosticating and monitoring the progression of Alzheimer's disease in a subject. Furthermore, methods of therapy control and screening for modulating agents of Alzheimer's disease are provided. The invention also discloses pharmaceutical compositions, the use of a kit, and the use of recombinant animal models.

Neurodegenerative diseases, in particular Alzheimer's disease (AD), have a strongly debilitating impact on a patient's life. Furthermore, these diseases constitute an enormous health, social, and economic burden. AD is the most common neurodegenerative disease, accounting for about 70% of all dementia cases, and it is probably the most devastating age-related neurodegenerative condition affecting about 10% of the population over 65 years of age and up to 45% over age 85 (for a recent review see Vickers et al., Progress in Neurobiology 2000, 60: 139-165). Presently, this amounts to an estimated 12 million cases in the US, Europe, and Japan. This situation will inevitably worsen with the demographic increase in the number of old people ("aging of the baby boomers") in developed countries. The neuropathological hallmarks that occur in the brains of individuals with AD are senile plaques, composed of amyloid-β protein, and profound cytoskeletal changes coinciding with the appearance of abnormal filamentous structures and the formation of neurofibrillary tangles.

The amyloid-β (Aβ) protein evolves from the cleavage of the amyloid precursor protein (APP) by different kinds of proteases. The cleavage by the β/γ-secretase leads to the formation of Aβ peptides of different lengths, typically a short more soluble and slow aggregating peptide consisting of 40 amino acids and a longer 42 amino acid peptide, which rapidly aggregates outside the cells, forming the characteristic amyloid plaques (Selkoe, Physiological Rev 2001, 81: 741-66; Greenfield et al., Frontiers Bioscience 2000, 5: D72-83). Two types of plaques, diffuse plaques and neuritic plaques, can be detected in the brain of AD patients, the latter ones being the classical, most prevalent type. They are primarily found in the cerebral cortex and hippocampus. The neuritic plaques have a diameter of 50μm to 200μm and are composed of insoluble fibrillar amyloids, fragments of dead neurons, of microglia and astrocytes, and other components such as neurotransmitters, apolipoprotein E, glycosaminoglycans, α1-antichymotrypsin and others. The generation of toxic Aβ deposits in the brain starts very early in the course of AD, and it is discussed to be a key player for the subsequent destructive processes leading to AD pathology. The other pathological hallmarks of AD are neurofibrillary tangles (NFTs) and abnormal neurites, described as neuropil threads (Braak and Braak, Acta Neuropathol 1991, 82: 239-259). NFTs emerge inside neurons and consist of chemically altered tau, which forms paired helical filaments twisted around each other. Along the formation of NFTs, a loss of neurons can be observed. It is discussed that said neuron loss may be due to a damaged microtubule-associated transport system (Johnson and Jenkins, J Alzheimers Dis 1996, 1: 38-58; Johnson and Hartigan, J Alzheimers Dis 1999, 1: 329-351). The appearance of neurofibrillary tangles and their increasing number correlates well with the clinical severity of AD (Schmitt et al., Neurology 2000, 55: 370-376).

AD is a progressive disease that is associated with early deficits in memory formation and ultimately leads to the complete erosion of higher cognitive function. The cognitive disturbances include among other things memory impairment, aphasia, agnosia and the loss of executive functioning. A characteristic feature of the pathogenesis of AD is the selective vulnerability of particular brain regions and subpopulations of nerve cells to the degenerative process. Specifically, the temporal lobe region and the hippocampus are affected early and more severely during the progression of the disease. On the other hand, neurons within the frontal cortex, occipital cortex, and the cerebellum remain largely intact and are protected from neurodegeneration (Terry et al., Annals of Neurology 1981, 10: 184-92).

The age of onset of AD may vary within a range of 50 years, with early-onset AD occurring in people younger than 65 years of age, and late-onset of AD occurring in those older than 65 years. About 10% of all AD cases suffer from early-onset AD, with only 1-2% being familial, inherited cases.

Currently, there is no cure for AD, nor is there an effective treatment to halt the progression of AD or even to diagnose AD ante-mortem with high probability. Several risk factors have been identified that predispose an individual to develop AD, among them most prominently the epsilon 4 allele of the three different existing alleles (epsilon 2, 3, and 4) of the apolipoprotein E gene (ApoE) (Strittmatter et al., Proc Natl Acad Sci USA 1993, 90: 1977-81; Roses, Ann NY Acad Sci 1998, 855: 738-43). The polymorphic plasmaprotein ApoE plays a role in the intercellular cholesterol and phospholipid transport by binding low-density lipoprotein receptors, and it seems to play a role in neurite growth and regeneration. Efforts to detect further susceptibility genes and disease-linked polymorphisms, lead to the assumption that specific regions and genes on human chromosomes 10 and 12 may be associated with late-onset AD (Myers et al., Science 2000, 290: 2304-5; Bertram et al., Science 2000, 290: 2303; Scott et al., Am J Hum Genet 2000, 66: 922-32).

Although there are rare examples of early-onset AD which have been attributed to genetic defects in the genes for amyloid precursor protein (APP) on chromosome 21, presenilin-1 on chromosome 14, and presenilin-2 on chromosome 1, the prevalent form of late-onset sporadic AD is of hitherto unknown etiologic origin. The mutations found to date account for only half of the familial AD cases, which is less than 2% of all AD patients. The late onset and complex pathogenesis of neurodegenerative disorders pose a formidable challenge to the development of therapeutic and diagnostic agents. It is pivotal to expand the pool of potential drug targets and diagnostic markers. It is therefore an object of the present invention to provide insight into the pathogenesis of neurological diseases and to provide methods, materials, agents, compositions, and animal models which are suited inter alia for the diagnosis and development of a treatment of these diseases. This object has been solved by the features of the independent claims. The subclaims define preferred embodiments of the present invention.

Neurons conduct signals in the form of electrical impulses. The communication between the cells of a neuronal network involves several chemical steps, including the production, release, and transport of signal molecules and the recognition of such messengers by a receptor. These steps take place at the anatomical contact of an axon with the dendrites (axonodendritic), the cell body (axonosomatic), or rarely with the axon of another neuron, and also between neurons and cells of muscle- and gland tissue. Trillions of such specialized cell junctions (synapses) in the human brain are crucial for controlling mental activity and learning processes (Tessier-Lavigne and Goodman, Science 1996, 274: 1123-1133). A synapse is composed of the presynaptic element, the synaptic cleft with a spacing distance of about 20-30 nm and the postsynaptic component. They are responsible for altering the membrane potential of the postsynaptic neuron or other effector cells. The quality and intensity of information transferred relies on the number, location, and distribution of synapses. The basis of learning and memory is believed to be due to brain plasticity, i.e. to the plasticity of synapses. The storage and processing of information cause alterations in the structure, chemistry, and strength of synapses and the formation of new synapses (Poirazi and Mel, Neuron 2001, 29: 779-796). The postsynaptic density (PSD) is a specialized synaptic signaling assemblage, composed of a specific set of proteins which are assembled together and linked to the cytoplasmic face of the postsynaptic membrane. An important function of the postsynaptic density is the provision of a structural matrix consisting of cytoskeletal and regulatory proteins which localize and accumulate neurotransmitter receptors (e.g. glutamate receptors) and anchor signaling molecules at the postsynaptic membrane (Sheng and Kim, Current Opinion Neurobiology 1996, 6: 602-608). Neurotransmitter receptors convert the extracellular chemical signals into intracellular signals. Neurotransmitters are released from the presynaptic membrane into the synaptic cleft via exocytotic processes (vesicle formation). At the postsynaptic membrane they are bound by their specific receptors, resulting in the generation of second messengers. Thus, neurotransmitters function as mediators of nerve impulse transmission across the synapse. The PSD organizes and regulates postsynaptic signal transduction (Kim and Huganir, Current Opinion Cell Biology 1999, 11:248-254). To date, several components of the PSD have been identified, among them the prototypic synaptic scaffolding protein postsynaptic density (PSD)-95/synapse-associated protein (SAP) 90. PSD-95/SAP90 and its isoforms belong to a family of membrane-associated guanylate kinases (MAGUK) (Hirao et al., Journal of Biological Chemistry 1998, 273: 21105-21110; Hirao et al., Journal of Biological Chemistry 2000, 275: 2966-2972). The members of the MAGUK protein family are multiple PDZ-domain containing proteins which interact with many neuronal adhesion proteins, receptors (e.g. N-methyl-D-aspartate (NMDA)) and ion-channels (e.g. potassium channels) through these domains and mediate their assembling at the PSD (Kim et al., Neuron 1996, 17: 103-113).

In this context, a novel neuronal membrane-associated guanylate kinase-interacting protein, denoted MAGUIN, was found by Yao et al. (Journal of Biological Chemistry 1999, 274: 11889-11896). Using the PDZ-domain sequence of the neurospecific synaptic scaffolding molecule (S-SCAM) as bait, the authors screened a rat brain yeast two-hybrid library and obtained several positive clones, among them two clones subsequently named rat MAGUIN-1 and rat MAGUIN-2 (GenBank accession numbers AF102853 and AF102854, respectively). A Northern blot analysis of different rat tissues with a rat MAGUIN-1 probe revealed brain specific hybridization signals at 4.4 kDa and 5.4 kDa. MAGUIN proteins have a chimerical molecular structure consisting of several protein modules, an N-terminally located sterile alpha-motif (SAM) (aa 8-75), a PDZ-domain (aa 156-296) and a C-terminally located Pleckstrin-homology (PH)-domain (aa 571-667). The Sterile Alpha Motif (SAM) contains four different domain structures, spanning approximately 70 amino acids generating a compact five-helix bundle with a highly conserved hydrophobic core. This motif was found to be part of a number of types of proteins, including signal transduction proteins, playing a role in mediating protein-protein interactions or DNA binding (Schultz et al., Protein Science 1997, 6: 249-253). The term PDZ is named after three proteins (i.e. PSD-95/SAP90, *Drosophila* discs-large tumor suppressor protein (Dig-A) and the tight junction protein ZO-1), originally identified as proteins sharing the same repeats of about 90 amino acids. These amino acids form a distinctive structure of two $\alpha$ helices and six $\beta$ sheets which mediate the interaction with the carboxyl termini of various proteins. Often, PDZ-domains of different proteins can heterodimerize with each other. The target proteins are transmembrane receptors, ion channels, or signaling proteins. PDZ-domain binding seems to be important in receptor clustering and in recruiting signal transduction molecules to the plasma membrane. PDZ-domain harboring proteins are for example tyrosine phosphatases and the previously described membrane-associated guanylate kinase-like proteins (Doyle et al., Cell 1996, 85: 1067-1076). The Pleckstrin-homology (PH)-domain forms an anti-parallel perpendicular $\beta$-sheet sandwich with a succeeding $\alpha$-helical structure. The $\beta$-sheet-loops are important for high affinity binding to specific phosphatidylinositide phosphates, allowing signaling proteins containing the PH-domain to anchor to membranes, for example as studied for GTP binding proteins, protein kinases and phospholipase C isoforms (Lemmon et al., Trends Cell Biology 1997, 7: 237-242).

Rat MAGUIN-1 and rat MAGUIN-2 cover 3099 and 2691 nucleotides of coding sequence, respectively, (Gen-

Bank accession numbers AF102853, AF102854) which encode for proteins of 1032 aa amino acids (rat MAGUIN-1) (GenBank accession number aad04568) and 896 amino acids (rat MAGUIN-2) (GenBank accession number aad04567), respectively. Rat MAGUIN-2 lacks the 3' terminal PDZ binding motif of rat MAGUIN-1. In neurons, the rat MAGUIN proteins are localized in the cell body and in neurites where they are associated with the plasma-membrane via their PH-domains. Full-length rat MAGUIN-1 could be recovered from neuronal membrane fractions. Rat MAGUIN-1, but not rat MAGUIN-2, interacts via the PDZ-binding motif with the PDZ-domain of PSD-95/SAP90 and the synaptic scaffolding molecule (S-SCAM) (Hirao et al., Journal of Biological Chemistry 1998, 273: 21105-21110). S-SCAM has recently been identified as a multiple PDZ-domain containing protein, interacting with SAP90/PSD-95-associated protein (SAPAP), the NMDA receptor, and neuronal adhesion molecules. Additionally, interaction of the PH-domain of rat MAGUIN-1 with the kinase domain of Raf could be confirmed *in vitro* and *in vivo,* but their is no evidence for the activation of Raf or its recruitment to the plasma membrane by rat MAGUIN-1.

To date, the precise function of rat MAGUIN-1 and rat MAGUIN-2 is still not clear. Some clues for the role of rat MAGUIN-1 in the cellular context come from particular protein domains and motifs, specific brain expression patterns, and structural homologies to other proteins like CNK (connector enhancer of kinase suppressor of ras (ksr)), as described for the fruit fly. CNK binds kinase suppressor for Ras and Raf kinase, functions in the Ras/MAP kinase pathway, and has been found to play a profound role in the regulation of eye development (Therrien et al., Cell 1998, 95: 343-353). On the basis of the similarities between *Drosophila* CNK (contains SAM, PDZ and PH-domains) and rat MAGUIN-1, rat MAGUIN-1 is discussed as a rodent homolog to CNK. Lanigan and Guan published the complete coding sequence of the human connector enhancer of KSR2A mRNA, which is the human homolog of Drosophila CNK interacting with the ras effector protein raf and turned out to be 100% homolog to human MAGUIN-1 (Database accession number AF418269, 2002-01-16). Thus, it is likely that rat MAGUIN-1 assembles components of synaptic junctions and regulators of the MAP kinase pathway and links them to NMDA receptors and neuronal adhesion molecules. To date, no experiments have been described that show a relationship between a differential expression of human MAGUIN genes, neither on a transcriptional nor on a translational level, and the pathology of neurodegenerative disorders.

The disclosure in the present invention of the human MAGUIN-1 gene and the identification of a link of both human MAGUIN-1 and/or human MAGUIN-2 to Alzheimer's disease, offers new ways, inter alia, for the diagnosis and treatment of such diseases.

The singular forms "a", "an", and "the" as used herein and in the claims include plural reference unless the context dictates otherwise. For example "a cell" means as well a plurality of cells, and so forth. The term "and/or" as used in the present specification and in the claims implies that the phrases before and after this term are to be considered either as alternatives or in combination. For instance, the wording "determination of a level and/or an activity" means that either only a level, or only an activity, or both a level and an activity are determined. The term "level" as used herein is meant to comprise a gage of, or a measure of the amount of, or a concentration of a transcription product, for instance an mRNA, or a translation product, for instance a protein or polypeptide. The term "activity" as used herein shall be understood as a measure for the ability of a transcription product or a translation product to produce a biological effect or a measure for a level of biologically active molecules. The term "activity" also refers to enzymatic activity. The terms "level" and/or "activity" further refer to gene expression levels or gene activity. Gene expression can be defined as the utilization of the information contained in a gene by transcription and translation leading to the production of a gene product. "Dysregulation" shall mean an upregulation or downregulation of gene expression. A gene product comprises either RNA or protein and is the result of expression of a gene. The amount of a gene product can be used to measure how active a gene is. The term "gene" as used in the present specification and in the claims comprises both coding regions (exons) as well as non-coding regions (e.g. non-coding regulatory elements such as promotors or enhancers, introns, leader and trailer sequences). The term "ORF" is an acronym for "open reading frame" and refers to a nucleic acid sequence that does not possess a stop codon in at least one reading frame and therefore can potentially be translated into a sequence of amino acids. "Regulatory elements" as used in the present disclosure shall comprise inducible and non-inducible promotors, enhancers, operators and other elements that drive and regulate gene expression. The term "fragment" as used herein is meant to comprise e.g. an alternatively spliced, or truncated, or otherwise cleaved transcription product or translation product. The term "derivative" as used herein refers to a mutant, or an RNA-edited, or a chemically modified, or otherwise altered transcription product, or to a mutant, or chemically modified, or otherwise altered translation product. For instance, a "derivative" may be generated by processes such as altered phosphorylation, or glycosylation, or acetylation, or lipidation, or by altered signal peptide cleavage or other types of maturation cleavage. These processes may occur post-translationally. The term "modulator" as used in the present invention and in the claims refers to a molecule capable of changing or altering the level and/or the activity of a gene, or a transcription product of a gene, or a translation product of a gene. Preferably, a "modulator" is capable of changing or altering the biological activity of a transcription product or a translation product of a gene. Said modulation, for instance, may be an increase or a decrease in enzyme activity, a change in binding characteristics, or any other change or alteration in the biological, functional, or immunological properties of said translation product of a gene.

The terms "agent", "reagent", or "compound" refer to any substance, chemical, composition or extract that have a positive or negative biological effect on a cell, tissue, body fluid, or within the context of any biological system, or any assay system examined. They can be agonists, antagonists, partial agonists or inverse agonists of a target. Such agents, reagents, or compounds may be nucleic acids, natural or synthetic peptides or protein complexes, or fusion proteins. They may also be antibodies, organic or anorganic molecules or compositions, small molecules, drugs and any combinations of any of said agents above. They may be used for testing, for diagnostic or for therapeutic purposes. The terms "oligonucleotide primer" or "primer" refer to short nucleic acid sequences which can anneal to a given target polynucleotide by hybridization of the complementary base pairs and can be extended by a polymerase. They may be chosen to be specific to a particular sequence or they may be randomly selected, e.g. they will prime all possible sequences in a mix. The length of primers used herein may vary from 10 nucleotides to 80 nucleotides. "Probes" are short nucleic acid sequences of the nucleic acid sequences described and disclosed herein or sequences complementary therewith. They may comprise full length sequences, or fragments, derivatives, isoforms, or variants of a given sequence. The identification of hybridization complexes between a "probe" and an assayed sample allows the detection of the presence of other similar sequences within that sample. As used herein, "homolog or homology" is a term used in the art to describe the relatedness of a nucleotide or peptide sequence to another nucleotide or peptide sequence, which is determined by the degree of identity and/or similarity between said sequences compared.

The term "variant" as used herein refers to any polypeptide and protein, in reference to polypeptides and proteins disclosed in the present invention, in which one or more amino acids are added and/or substituted and/or deleted and/or inserted at the N-terminus, and/or the C-terminus, and/or within the native amino acid sequences of the native polypeptides or proteins of the present invention. Furthermore, the term "variant" shall include any shorter or longer version of a polypeptide or protein. "Variants" shall also comprise a sequence that has at least about 80% sequence identity, more preferably at least about 90% sequence identity, and most preferably at least about 95% sequence identity with the amino acid sequences of SEQ ID NO. 1 and/or SEQ ID NO. 2, respectively. Derivatives, variants, and fragments may include, but are not limited to, a functional SAM, a functional PDZ and a functional PH domain or other functional modules within the polypeptide sequence of human MAGUIN-1 and/or human Maguin-2 proteins. "Variants" of a protein molecule shown in SEQ ID NO. 1 and/or SEQ ID NO. 2 may include, for example, proteins with conservative amino acid substitutions in highly conservative regions. For example, isoleucine, valine and leucine can each be substituted for one another. Aspartate and glutamate can be substituted for each other. Glutamine and asparagine can be substituted for each other. Serine and threonine can be substituted for each other. Amino acid substitutions in less conservative regions include, for example, isoleucine, valine and leucine, which can each be substituted for one another. Aspartate and glutamate can be substituted for each other. Glutamine and asparagine can be subsituted for each other. Serine and threonine can be substituted for each other. Glycine and alanine can be substituted for each other. Alanine and valine can be substituted for each other. Methionine can be substituted for each of leucine, isoleucine or valine, and vice versa. Lysine and arginine can be substituted for each other. One of aspartate and glutamate can be substituted for one of arginine or lysine, and vice versa. Histidine can be substituted for arginine or lysine, and vice versa. Glutamine and glutamate can be substituted for each other. Asparagine and aspartate can be substituted for each other. Other examples of protein modifications include glycosylation and further post-translational modifications. "Proteins and polypeptides" of the present invention include variants, fragments and chemical derivatives of the protein comprising SEQ ID NO. 1 and/or SEQ ID NO. 2. They can include proteins and polypeptides which can be isolated from nature or which can be produced by recombinant and/or synthetic means. Native proteins or polypeptides refer to naturally-occurring truncated or secreted forms, naturally occurring variant forms (e.g. splice variants) and naturally occurring allelic variants. As used herein, protein and polypeptide refers to a linear series of amino acid residues connected to one another by peptide bonds between the alpha-amino group and carboxy groups of adjacent amino acid residues. Other covalent bonds, such as amide and disulfide bonds, may also be present.

The term "isolated" as used herein is considered to refer to molecules that are removed from their natural environment, i.e. isolated from a cell or from a living organism in which they normally occur, and that are separated or essentially purified from the coexisting components with which they are found to be associated in nature. This notion further means that the sequences encoding such molecules can be linked by the hand of man to polynucleotides, to which they are not linked in their natural state, and that such molecules can be produced by recombinant and/or synthetic means. Even if for said purposes those sequences may be introduced into living or non-living organisms by methods known to those skilled in the art, and even if those sequences are still present in said organisms, they are still considered to be isolated. In the present invention, the terms "risk", "susceptibility", and "predisposition" are tantamount and are used with respect to the probability of developing a neurodegenerative disease, preferably Alzheimer's disease.

The term 'AD' shall mean Alzheimer's disease. "AD-type neuropathology" as used herein refers to neuropathological, neurophysiological, histopathological and clinical hallmarks as described in the instant invention and as commonly known from state-of-the-art literature (see: Iqbal, Swaab, Winblad and Wisniewski, Alzheimer's Disease and Related Disorders (Etiology, Pathogenesis and Therapeutics), Wiley & Sons, New York, Weinheim, Toronto, 1999; Scinto and Daffner, Early Diagnosis of Alzheimer's Disease, Humana Press, Totowa, New Jersey, 2000; Mayeux and Christen, Epidemiology of Alzheimer's Disease: From Gene to Prevention, Springer Press, Berlin, Heidelberg, New York, 1999;

Younkin, Tanzi and Christen, Presenilins and Alzheimer's Disease, Springer Press, Berlin, Heidelberg, New York, 1998). Neurodegenerative diseases or disorders according to the present invention comprise Alzheimer's disease, Parkinson's disease, Huntington's disease, amyotrophic lateral sclerosis, Pick's disease, fronto-temporal dementia, progressive nuclear palsy, corticobasal degeneration, cerebro-vascular dementia, multiple system atrophy, argyrophilic grain dementia and other tauopathies, and mild-cognitive impairment. Further conditions involving neurodegenerative processes are, for instance, age-related macular degeneration, narcolepsy, motor neuron diseases, prion diseases, traumatic nerve injury and repair, and multiple sclerosis.

In one aspect, the invention features a method of diagnosing or prognosticating Alzheimer's disease in a subject, or determining whether a subject is at increased risk of developing said disease. The method comprises: determining a level, or an activity, or both said level and said activity of (i) a transcription product of a gene coding for human MAGUIN-1 and/or human MAGUIN-2, and/or of (ii) a translation product of a gene coding for human MAGUIN-1 and/or human MAGUIN-2, and/or of (iii) a fragment, or derivative, or variant of said transcription or translation product in a sample from said subject and comparing said level, and/or said activity to a reference value representing a known disease or health status, thereby diagnosing said Alzheimer's disease in said subject, or determining whether said subject is at increased risk of developing said Alzheimer's disease.

The invention also relates to the construction and the use of primers and probes which are unique to the nucleic acid sequences, or fragments, or variants thereof, as disclosed in the present invention. The oligonucleotide primers and/or probes can be labeled specifically with fluorescent, bioluminescent, magnetic, or radioactive substances. The invention further relates to the detection and the production of said nucleic acid sequences, or fragments and variants thereof, using said specific oligonucleotide primers in appropriate combinations. PCR-analysis, a method well known to those skilled in the art, can be performed with said primer combinations to amplify said gene specific nucleic acid sequences from a sample containing nucleic acids. Such sample may be derived either from healthy or diseased subjects. Whether an amplification results in a specific nucleic acid product or not, and whether a fragment of different length can be obtained or not, may be indicative for Alzheimer's disease. Thus, the invention provides nucleic acid sequences, oligonucleotide primers, and probes of at least 10 bases in length up to the entire coding and gene sequences, useful for the detection of gene mutations and single nucleotide polymorphisms in a given sample comprising nucleic acid sequences to be examined, which may be associated with Alzheimer's disease. This feature has utility for developing rapid DNA-based diagnostic tests, preferably also in the format of a kit.

In a further aspect, the invention features a method of monitoring the progression of Alzheimer's disease in a subject. A level, or an activity, or both said level and said activity, of (i) a transcription product of a gene coding for human MAGUIN-1 and/or human MAGUIN-2, and/or of (ii) a translation product of a gene coding for human MAGUIN-1 and/or human MAGUIN-2, and/or of (iii) a fragment, or derivative, or variant of said transcription or translation product in a sample from said subject is determined. Said level and/or said activity is compared to a reference value representing a known disease or health status. Thereby the progression of said disease in said subject is monitored.

In still a further aspect, the invention features a method of evaluating a treatment for Alzheimer's disease, comprising determining a level, or an activity, or both said level and said activity of (i) a transcription product of a gene coding for human MAGUIN-1 and/or human MAGUIN-2, and/or of (ii) a translation product of a gene coding for human MAGUIN-1 and/or human MAGUIN-2, and/or of (iii) a fragment, or derivative, or variant of said transcription or translation product in a sample obtained from a subject being treated for said disease. Said level, or said activity, or both said level and said activity are compared to a reference value representing a known disease or health status, thereby evaluating the treatment for said disease.

In a preferred embodiment of the herein claimed methods, kits, recombinant animals, molecules, assays, and uses of the instant invention, said gene coding for the membrane-associated guanylate kinase-interacting proteins is the gene coding for the human neuronal membrane-associated guanylate kinase-interacting protein 1 (SEQ ID NO. 5), also termed (MAGUIN-1), and the human neuronal membrane-associated guanylate kinase-interacting protein 2 (SEQ ID NO. 6).

The present invention discloses the differential expression and regulation of the human MAGUIN-1 and/or human MAGUIN-2 gene in specific brain regions of Alzheimer's disease patients. Consequently, human MAGUIN-1 and/or human MAGUIN-2 and their corresponding translation products may have a causative role in the regional selective neuronal degeneration typically observed in Alzheimer's disease. Alternatively, human MAGUIN-1 and/or human MAGUIN-2 may confer a neuroprotective function to the remaining surviving nerve cells. Based on these disclosures, the present invention has utility for the diagnostic evaluation and prognosis as well as for the identification of a predisposition to Alzheimer's disease. Furthermore, the present invention provides methods for the diagnostic monitoring of patients undergoing treatment for such a disease.

It is preferred that the sample to be analyzed and determined is selected from the group comprising brain tissue, or other tissues, or other body cells. The sample can also comprise cerebrospinal fluid or other body fluids including saliva, urine, serum plasma, or mucus. Preferably, the methods of diagnosis, prognosis, monitoring the progression or evaluating a treatment for Alzheimer's disease, according to the instant invention, can be practiced ex corpore, and such methods preferably relate to samples, for instance, body fluids or cells, removed, collected, or isolated from a subject or patient.

In further preferred embodiments, said reference value is that of a level, or an activity, or both said level and said activity of (i) a transcription product of a gene coding for human MAGUIN-1 and/or human MAGUIN-2, and/or of (ii) a translation product of a gene coding for human MAGUIN-1 and/or human MAGUIN-2, and/or of (iii) a fragment, or derivative, or variant of said transcription or translation product in a sample from a subject not suffering from Alzheimer's disease.

In preferred embodiments, an alteration in the level and/or activity of a transcription product of the gene coding for human MAGUIN-1 and/or human MAGUIN-2 and/or a translation product of the gene coding for human MAGUIN-1 and/or human MAGUIN-2 protein in a sample cell, or tissue, or body fluid from said subject relative to a reference value representing a known health status indicates a diagnosis, or prognosis, or increased risk of becoming diseased with Alzheimer's disease.

In preferred embodiments, measurement of the level of transcription products of a gene coding for human MAGUIN-1 and/or human MAGUIN-2 is performed in a sample from a subject using a quantitative PCR-analysis with primer combinations to amplify said gene specific sequences from cDNA obtained by reverse transcription of RNA extracted from a sample of a subject. A Northern blot with probes specific for said gene can also be applied. It might also be preferred to measure transcription products by means of chip-based micro-array technologies. These techniques are known to those of ordinary skill in the art (see Sambrook and Russell, Molecular Cloning: A Laboratory Manual, Cold Spring Harbor Laboratory Press, Cold Spring Harbor, New York, 2001; Schena M., Microarray Biochip Technology, Eaton Publishing, Natick, MA, 2000). An example of an immunoassay is the detection and measurement of enzyme activity as disclosed and described in the patent application WO 02/14543.

Furthermore, the level and/or an activity of a translation product of a gene coding for human MAGUIN-1 and/or human MAGUIN-2, and/or a fragment, or derivative, or variant of said translation product, and/or the level of activity of said translation product, and/or a fragment, or derivative, or variant thereof, can be detected using an immunoassay, an activity assay, and/or a binding assay. These assays can measure the amount of binding between said protein molecule and an anti-protein antibody by the use of enzymatic, chromodynamic, radioactive, magnetic, or luminescent labels which are attached to either the anti-protein antibody or a secondary antibody which binds the anti-protein antibody. In addition, other high affinity ligands may be used. Immunoassays which can be used include e.g. ELISAs, Western blots and other techniques known to those of ordinary skill in the art (see Harlow and Lane, Using Antibodies: A Laboratory Manual, Cold Spring Harbor Laboratory Press, Cold Spring Harbor, New York, 1999 and Edwards R, Immunodiagnostics: A Practical Approach, Oxford University Press, Oxford; England, 1999). All these detection techniques may also be employed in the format of microarrays, protein-arrays, antibody microarrays, tissue microarrays, electronic biochip or protein-chip based technologies (see Schena M., Microarray Biochip Technology, Eaton Publishing, Natick, MA, 2000).

In a preferred embodiment, the level, of (i) a transcription product of human MAGUIN-1 and/or human MAGUIN-2, and/or of (ii) a translation product of human MAGUIN-1 and/or human MAGUIN-2, in a series of brain samples taken from said subject over a period of time is compared, in order to monitor the progression of said disease. In further preferred embodiments, said subject receives a treatment prior to one or more of said sample gatherings. In yet another preferred embodiment, said level and/or activity is determined before and after said treatment of said subject.

In another aspect, the invention features the use of a kit for diagnosing or prognosticating Alzheimer's disease in a subject, or determining the propensity or predisposition of a subject to develop Alzheimer's disease, said kit comprising,

(a) at least one reagent which is selected from the group consisting of (i) reagents that selectively detect a transcription product of a gene coding for human MAGUIN-1 and/or human MAGUIN-2 (ii) antibodies that selectively detect a translation product of a gene coding for human MAGUIN-1 and/or human MAGUIN-2; and
(b) instruction for diagnosing Alzheimer's disease or determining the propensity or predisposition of a subject to develop such a disease by

- detecting a level, or an activity, or both said level and said activity, of said transcription product and/or said translation product of a gene coding for human MAGUIN-1 and/or human MAGUIN-2, in a brain sample from said subject; and
- diagnosing Alzheimer's disease, in particular Alzheimer's disease, or determining the propensity or predisposition of said subject to develop such a disease,

wherein a varied level, or activity, or both said level and said activity, of said transcription product and/or said translation product compared to a reference value representing a known health status; or a level, or activity, or both said level and said activity, of said transcription product and/or said translation product similar or equal to a reference value representing a known disease status, indicates a diagnosis of Alzheimer's disease, or an increased propensity or predisposition of developing such a disease. The kit, according to the present invention, may be particularly useful for the identification of individuals that are at risk of developing Alzheimer's disease. Consequently, the kit, according to the invention, may serve as a means for targeting identified individuals for early preventive measures or therapeutic intervention prior to disease onset, before irreversible damage in the course of the disease has been inflicted. Furthermore, in preferred embodiments, the kit featured in the invention is useful for monitoring a progression of Alzheimer's disease, in a subject, as well as monitoring success or failure of therapeutic treatment for such a disease of said subject.

In a further aspect, the invention features the use of a recombinant, non-human animal comprising a non-native gene sequence coding for human MAGUIN-1 and/or human MAGUIN-2, or a fragment, or a variant, or a derivative thereof. The generation of said recombinant, non-human animal comprises (i) providing a gene targeting construct containing said gene sequence and a selectable marker sequence, and (ii) introducing said targeting construct into a stem cell of a non-human animal, and (iii) introducing said non-human animal stem cell into a non-human embryo, and (iv) transplanting said embryo into a pseudopregnant non-human animal, and (v) allowing said embryo to develop to term, and (vi) identifying a genetically altered non-human animal whose genome comprises a modification of said gene sequence in both alleles, and (vii) breeding the genetically altered non-human animal of step (vi) to obtain a genetically altered non-human animal whose genome comprises a modification of said endogenous gene, wherein said gene is mis-expressed, or under-expressed, or over-expressed, and wherein said disruption or alteration results in said non-human animal exhibiting a predisposition to developing symptoms of Alzheimer's disease. Strategies and techniques for the generation and construction of such an animal are known to those of ordinary skill in the art (see e.g. Capecchi, Science 1989, 244: 1288-1292 and Hogan et al., 1994, Manipulating the Mouse Embryo: A Laboratory Manual, Cold Spring Harbor Laboratory Press, Cold Spring Harbor, New York and Jackson and Abbott, Mouse Genetics and Transgenics: A Practical Approach, Oxford University Press, Oxford, England, 1999). It is preferred to make use of such a recombinant non-human animal as an animal model for investigating Alzheimer's disease. Such an animal may be useful for screening, testing and validating compounds, agents and modulators in the development of diagnostics and therapeutics to treat Alzheimer's disease.

In another aspect, the invention features an assay for screening for a modulator of Alzheimer's disease of one or more substances selected from the group consisting of (i) a gene coding for human MAGUIN-1 and/or human MAGUIN-2, and/or (ii) a transcription product of a gene coding for human MAGUIN-1 and/or human MAGUIN-2, and/or (iii) a translation product of a gene coding for human MAGUIN-1 and/or human MAGUIN-2, and/or (iv) a fragment, or derivative, or variant of (i) to (iii). This screening method comprises (a) contacting a cell with a test compound, and (b) measuring the level of one or more substances recited in (i) to (iv), and (c) measuring the level of said substances in a control cell not contacted with said test compound, and (d) comparing the levels of the substance in the cells of step (b) and (c), wherein an alteration in the activity and/or level of said substances in the contacted cells indicates that the test compound is a modulator of said diseases and disorders.

In one further aspect, the invention features a screening assay for a modulator of diseases, Alzheimer's disease of one or more substances selected from the group consisting of (i) a gene coding for human MAGUIN-1 and/or human MAGUIN-2, and/or (ii) a transcription product of a gene coding for human MAGUIN-1 and/or human MAGUIN-2, and/or (iii) a translation product of a gene coding for human MAGUIN-1 and/or human MAGUIN-2, comprising (a) administering a test compound to a test animal which is predisposed to developing or has already developed symptoms of Alzheimer's disease and (b) measuring the level of one or more substances recited in (i) to (iv), and (c) measuring the level of said substances in a matched control animal which is equally predisposed to developing or has already developed said symptoms and to which animal no such test compound has been administered, and (d) comparing the level of the substance in the animals of step (b) and (c), wherein an alteration in the level of substances in the test animal indicates that the test compound is a modulator of said disease.

In a preferred embodiment, said test animal and/or said control animal is a recombinant, non-human animal which expresses the gene coding for human MAGUIN-1 and/or human MAGUIN-2, or a fragment, or derivative, or a variant thereof, under the control of a transcriptional regulatory element which is not the native human MAGLHN-1 and/or human MAGUIN-2 gene transcriptional control regulatory element.

Also disclosed is a method for producing a medicament comprising the steps of (i) identifying a modulator of Alzheimer's disease by a method of the aforementioned screening assays and (ii) admixing the modulator with a pharmaceutical carrier. However, said modulator may also be identifiable by other types of screening assays.

Also disclosed is a medicament obtainable by any of the methods according to the herein claimed screening assays.

Also disclosed are protein molecules shown in SEQ ID NO. 1, and SEQ ID NO. 2, or fragments, or derivatives, or variants thereof, for use as diagnostic targets for detecting Alzheimer's disease.

The invention further features the use of an antibody specifically immunoreactive with an immunogen, wherein said immunogen is a translation product of the human MAGUIN-1 gene shown in SEQ ID NO. 1, or a fragment, or a variant, or a derivative thereof. The immunogen may comprise immunogenic or antigenic epitopes or portions of a translation product of said gene, wherein said immunogenic or antigenic portion of a translation product is a polypeptide, and wherein said polypeptide elicits an antibody response in an animal, and wherein said polypeptide is immunospecifically bound by said antibody. Methods for generating antibodies are well known in the art (see Harlow et al., Antibodies, A Laboratory Manual, Cold Spring Harbor Laboratory Press, Cold Spring Harbor, New York, 1988). The term "antibody", as employed in the present invention, encompasses all forms of antibodies known in the art, such as polyclonal, monoclonal, chimeric, recombinatorial, anti-idiotypic, humanized, or single chain antibodies, as well as fragments thereof (see Dubel and Breitling, Recombinant Antibodies, Wiley-Liss, New York, NY, 1999). Antibodies of the present invention are useful, for instance, in a variety of diagnostic and therapeutic methods, based on state-in-the-art techniques (see Harlow and Lane, Using Antibodies: A Laboratory Manual, Cold Spring Harbor Laboratory Press, Cold Spring Harbor, New York, 1999 and Edwards R., Immunodiagnostics: A Practical Approach, Oxford University Press, Oxford, England, 1999) such as enzyme-immuno assays (e.g. enzyme-linked immunosorbent assay, ELISA), radioimmuno assays, chemoluminescence-immuno assays, Western-blot, immunoprecipitation and antibody microarrays. These methods involve the detection of translation products of the human MAGUIN-1 gene.

The invention also features the use of an antibody specifically immunoreactive with an immunogen, wherein said immunogen is a translation product of the human MAGUIN-2 gene shown in SEQ ID NO. 2, or a fragment, a variant, or a derivative thereof.

In a preferred embodiment of the present invention, said antibodies can be used for detecting the pathological state of a cell in a sample from a subject, comprising immunocytochemical staining of said cell with said antibody, wherein an altered degree of staining, or an altered staining pattern in said cell compared to a cell representing a known health status indicates a pathological state of said cell. The invention is particularly suited to detect pathological structures in the brain of a subject. It is also especially suited to detect pathological cells of the muscular system, prostate, stomach, testis, ovary, adrenal glands, mammary glands, liver, spleen, lung, trachea or placenta.

Preferably, the pathological state relates to Alzheimer's disease. Immunocytochemical staining of a cell can be carried out by a number of different experimental methods well known in the art. It might be preferred, however, to apply an automated method for the detection of antibody binding, wherein the determination of the degree of staining of a cell, or the determination of the cellular or subcellular staining pattern of a cell, or the topological distribution of an antigen on the cell surface or among organelles and other subcellular structures within the cell, are carried out according to the method described in US patent 6150173.

Other features and advantages of the invention will be apparent from the following description of figures and examples which are illustrative only and not intended to limit the remainder of the disclosure in any way.

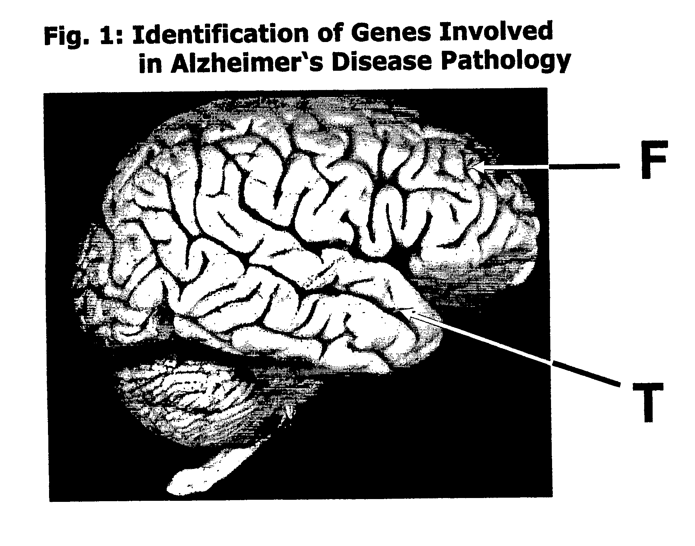

Figure 1 depicts the brain regions with selective vulnerability to neuronal loss and degeneration in Alzheimer's disease. Primarily, neurons within the inferior temporal lobe, the entorhinal cortex, the hippocampus, and the amygdala are subject to degenerative processes in Alzheimer's disease (Terry et al., Annals of Neurology 1981, 10: 184-192). These brain regions are mostly involved in the processing of learning and memory functions. In contrast, neurons within the frontal cortex, the occipital cortex, and the cerebellum remain largely intact and preserved from neurodegenerative processes in Alzheimer's disease. Brain tissues from the frontal cortex (F), the temporal cortex (T), and the hippocampus (H) of Alzheimer's disease patients and healthy, age-matched control individuals were used for the herein disclosed examples. For illustrative purposes, the image of a normal healthy brain was taken from a publication by Strange (Brain Biochemistry and Brain Disorders, Oxford University Press, Oxford, 1992, p.4).

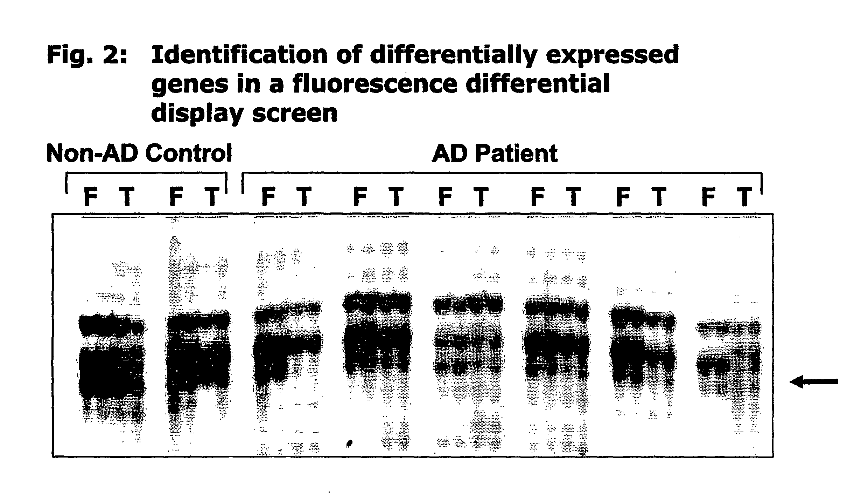

Figure 2 discloses the initial identification of the differential expression of the human MAGUIN-1 gene in a fluorescence differential display screen. The figure shows a clipping of a large preparative fluorescent differential display gel. PCR products from the frontal cortex (F) and the temporal cortex (T) of two healthy control subjects and six Alzheimer's diseased patients were loaded in duplicate onto a denaturing polyacrylamide gel (from left to right). PCR products were obtained by amplification of the individual cDNAs with the corresponding one-base-anchor oligonucleotide and the specific Cy3 labelled random primers. The arrow indicates the lane where significant differences in intensity of the signals for human MAGUIN-1 transcript derived from frontal cortex, compared to the signals for human MAGUIN-1 transcript derived from the temporal cortex of Alzheimer's patients exist. The differential expression reflects a down-regulation of human MAGUIN RNA expression in the temporal cortex in comparison to the frontal cortex of AD patients. Comparing the signals derived from frontal cortex and temporal cortex of healthy non-AD control subjects with each other, no distinction in signal intensity, i.e. no altered expression level can be detected.

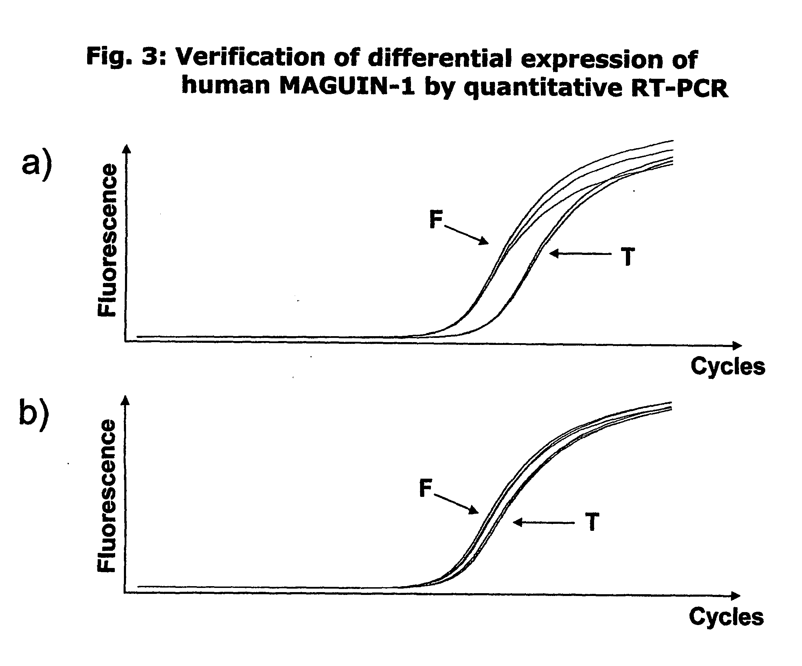

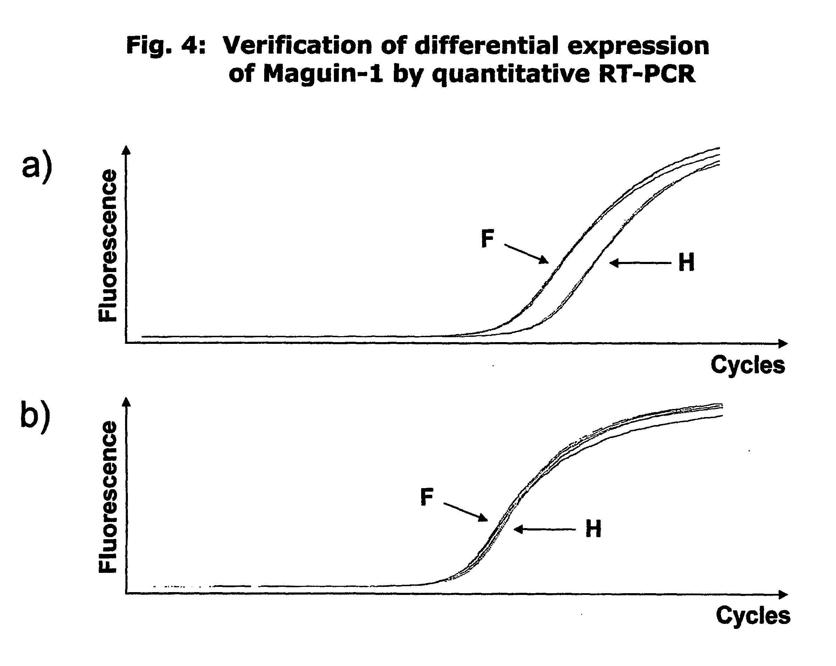

Figure 3 and Figure 4 illustrate the verification of the differential expression of human MAGUIN-1 in AD brain tissues by quantitative RT-PCR analysis. Quantification of RT-PCR products from RNA samples collected from the frontal cortex (F) and the temporal cortex (T) of AD patients (Figure 3a) and samples from the frontal cortex (F) and the hippocampus (H) of AD patients (Figure 4a) was performed by the LightCycler rapid thermal cycling technique. Likewise, samples of healthy, age-matched control individuals were compared (Figure 3b for frontal cortex and temporal cortex, Figure 4b for frontal cortex and hippocampus). The data were normalized to the combined average values of a set of standard genes which showed no significant differences in their gene expression levels. Said set of standard genes consisted of genes for cyclophilin B, the ribosomal protein S9, the transferrin receptor, GAPDH, and beta-actin. The figure depicts the kinetics of amplification by plotting the cycle number against the amount of amplified material as measured by its fluorescence. Note that the amplification kinetics of Maguin-1 cDNA from both, the frontal and temporal cortices of a normal control individual, and from the frontal cortex and hippocampus of a normal control individual, respectively, during the exponential phase of the reaction are juxtaposed (Figures 3b and 4b, arrowheads), whereas in Alzheimer's disease (Figures 3a and 4a, arrowheads) there is a significant separation of the corresponding curves, indicating a differential expression of Maguin-1 in the respective analyzed brain regions.

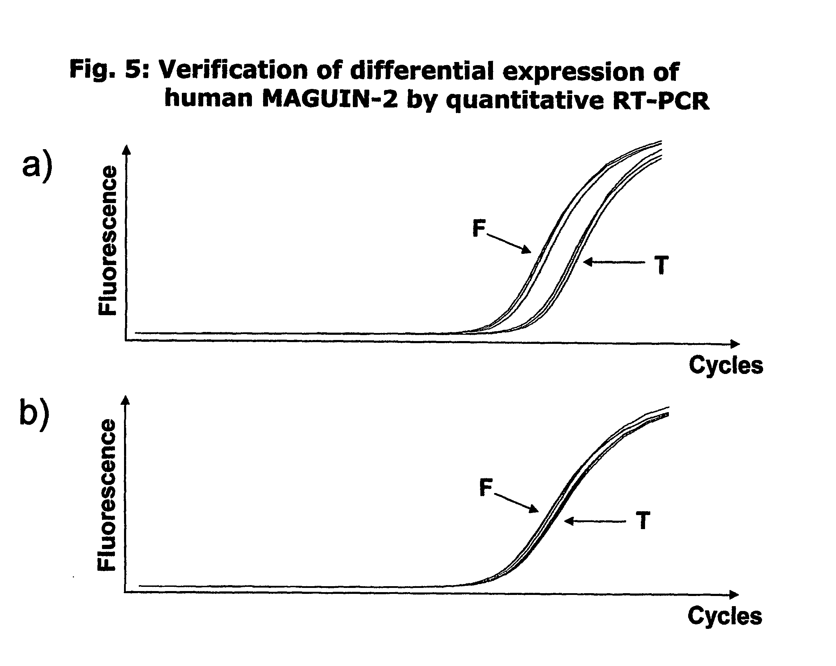

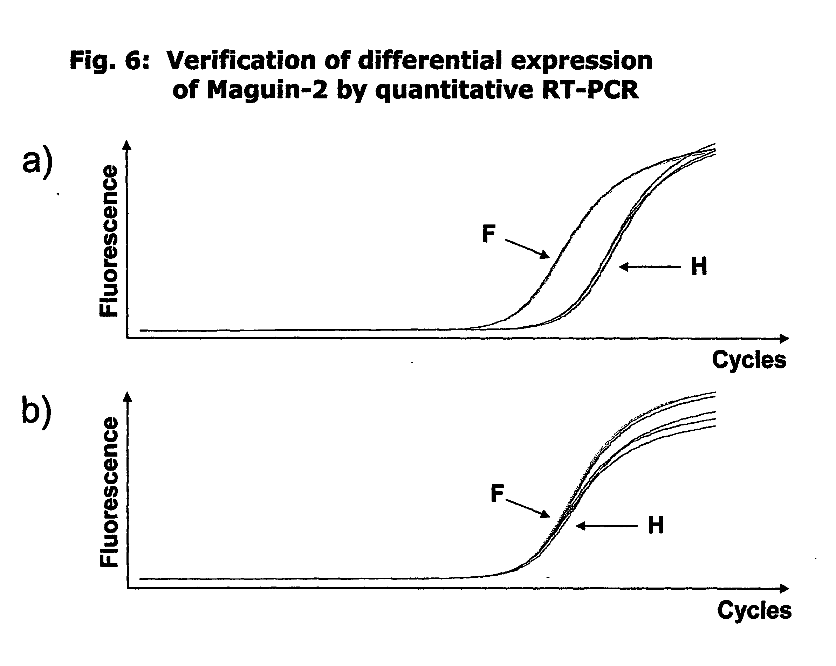

Figure 5 and Figure 6 illustrate the verification of the differential expression of human MAGUIN-2 in AD brain tissues by quantitative RT-PCR analysis. Quantification of RT-PCR products from RNA samples collected from the frontal cortex (F) and the temporal cortex (T) of AD patients (Figure 5a) and samples from the frontal cortex (F) and the hippocampus (H) of AD patients (Figure 6a) was performed by the LightCycler rapid thermal cycling technique. Likewise, samples of healthy, age-matched control individuals were compared (Figure 5b for frontal cortex and temporal cortex, Figure 6b for frontal cortex and hippocampus). The data were normalized to the combined average values of a set of standard genes which showed no significant differences in their gene expression levels. Said set of standard genes consisted of genes for cyclophilin B, the ribosomal protein S9, the transferrin receptor, GAPDH, and beta-actin. The figure depicts the kinetics of amplification by plotting the cycle number against the amount of amplified material as measured by its fluorescence. Note that the amplification kinetics of Maguin-2 cDNA from both, the frontal and temporal cortices of a normal control individual, and from the frontal cortex and hippocampus of a normal control individual, respectively, during the exponential phase of the reaction are juxtaposed (Figures 5b and 6b, arrowheads), whereas in Alzheimer's disease (Figures 5a and 6a, arrowheads) there is a significant separation of the corresponding curves, indicating a differential expression of Maguin-2 in the respective analyzed brain regions.

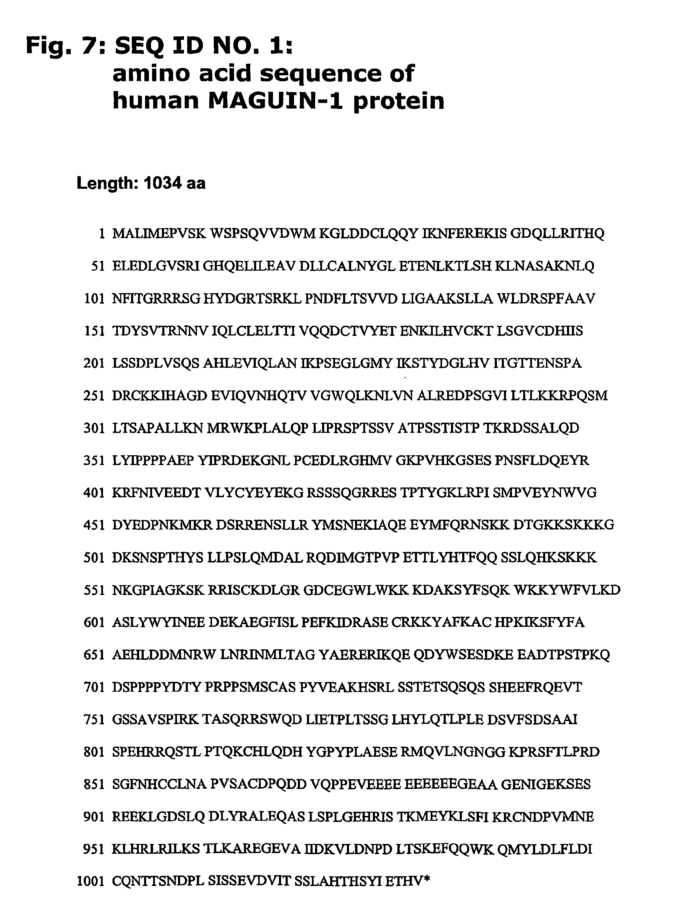

Figure 7 discloses the amino acid sequence of the human MAGUIN-1 protein comprising 1034 amino acids; SEQ ID NO. 1. The protein harbors several distinct functional domains which are located as follows: amino acid residues 8 to 75 form the 'Sterile Alpha Motif' (SAM), amino acid residues 156 to 296 constitute the PDZ domain, and the Pleckstrin-homology (PH) domain consists of amino acid residues 572 to 667.

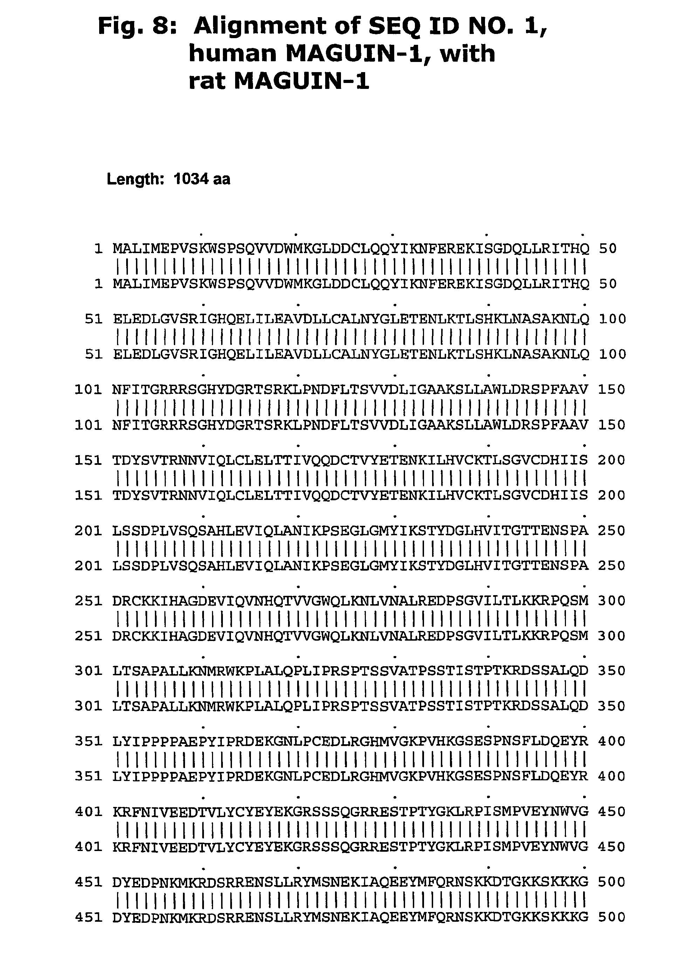

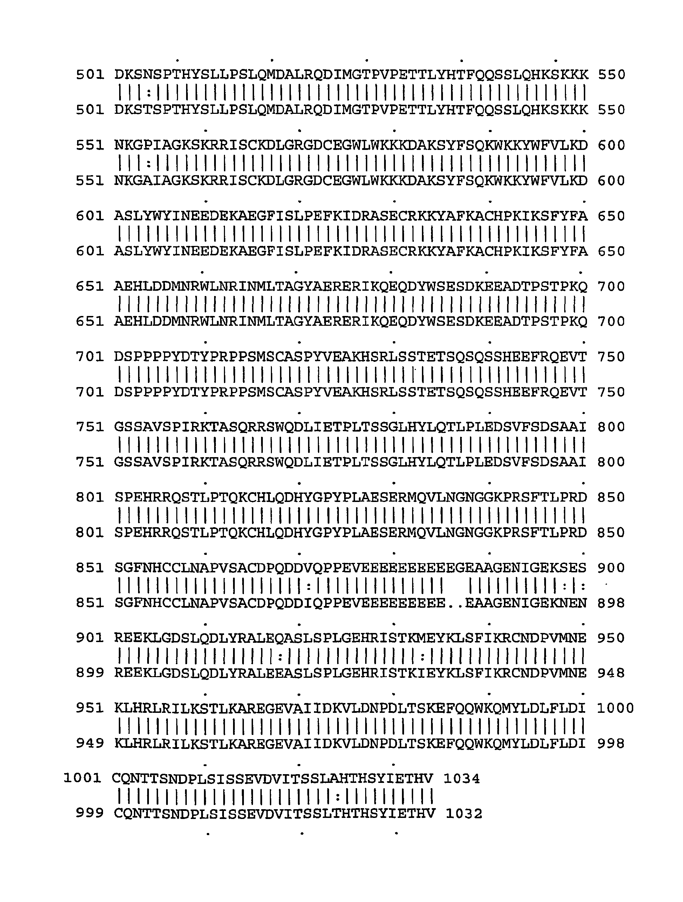

Figure 8 shows an alignment of the amino acid sequence of SEQ ID NO.1, human MAGUIN-1, with the rat MAGUIN-1 amino acid sequence (GenBank accession number aad04568). The full length human MAGUIN-1 protein consists of 1034 amino acids (residues given in the single-letter amino acid code).

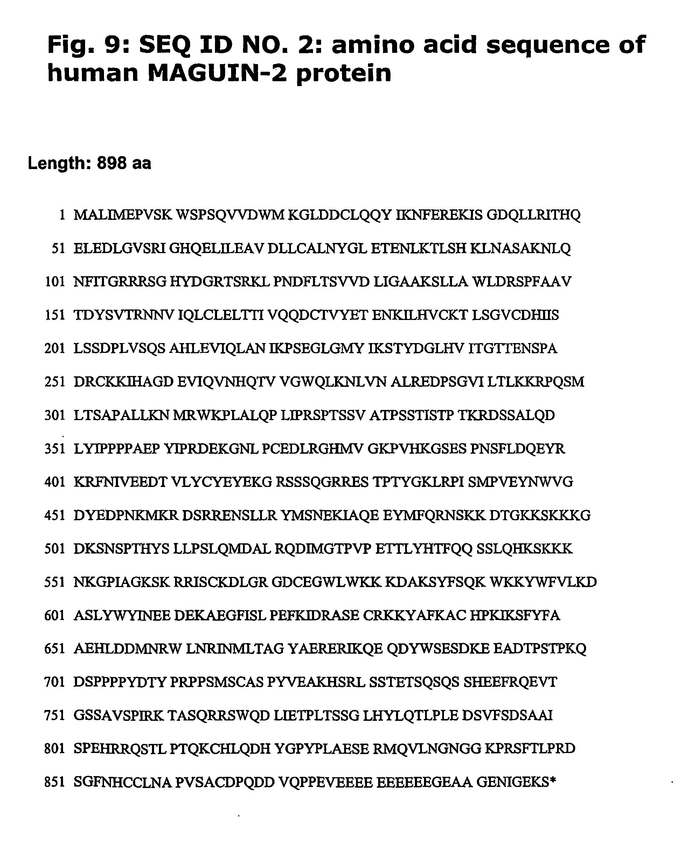

Figure 9 discloses the amino acid sequence of the human MAGUIN-2 protein comprising 898 amino acids; SEQ ID NO. 2. The protein harbors several distinct functional domains which are located as follows: amino acid residues 8 to 75 form the 'Sterile Alpha Motif' (SAM), amino acid residues 156 to 296 constitute the PDZ domain, and the Pleckstrin-homology (PH) domain consists of amino acid residues 572 to 667.

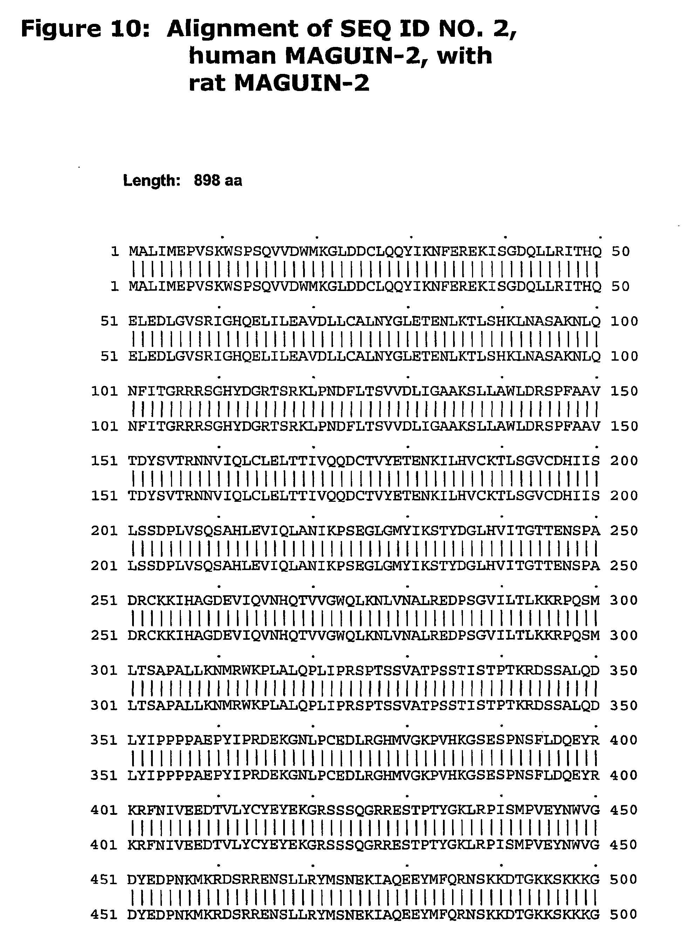

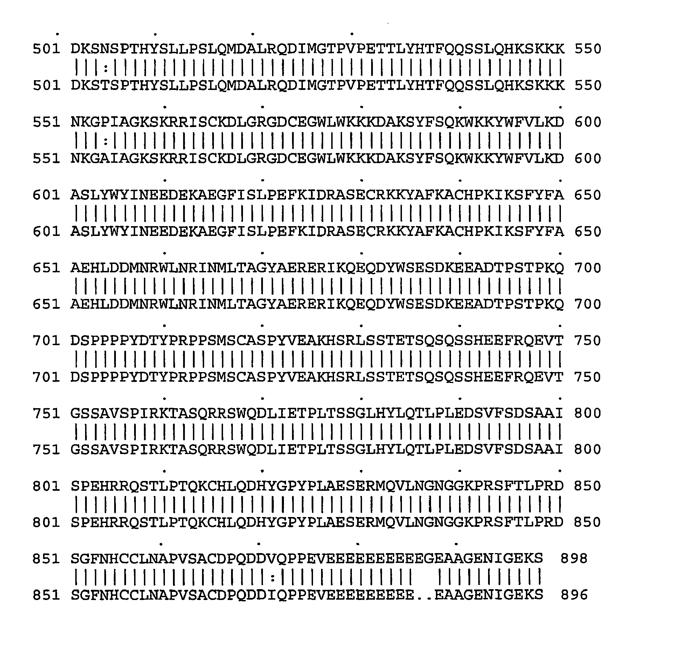

Figure 10 shows an alignment of the amino acid sequence of SEQ ID NO. 2, human MAGUIN-2, with rat MAGUIN-2 amino acid sequence (GenBank accession number aad04567). The full length human MAGUIN-2 protein consists of 898 amino acids (residues given in the single-letter amino acid code).

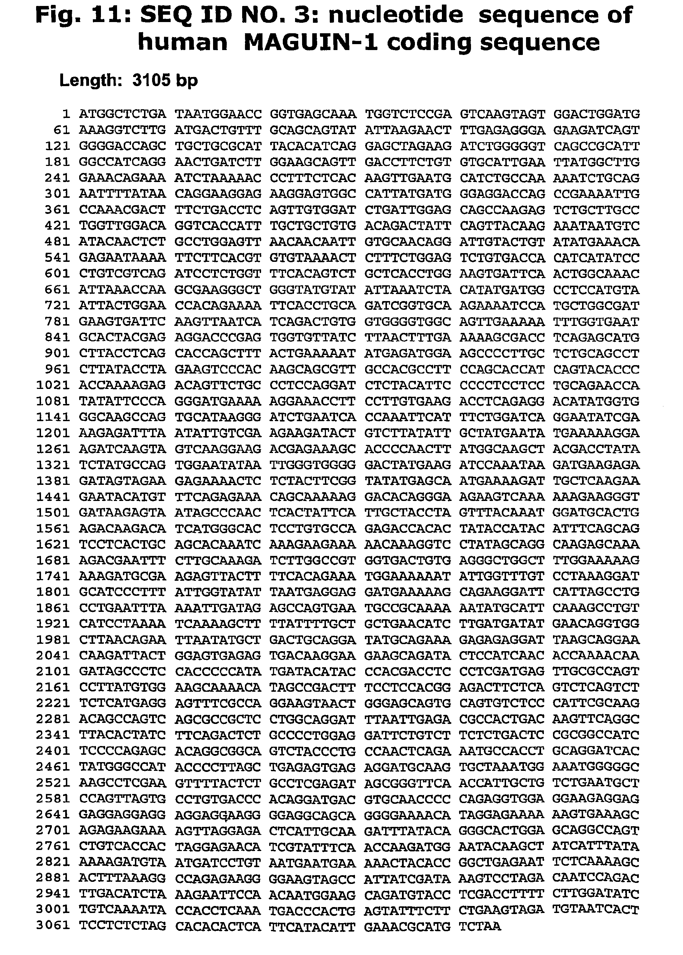

Figure 11 represents the nucleotide sequence of SEQ ID NO. 3, the coding sequence of the human MAGUIN-1 gene, comprising 3105 nucleotides.

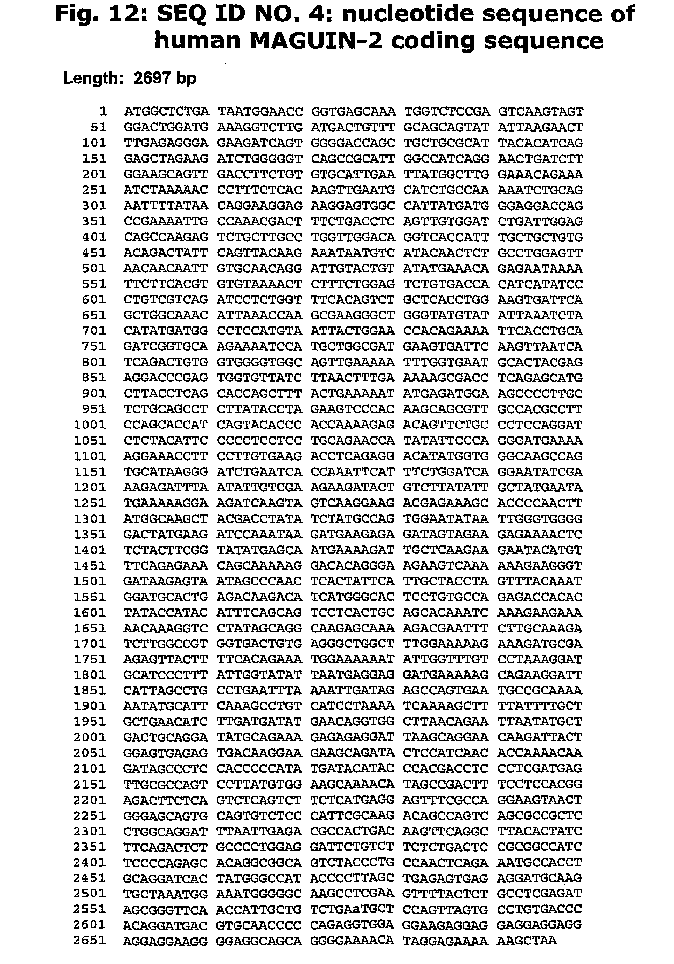

Figure 12 represents the nucleotide sequence of SEQ ID NO. 4, the coding sequence of the human MAGUIN-2 gene, comprising 2697 nucleotides.

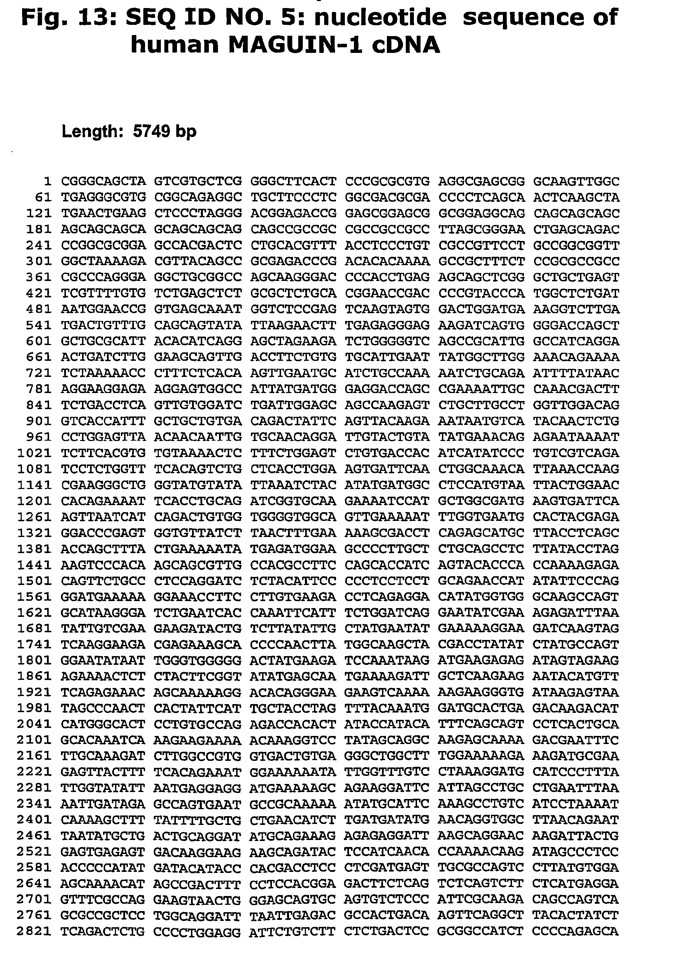

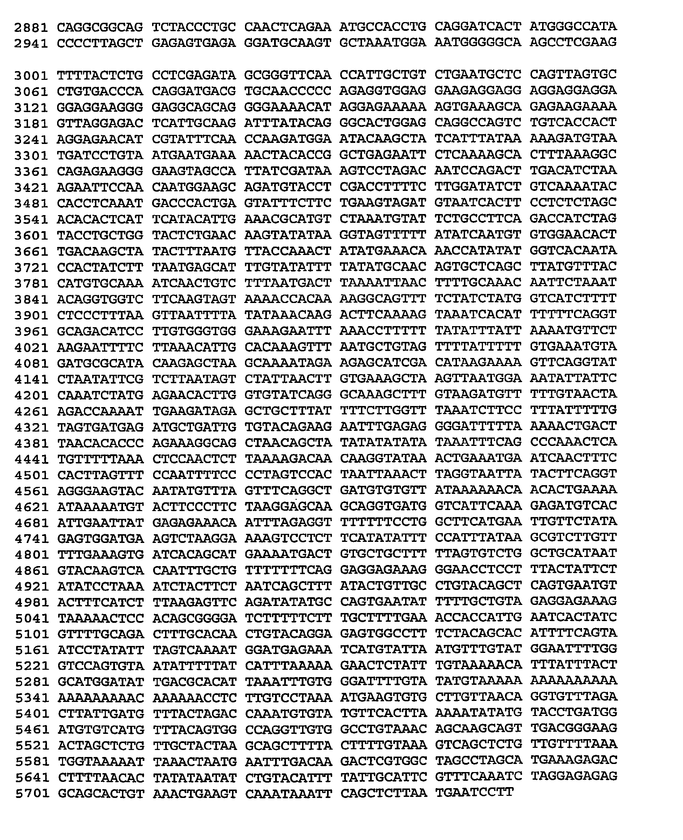

Figure 13 shows SEQ ID NO. 5, the nucleotide sequence of the human MAGUIN-1 cDNA, comprising 5749 nucleotides.

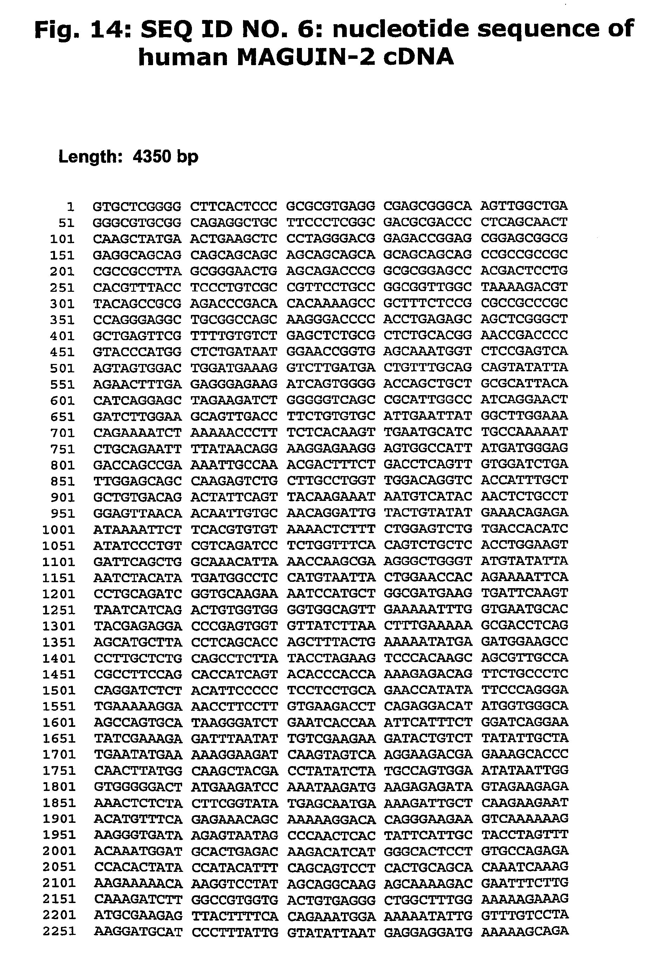

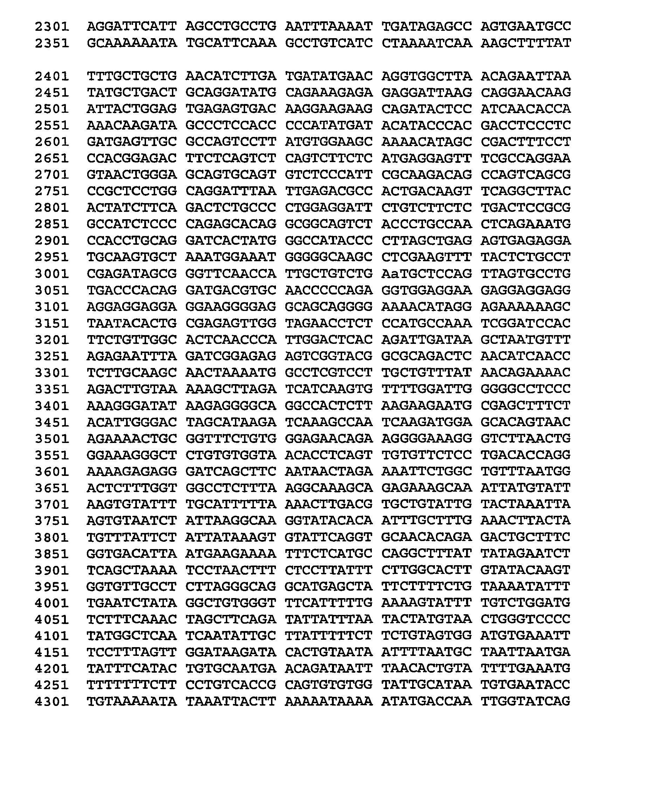

Figure 14 shows SEQ ID NO. 6, the nucleotide sequence of the human MAGUIN-2 cDNA; comprising 4350 nucleotides.

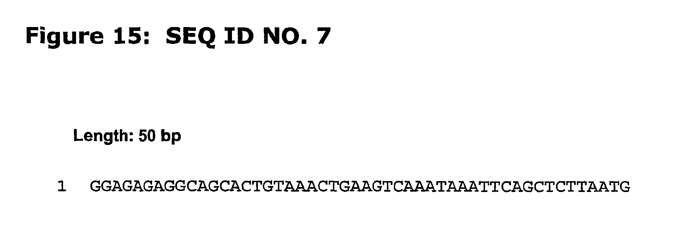

Figure 15 depicts SEQ ID NO. 7, the nucleotide sequence of the 50 bp MAGUIN-1 cDNA fragment, identified and obtained by fluorescence differential display and subsequent cloning.

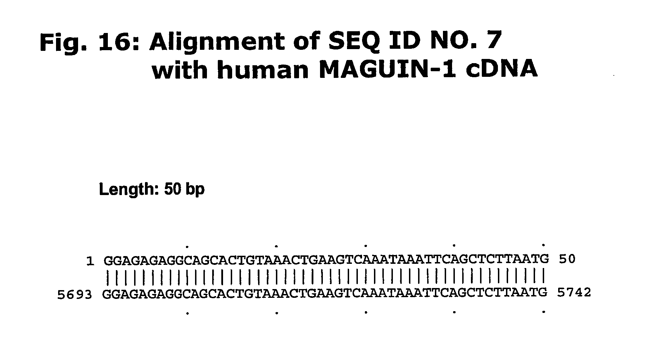

Figure 16 outlines the sequence alignment of SEQ ID NO. 7, the 50 bp human MAGUIN- . 1 cDNA fragment, with the 3'UTR nucleotide sequence of SEQ ID NO. 5 (nucleotide 5693 to 5742), the nucleotide sequence of human MAGUIN-1 cDNA.

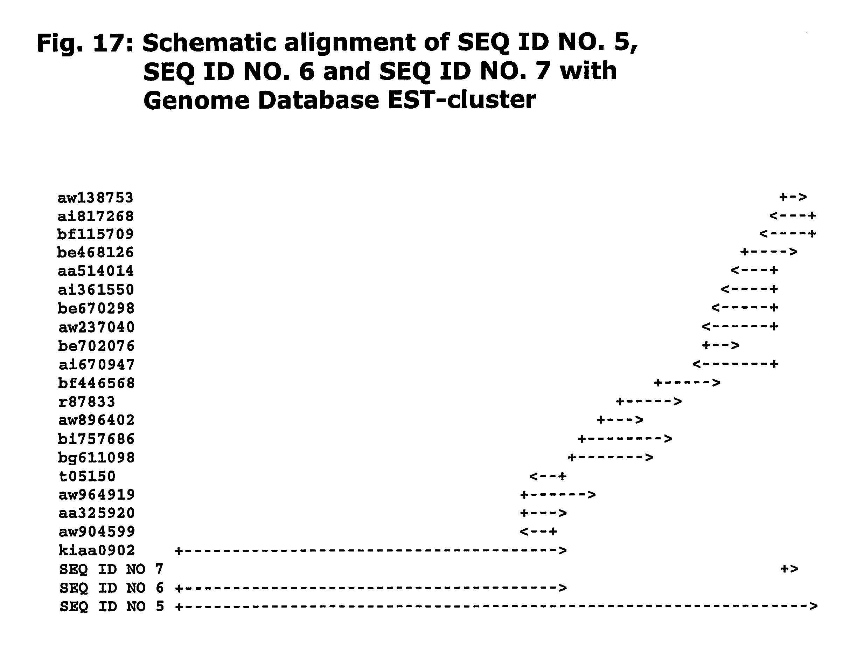

Figure 17 charts the schematic alignment of SEQ ID NO. 7, the human MAGUIN-1 cDNA fragment, SEQ ID NO. 6, the human MAGUIN-2 cDNA sequence and the nucleotide sequence of SEQ ID NO. 5, the nucleotide sequence of human MAGUIN-1 cDNA, derived from the alignment of EST nucleotide sequences as found in the GenBank genetic sequence database. EST numbers are written on the left side, all sequences are 5' to 3' directed.

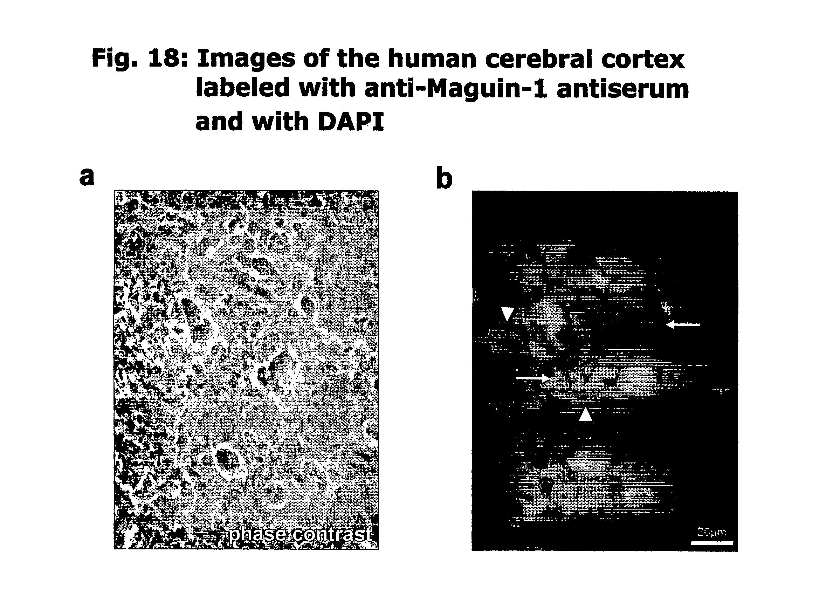

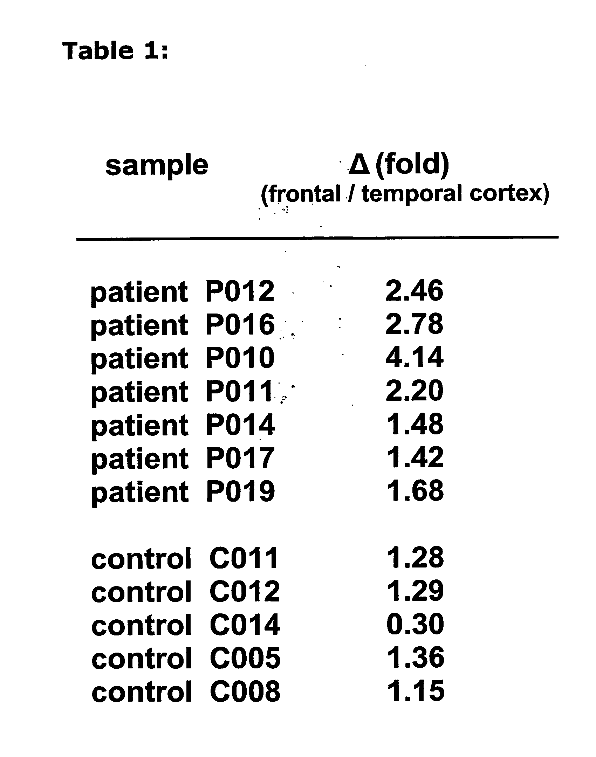

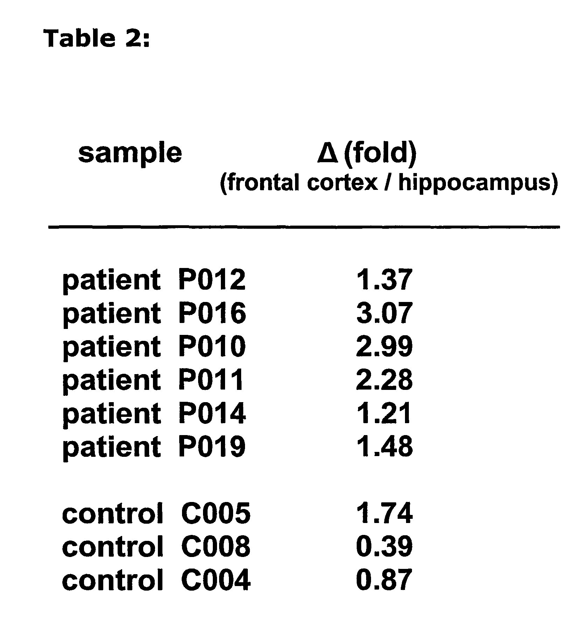

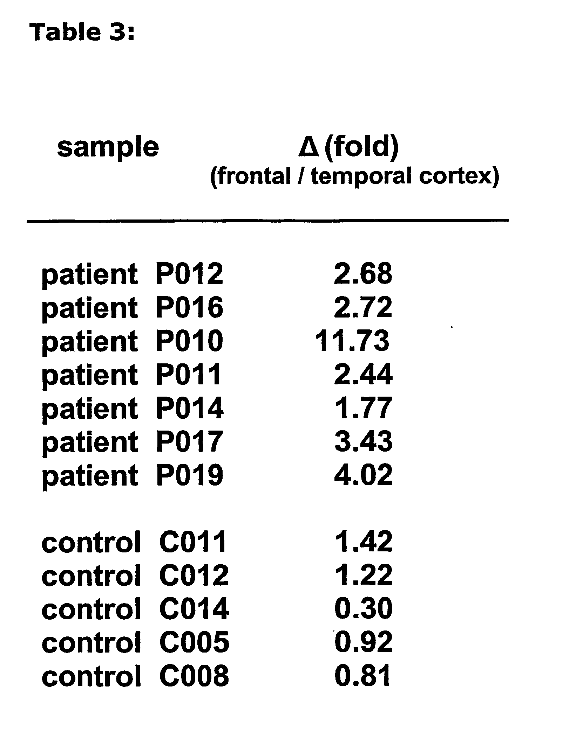

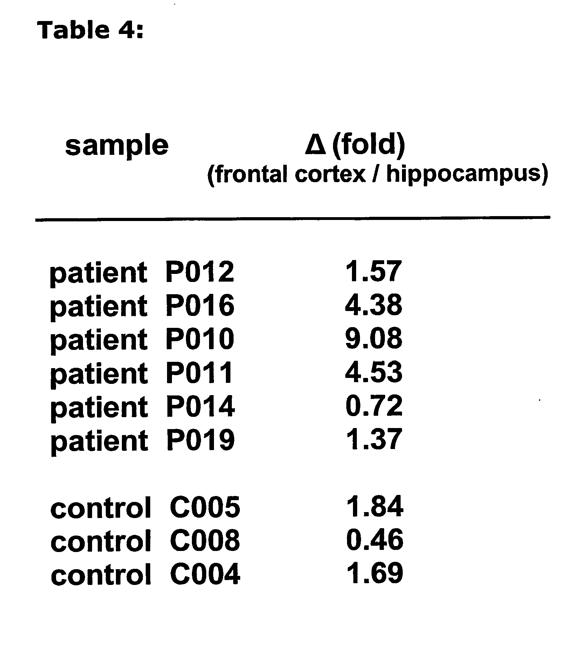

Figure 18 depicts human cerebral cortex labeled with an affinity-purified rabbit anti-Maguin-1 antiserum raised against a peptide corresponding to amino acids 914-928 (green signals). Strong immunoreactivity of human Maguin-1 was detected in neuronal cell bodies (indicated by arrowheads) and in neurites, whereas glial cells were immuno-negative (see arrows). The same immunostaining pattern was observed by using another antiserum raised against a peptide mapping to amino acids 973-987 of Maguin-1. Blue signals indicate nuclei stained with DAPI.

Table 1 lists the gene expression levels in the frontal cortex relative to the temporal cortex for the human MAGUIN-1 gene in seven Alzheimer's disease patients, herein identified by internal reference numbers P010, P011, P012, P014, P016, P017, P019 (1.42 to 4.14 fold) and five healthy, age-matched control individuals, herein identified by internal reference numbers C005, C008, C011, C012, C014 (0.30 to 1.36 fold). The values shown are reciprocal values according to the formula described herein.

Table 2 lists the gene expression levels in the frontal cortex relative to the hippocampus for the human MAGUIN-1 gene in six Alzheimer's disease patients, herein identified by internal reference numbers P010, P011, P012, P014, P016, P019 (1.21 to 3.07 fold) and three healthy, age-matched control individuals, herein identified by internal reference numbers C004, C005, C008 (0.39 to 1.74 fold). The values shown are reciprocal values according to the formula described herein.

Table 3 lists the gene expression levels in the frontal cortex relative to the temporal cortex for the human MAGUIN-2 gene in seven Alzheimer's disease patients, herein identified by internal reference numbers P010, P011, P012, P014, P016, P017, P019 (1.77 to 11.73 fold) and five healthy, age-matched control individuals, herein identified by internal reference numbers C005, C008, C011, C012, C014 (0.30 to 1.42 fold). The values shown are reciprocal values according to the formula described herein.

Table 4 lists the gene expression levels in the frontal cortex relative to the hippocampus for the human MAGUIN-2 gene in six Alzheimer's disease patients, herein identified by internal reference numbers P010, P011, P012, P014, P016, P019 (0.72 to 9.08 fold) and three healthy, age-matched control individuals, herein identified by internal reference numbers C004, C005, C008 (0.46 to 1.69 fold). The values shown are reciprocal values according to the formula described herein.

EXAMPLE I:

(i) Brain tissue dissection from patients with Alzheimer's disease:

Brain tissues from Alzheimer's disease patients and age-matched control subjects were collected within 6 hours post-mortem and immediately frozen on dry ice. Sample sections from each tissue were fixed in paraformaldehyde for histopathological confirmation of the diagnosis. Brain areas for differential expression analysis were identified (see Figure 1) and stored at - 80 °C until RNA extractions were performed.

(ii) Isolation of total RNA:

Total RNA was extracted from post-mortem brain tissue by using the RNeasy kit (Qiagen) according to the manufacturer's protocol. The accurate RNA concentration and the RNA quality were determined with the DNA LabChip system using the Agilent 2100 Bioanalyzer (Agilent Technologies). For additional quality testing of the prepared RNA, i.e. exclusion of partial degradation and testing for DNA contamination, specifically designed intronic GAPDH oligonucleotides and genomic DNA as reference control were utilised to generate a melting curve with the LightCycler technology as described in the manufacturer's protocol (Roche).

(iii) cDNA synthesis and identification of differentially expressed genes by

fluorescence differential display (FDD):

In order to identify changes in gene expression in different tissues we employed a modified and improved differential display (DD) screening method. The original DD screening method is known to those skilled in the art (Liang and Pardee, Science 1995, 267:1186-7). This technique compares two populations of RNA and provides clones of genes that are expressed in one population but not in the other. Several samples can be analyzed simultaneously and both up- and down-regulated genes can be identified in the same experiment. By adjusting and refining several steps in the DD method as well as modifying technical parameters, e.g. increasing redundancy, evaluating optimized reagents and conditions for reverse transcription of total RNA, optimizing polymerase chain reactions (PCR) and separation of the products thereof, a technique was developed which allows for highly reproducible and sensitive results. The applied and improved DD technique was described in detail by von der Kammer et al. (Nucleic Acids Research 1999, 27: 2211-2218). A set of 64 specifically designed random primers were developed (standard set) to achieve a statistically comprehensive analysis of all possible RNA species. Further, the method was modified to generate a preparative DD slab-gel technique, based on the use of fluorescently labelled primers. In the present invention, RNA populations from carefully selected post-mortem brain tissues (frontal and temporal cortex) of Alzheimer's disease patients and age-matched control subjects were compared.

As starting material for the DD analysis we used total RNA, extracted as described above (ii). Equal amounts of 0.05 μg RNA each were transcribed into cDNA in 20 μl reactions containing 0.5 mM each dNTP, 1 μl Sensiscript™ Reverse Transcriptase and 1x RT buffer (Qiagen), 10 U RNase inhibitor (Qiagen) and 1 μM of either one-base-anchor oligonucleotides $HT_{11}A$, $HT_{11}$ G or $HT_{11}C$ (Liang et al., Nucleic Acids Research 1994, 22: 5763-5764; Zhao et al., Biotechniques 1995, 18: 842-850). Reverse transcription was performed for 60 min at 37 °C with a final denaturation step at 93 °C for 5 min. 2 μl of the obtained cDNA each was subjected to a polymerase chain reaction (PCR) employing the corresponding one-base-anchor oligonucleotide (1 μM) along with either one of the Cy3 labelled random DD primers (1 μM), 1x GeneAmp PCR buffer (Applied Biosystems), 1.5 mM $MgCl_2$ (Applied Biosystems), 2 μM dNTP-Mix (dATP, dGTP, dCTP, dTTP Amersham Pharmacia Biotech), 5 % DMSO (Sigma), 1 U AmpliTaq DNA Polymerase (Applied Biosystems) in a 20 μl final volume. PCR conditions were set as follows: one round at 94 °C for 30 sec for denaturing, cooling 1 °C/sec down to 40 °C, 40 °C for 4 min for low-stringency annealing of primer, heating 1 °C/sec up to 72 °C, 72 °C for 1 min for extension. This round was followed by 39 high-stringency cycles: 94 °C for 30 sec, cooling 1 °C/sec down to 60 °C, 60 °C for 2 min, heating 1 °C/sec up to 72 °C, 72 °C for 1 min. One final step at 72 °C for 5 min was added to the last cycle (PCR cycler: Multi Cycler PTC 200, MJ Research). 8 μl DNA loading buffer were added to the 20 μl PCR product preparation, denatured for 5 min and kept on ice until loading onto a gel. 3.5 μl each were separated on 0.4 mm thick, 6 %-polyacrylamide (Long Ranger)/ 7 M urea sequencing gels in a slab-gel system (Hitachi Genetic Systems) at 2000 V, 60W, 30 mA, for 1 h 40 min. Following completion of the electrophoresis, gels were scanned with a FMBIO II fluorescence-scanner (Hitachi Genetic Systems), using the appropriate FMBIO II Analysis 8.0 software. A full-scale picture was printed, differentially expressed bands marked, excised from the gel, transferred into 1.5 ml containers, overlayed with 200 μl sterile water and kept at -20°C until extraction.

Elution and reamplification of differential display products: The differential bands were extracted from the gel by boiling in 200 μl $H_2O$ for 10 min, cooling down on ice and precipitation from the supernatant fluids by using ethanol (Merck) and glycogen/sodium acetate (Merck) at - 20 °C over night, and subsequent centrifugation at 13.000 rpm for 25 min at 4 °C. Pellets were washed twice in ice-cold ethanol (80%), resuspended in 10 mM Tris pH 8.3 (Merck) and dialysed against 10 % glycerol (Merck) for 1 h at room temperature on a 0.025 μm VSWP membrane (Millipore). The obtained preparations were used as templates for reamplification by 15 high-stringency cycles in 25-μl PCR mixtures containing the corresponding primer pairs as used for the differential display PCR (see above) under identical conditions, with the exception of the initial round at 94 °C for 5 min, followed by 15 cycles of: 94 °C for 45 sec, 60 °C for 45 sec, ramp 1°C/sec to 70 °C for 45 sec, and one final step at 72 °C for 5 min.

Cloning and sequencing of differential display products: Re-amplified cDNAs were analyzed with the DNA LabChip system (Agilent 2100 Bioanalyzer, Agilent Technologies) and were ligated into the pCR-Blunt II-TOPO vector and transformed into E.coli Top10F' cells (Zero Blunt TOPO PCR Cloning Kit, Invitrogen) according to the manufacturer's instructions. Cloned cDNA fragments were sequenced by commercially available sequencing facilities. The results of one such fluorescence differential display experiment for the human MAGUIN-1 gene are shown in Figure 2.

(iv) Confirmation of differential expression by quantitative RT-PCR:

Positive corroboration of differential expression of the human MAGUIN-1 gene and human MAGUIN-2 gene was performed using the LightCycler technology (Roche). This technique features rapid thermal cyling for the polymerase chain reaction as well as real-time measurement of fluorescent signals during amplification and therefore allows for highly accurate quantification of RT-PCR products by using a kinetic, rather than an endpoint approach. The ratios of human MAGUIN-1 and human MAGUIN-2 cDNA each from the temporal cortex and frontal cortex, and from the hippocampus and frontal cortex, respectively, were determined (relative quantification).

First, a standard curve was generated to determine the efficiency of the PCR with specific primers for human MAGUIN-1:

5'-CAGCAAGCAGTTGACGGGA-3'
and 5'-GCCACGAGTCTTGTCAAATTCA -3'
and human MAGUIN-2:
5'-GGGCCTCCCAAAGGGATAT-3'
and 5'-CCCAATGTAGAAAGCTCGCATT-3'.

PCR amplification (95 °C and 1 sec, 56 °C and 5 sec, and 72 °C and 5 sec) was performed in a volume of 20 μl containing Lightcycler-FastStart DNA Master SYBR Green I mix (contains FastStart Taq DNA polymerase, reaction buffer, dNTP mix with dUTP instead of dTTP, SYBR Green I dye, and 1 mM $MgCl_2$; Roche), 0.5 μM primers, 2 μl of a cDNA dilution series (final concentration of 40, 20, 10, 5, 1 and 0.5 ng human total brain cDNA; Clontech) and depending on the primers used, additional 3 mM $MgCl_2$-Melting curve analysis revealed a single peak at approximately 80°C and 80.6°C with no visible primer dimers. Quality and size of the PCR product were determined with the DNA LabChip system (Agilent 2100 Bioanalyzer, Agilent Technologies). A single peak at the expected size of 121 bp for human MAGUIN-1 and 67 bp for human MAGUIN-2 was observed in the electropherogram of the sample.

In an analogous manner, the PCR protocol was applied to determine the PCR efficiency of a set of reference genes which were selected as a reference standard for quantification. In the present invention, the mean value of five such reference genes was determined: (1) cyclophilin B, using the specific primers 5'-ACTGAAGCACTACGGGCCTG-3' and 5'-AGCCGTTGGTGTCTTTGCC-3' except for $MgCl_2$ (an additional 1 mM was added instead of 3 mM). Melting curve analysis revealed a single peak at approximately 87 °C with no visible primer dimers. Agarose gel analysis of the PCR product showed one single band of the expected size (62 bp). (2) Ribosomal protein S9 (RPS9), using the specific primers 5'-GGTCAAATTTACCCTGGCCA-3' and 5'- TCTCATCAAGCGTCAGCAGTTC-3' (exception: additional 1 mM $MgCl_2$ was added instead of 3 mM). Melting curve analysis revealed a single peak at approximately 85°C with no visible primer dimers. Agarose gel analysis of the PCR product showed one single band with the expected size (62 bp). (3) beta-actin, using the specific primers 5'-TGGAACGGTGAAGGTGACA-3' and 5'-GGCAAGGGACTTCCTGTAA-3'. Melting curve analysis revealed a single peak at approximately 87°C with no visible primer dimers. Agarose gel analysis of the PCR product showed one single band with the expected size (142 bp). (4) GAPDH, using the specific primers 5'-CGTCATGGGTGTGAACCATG-3' and 5'-GCTAAGCAGTTGGTGGTGCAG-3'. Melting curve analysis revealed a single peak at approximately 83°C with no visible primer dimers. Agarose gel analysis of the PCR product showed one single band with the expected size (81 bp). (5) Transferrin receptor TRR, using the specific primers 5'-GTCGCTGGTCAGTTCGTGATT-3' and 5'-AGCAGTTGGCTGTTGTACCTCTC-3'. Melting curve analysis revealed a single peak at approximately 83°C with no visible primer dimers. Agarose gel analysis of the PCR product showed one single band with the expected size (80 bp).

For calculation of the values, first the logarithm of the cDNA concentration was plotted against the threshold cycle number $C_t$ for human MAGUIN-1 and human MAGUIN-2, respectively, and the five reference standard genes. The slopes and the intercepts of the standard curves (i.e. linear regressions) were calculated for all genes. In a second step, cDNAs from temporal cortex and frontal cortex, and from hippocampus and frontal cortex, for human MAGUIN-1 and human MAGUIN-2, respectively, were analyzed in parallel and normalized to cyclophilin B. The $C_t$ values were measured and converted to ng total brain cDNA using the corresponding standard curves:

$$10 \wedge ( (C_t \text{ value - intercept}) / \text{slope} ) \qquad [\text{ng total brain cDNA}]$$

The values for temporal cortex and frontal cortex of human MAGUIN-1 and human MAGUIN-2 cDNAs, and the values for hippocampus and frontal cortex of human MAGUIN-1 and human MAGUIN-2 cDNAs, respectively, were normalized to cyclophilin B, and the ratios were calculated using the following formulas:

$$\text{Ratio} = \frac{\text{human MAGUIN-1(MAGUIN-2) temporal [ng] / cyclophilin B temporal [ng]}}{\text{human MAGUIN-1(MAGUIN-2) frontal [ng] / cyclophilin B frontal [ng]}}$$

$$\text{Ratio} = \frac{\text{human MAGUIN-1(MAGUIN-2) hippocampus [ng] / cyclophilin B hippocampus [ng]}}{\text{human MAGUIN-1(MAGUIN-2) frontal [ng] / cyclophilin B frontal [ng]}}$$

In a third step, the set of reference standard genes was analyzed in parallel to determine the mean average value of the temporal to frontal ratios, and of the hippocampal to frontal ratios, respectively, of expression levels of the reference standard genes for each individual brain sample. As cyclophilin B was analyzed in step 2 and step 3, and the ratio from one gene to another gene remained constant in different runs, it was possible to normalize the values for human MAGUIN-1 and human MAGUIN-2 to the mean average value of the set of reference standard genes instead of normalizing to one single gene alone. The calculation was performed by dividing the ratio shown above by the deviation of cyclophilin B from the mean value of all housekeeping genes. The results of such quantitative RT-PCR analysis for the human MAGUIN-1 gene are shown in Figure 3 and 4 and for the human MAGUIN-2 gene in Figure 5 and 6.

(v) <u>Sequence Analysis</u>

Searching the EST database of the GenBank database for sequence similarities to the identified differentially expressed human cDNA fragment, SEQ ID NO. 7, as stated in the present invention, it was found that SEQ ID NO. 7 was identical to portions of the human EST sequences ai817268 and bf115709, (shown in Figure 17). These human ESTs showed high homology to rat *(Rattus norvegicus)* MAGUIN-1. Aligning human ESTs in addition to the identified expressed SEQ ID NO. 7, a complete EST cluster representing the human MAGUIN-1 cDNA, SEQ ID NO. 5, was constructed. The amino acid sequence of a large open reading frame with the potential to encode a protein of 1034 amino acid residues was deduced (SEQ ID NO. 1). The human MAGUIN-1 protein, as denoted herein, is highly homologous to the rat MAGUIN-1 protein. In addition, it encodes a protein (SEQ ID NO. 1) harboring a number of structurally and functionally important domains. One SAM, one PDZ, and one PH domain are located from amino acid residues 8 to 75 (SAM), amino acid residues 156 to 296 (PDZ), and amino acid residues 572 to 667 (PH) (refer to Figure 7).

(vi) <u>Immunohistochemistry:</u>

For immunofluorescence staining of Maguin-1 in human brain, frozen sections were prepared from post-mortem pre-central gyrus of a donor person (Cryostat Leica CM3050S) and fixed in acetone for 10 min. After washing in PBS, the sections were preincubated with blocking buffer (10% normal goat serum, 0.2% Triton X-100 in PBS) for 30min, and then incubated with affinity-purified rabbit anti-Maguin-1 antisera (1:20-1:50 diluted in blocking buffer, custom-made by Davids Biotechnologie, Regensburg) overnight at 4°C. After rinsing three times in 0.2% Triton X-100/PBS, the sections were incubated with FITC-conjugated goat anti-rabbit IgG (1:150 diluted in 1% BSA/PBS) for 2 hours at room temperature, and then again washed in PBS. Staining of the nuclei was performed by incubation of the sections with 5$\mu$M DAPI in PBS for 3min (blue signal). In order to block the autofluoresence of lipofuscin in human brain, the sections were treated with 1% Sudan Black B in 70% ethanol for 2-10 min at room temperature, sequentially dipped in 70% ethanol, destilled water and PBS. The sections were coverslipped by 'Vectrashield mounting medium' (Vector Laboratories, Burlingame, CA) and observed under an inverted microscope (IX81, Olympus Optical). The digital images were captured with the appropriate software (AnalySiS, Olympus Optical).

## Claims

**1.** An in vitro method of diagnosing Alzheimer's disease in a subject comprising:

determining a level of

(i) a transcription product of a gene coding for human MAGUIN-1 and/or human MAGUIN-2, and/or
(ii) a translation product of a gene coding for human MAGUIN-1 and/or human MAGUIN-2,

in a brain sample from said subject and comparing said level to a reference value representing a known disease or health status, thereby diagnosing said disease in said subject.

**2.** An in vitro method of monitoring the progression of Alzheimer's disease in a subject, comprising:

determining a level of

(i) a transcription product of a gene coding for human MAGUIN-1 and/or human MAGUIN-2, and/or
(ii) a translation product of a gene coding for human MAGUIN-1 and/or human MAGUIN-2,

in a brain sample from said subject and comparing said level to a reference value representing a known disease or health status, thereby monitoring the progression of said disease in said subject.

**3.** An in vitro method of evaluating a treatment for Alzheimer's disease, comprising:

determining a level of

(i) a transcription product of a gene coding for human MAGUIN-1 and/or human MAGUIN-2, and/or
(ii) a translation product of a gene coding for human MAGUIN-1 and/or human MAGUIN-2,

in a brain sample from a subject being treated for said disease and comparing said level to a reference value representing a known disease or health status, thereby evaluating said treatment for said disease.

**4.** The method according to any of claims 1 to 3 wherein said sample is a cell, or a tissue.

**5.** The method according to any of claims 1 to 4 wherein said reference value is that of a level of

(i) a transcription product of a gene coding for human MAGUIN-1 and/or human MAGUIN-2, and/or
(ii) a translation product of a gene coding for human MAGUIN-1 and/or human MAGUIN-2,

in a sample from a subject not suffering from Alzheimer's disease.

**6.** The method according to any of claims 1 to 5 wherein an alteration in the level of a transcription product of the gene coding for human MAGUIN-1 and/or human MAGUIN-2 and/or a translation product of the gene coding for human MAGUIN-1 and/or human MAGUIN-2, in a sample cell, or tissue from said subject relative to a reference value representing a known health status indicates a diagnosis of Alzheimer's disease in said subject.

**7.** The method according to any of claims 1 to 6, further comprising comparing a level of

(i) a transcription product of a gene coding for human MAGUIN-1 and/or human MAGUIN-2, and/or
(ii) a translation product of a gene coding for human MAGUIN-1 and/or human MAGUIN-2,

in a series of samples taken from said subject over a period of time.

**8.** Use of a kit in a method according to any one of claims 1-7, said kit comprising:

(a) at least one reagent which is selected from the group consisting of

(i) reagents that selectively detect a transcription product of a gene coding for human MAGUIN-1 and/or human MAGUIN-2, and
(ii) antibodies that selectively detect a translation product of a gene coding for human MAGUIN-1 and/or human MAGUIN-2 and

(b) an instruction for diagnosing Alzheimer's disease, by

(i) detecting a level of said transcription product and/or said translation product of a gene coding for human MAGUIN-1 and/or human MAGUIN-2 in a brain sample from said subject; and
(ii) diagnosing Alzheimer's disease,

wherein a varied level of said transcription product and/or said translation product compared to a reference value representing a known health status; or a level, of said transcription product and/or said translation product similar or equal to a reference value representing a known disease status indicates a diagnosis of Alzheimer's disease.

**9.** Use of a recombinant, non-human animal comprising a non-native gene sequence coding for human MAGUIN-1 and/or human MAGUIN-2 for screening, testing and validating compounds, agents and modulators in the development of diagnostics and therapeutics to treat Alzheimer's disease.

**10.** An in vitro method for screening for a modulator of Alzheimer's disease said method comprising:

(a) contacting a cell with a test compound;
(b) measuring the level of one or more substances from the group consisting of

(i) a gene coding for human MAGUIN-1 and/or human MAGUIN-2,
(ii) a transcription product of a gene coding for human MAGUIN-1 and/or human MAGUIN-2, and
(iii) a translation product of a gene coding for human MAGUIN-1 and/or human MAGUIN-2;

(c) measuring the level of one or m ore substances recited in (i) to (iii) i n a control cell not contacted with said test compound; and
(d) comparing the levels of the substance in the cells of step (b) and (c), wherein an alteration in the level of substances in the contacted cells indicates that the test compound is a modulator of said diseases.

**11.** A protein molecule shown in SEQ ID NO.1 or SEQ ID NO.2 for use as a diagnostic target for detecting Alzheimer's disease.

**12.** Use of an antibody specifically immunoreactive with a protein molecule according to SEQ ID No. 1 or SEQ ID No. 2, for detecting the pathological state of a cell in a brain sample from a subject, comprising immunocytochemical staining of said cell with said antibody, wherein an altered degree of staining, or an altered staining pattern in said cell compared to a cell representing a known health status indicates a pathological state of said cell, and wherein further said pathological state relates to Alzheimer's disease.

## Patentansprüche

**1.** In-vitro-Verfahren zum Diagnostizieren von Alzheimer-Krankheit bei einem Patienten, umfassend:

Bestimmen einer Konzentration von:

(i) einem Transcriptionsprodukt eines Gens, das für humanes MAGUIN-1 und/oder humanes MAGUIN-2 codiert; und/oder
(ii) einem Translationsprodukt eines Gens, das für humanes MAGUIN-1 und/oder humanes MAGUIN-2 codiert;

in einer Gehirnprobe von dem Patienten und Vergleichen der Konzentration mit einem Referenzwert, der einen bekannten Krankheits- oder Gesundheitszustand darstellt, wodurch die Krankheit bei dem Patienten diagnostiziert wird.

**2.** In-vitro-Verfahren zur Überwachung des Fortschritts von Alzheimer-Krankheit bei einem Patienten, umfassend:

Bestimmen einer Konzentration von:

(i) einem Transcriptionsprodukt eines Gens, das für humanes MAGUIN-1 und/oder humanes MAGUIN-2 codiert; und/oder
(ii) einem Translationsprodukt eines Gens, das für humanes MAGUIN-1 und/oder humanes MAGUIN-2 codiert;

in einer Gehirnprobe von dem Patienten und Vergleichen der Konzentration mit einem Referenzwert, der einen bekannten Krankheits- oder Gesundheitszustand darstellt, wodurch der Fortschritt der Krankheit bei dem Patienten überwacht wird.

**3.** In-vitro-Verfahren zur Bewertung einer Behandlung auf Alzheimer-Krankheit, umfassend:

Bestimmen einer Konzentration von:

(i) einem Transcriptionsprodukt eines Gens, das für humanes MAGUIN-1 und/oder humanes MAGUIN-2 codiert; und/oder

(ii) einem Translationsprodukt eines Gens, das für humanes MAGUIN-1 und/oder humanes MAGUIN-2 codiert;

in einer Gehirnprobe von einem Patienten, der auf die Krankheit behandelt wird, und Vergleichen der Konzentration mit einem Referenzwert, der einen bekannten Krankheits- oder Gesundheitszustand darstellt, wodurch die Behandlung der Krankheit bewertet wird.

**4.** Verfahren gemäß einem der Ansprüche 1 bis 3, wobei die Probe eine Zelle oder ein Gewebe ist.

**5.** Verfahren gemäß einem der Ansprüche 1 bis 4, wobei der Referenzwert der Referenzwert einer Konzentration von

(i) einem Transcriptionsprodukt eines Gens, das für humanes MAGUIN-1 und/oder humanes MAGUIN-2 codiert; und/oder
(ii) einem Translationsprodukt eines Gens, das für humanes MAGUIN-1 und/oder humanes MAGUIN-2 codiert;

in einer Probe von einem Patienten, der nicht an Alzheimer-Krankheit leidet, ist.

**6.** Verfahren gemäß einem der Ansprüche 1 bis 5, wobei eine Veränderung in der Konzentration eines Transcriptionsprodukts des Gens, das für humanes MAGUIN-1 und/oder humanes MAGUIN-2 codiert, und/oder eines Translationsprodukts des Gens, das für humanes MAGUIN-1 und/oder humanes MAGUIN-2 codiert, in einer Probenzelle oder einem Probengewebe von dem Patienten relativ zu einem Referenzwert, der einen bekannten Gesundheitszustand darstellt, eine Diagnose von Alzheimer-Krankheit bei dem Patienten anzeigt.

**7.** Verfahren gemäß einem der Ansprüche 1 bis 6, das weiterhin das Vergleichen einer Konzentration von

(i) einem Transcriptionsprodukt eines Gens, das für humanes MAGUIN-1 und/oder humanes MAGUIN-2 codiert; und/oder
(ii) einem Translationsprodukt eines Gens, das für humanes MAGUIN-1 und/oder humanes MAGUIN-2 codiert;

in einer Reihe von Proben, die im Laufe der Zeit von dem Patienten genommen wurden, umfasst.

**8.** Verwendung eines Kits bei einem Verfahren gemäß einem der Ansprüche 1 bis 7, wobei der Kit Folgendes umfasst:

(a) wenigstens ein Reagens, das aus der Gruppe ausgewählt ist, die aus Folgenden besteht:

(i) Reagentien, die selektiv ein Transcriptionsprodukt eines Gens, das für humanes MAGUIN-1 und/oder humanes MAGUIN-2 codiert, nachweisen; und
(ii) Antikörper, die selektiv ein Translationsprodukt eines Gens, das für humanes MAGUIN-1 und/oder humanes MAGUIN-2 codiert, nachweisen; und

(b) eine Anweisung zum Diagnostizieren von Alzheimer-Krankheit durch

(i) Bestimmen einer Konzentration des Transcriptionsprodukts und/oder des Translationsprodukts eines Gens, das für humanes MAGUIN-1 und/oder humanes MAGUIN-2 codiert, in einer Gehirnprobe von dem Patienten; und
(ii) Diagnostizieren von Alzheimer-Krankheit;

wobei eine veränderte Konzentration des Transcriptionsprodukts und/oder des Translationsprodukts im Vergleich zu einem Referenzwert, der einen bekannten Gesundheitszustand darstellt, oder eine Konzentration des Transcriptionsprodukts und/oder des Translationsprodukts, die ähnlich oder gleich einem Referenzwert ist, der einen bekannten Krankheitszustand darstellt, auf eine Diagnose von Alzheimer-Krankheit hinweist.

**9.** Verwendung eines rekombinanten nichthumanen Tiers, das eine nichtnative Gensequenz umfasst, die für humanes MAGUIN-1 und/oder humanes MAGUIN-2 codiert, zum Durchmustern, Testen und Bewerten von Verbindungen, Mitteln und Modulatoren bei der Entwicklung von Diagnostika und Therapeutika zur Behandlung von Alzheimer-Krankheit.

**10.** In-vitro-Verfahren zum Suchen nach einem Modulator von Alzheimer-Krankheit, wobei das Verfahren Folgendes umfasst:

a) In-Kontakt-Bringen einer Zelle mit einer Testverbindung;
b) Messen der Konzentration von einer oder mehreren Substanzen aus der Gruppe, die aus Folgenden besteht:

(i) einem Gen, das für humanes MAGUIN-1 und/oder humanes MAGUIN-2 codiert;
(ii) einem Transcriptionsprodukt eines Gens, das für humanes MAGUIN-1 und/oder humanes MAGUIN-2 codiert; und
(iii) einem Translationsprodukt eines Gens, das für humanes MAGUIN-1 und/oder humanes MAGUIN-2 codiert;

c) Messen der Konzentration von einer oder mehreren Substanzen, die in (i) bis (iii) genannt sind, in einer Kontrollzelle, die nicht mit der Testverbindung in Kontakt gebracht wurde; und
d) Vergleichen der Konzentrationen der Substanz in den Zellen der Schritte (b) und (c), wobei eine Änderung der Konzentration der Substanzen in den kontaktierten Zellen anzeigt, dass die Testverbindung ein Modulator der Krankheiten ist.

**11.** Proteinmolekül, das in SEQ ID Nr. 1 oder SEQ ID Nr. 2 gezeigt ist, zur Verwendung als diagnostisches Target zum Nachweisen von Alzheimer-Krankheit.

**12.** Verwendung eines Antikörpers, der spezifisch immunreaktiv gegenüber einem Proteinmolekül gemäß SEQ ID Nr. 1 oder SEQ ID Nr. 2 ist, zum Nachweisen des pathologischen Zustands einer Zelle in einer Gehirnprobe von einem Patienten, das das immuncytochemische Anfärben der Zelle mit dem Antikörper umfasst, wobei ein veränderter Grad der Anfärbung oder ein verändertes Anfärbungsmuster in der Zelle im Vergleich zu einer Zelle, die einen bekannten Gesundheitszustand repräsentiert, einen pathologischen Zustand der Zelle anzeigt und wobei sich der pathologische Zustand weiterhin auf Alzheimer-Krankheit bezieht.

## Revendications

**1.** Procédé *in vitro* de diagnostic de la maladie d'Alzheimer chez un sujet comprenant les étapes consistant à :

déterminer une quantité

(i) d'un produit de transcription d'un gène codant pour MAGUIN-1 humain et/ou MAGUIN-2 humain, et/ou
(ii) d'un produit de traduction d'un gène codant pour MAGUIN-1 humain et/ou MAGUIN-2 humain,

dans un prélèvement de cerveau provenant dudit sujet, et comparer ladite quantité à une valeur de référence représentant un état pathologique ou non pathologique, diagnostiquant ainsi ladite maladie chez ledit sujet.

**2.** Procédé *in vitro* de surveillance de la progression de la maladie d'Alzheimer chez un sujet, comprenant les étapes consistant à :

déterminer une quantité

(i) d'un produit de transcription d'un gène codant pour MAGUIN-1 humain et/ou MAGUIN-2 humain, et/ou
(ii) d'un produit de traduction d'un gène codant pour MAGUIN-1 humain et/ou MAGUIN-2 humain,

dans un prélèvement de cerveau provenant dudit sujet, et comparer ladite quantité à une valeur de référence représentant un état pathologique ou non pathologique, surveillant ainsi la progression de ladite maladie chez ledit sujet.

**3.** Procédé in vitro d'évaluation d'un traitement concernant la maladie d'Alzheimer, comprenant les étapes consistant à :

déterminer une quantité

(i) d'un produit de transcription d'un gène codant pour MAGUIN-1 humain et/ou MAGUIN-2 humain, et/ou
(ii) d'un produit de traduction d'un gène codant pour MAGUIN-1 humain et/ou MAGUIN-2 humain,

dans un prélèvement de cerveau provenant d'un sujet traité pour ladite maladie et comparer ladite quantité à une valeur de référence représentant un état pathologique ou non pathologique, évaluant ainsi ledit traitement pour ladite maladie.

**4.** Procédé selon l'une quelconque des revendications 1 à 3, dans lequel ledit prélèvement est une cellule ou un tissu.

**5.** Procédé selon l'une quelconque des revendications 1 à 4, dans lequel ladite valeur de référence est celle correspondant à une quantité

(i) d'un produit de transcription d'un gène codant pour MAGUIN-1 humain et/ou MAGUIN-2 humain, et/ou
(ii) d'un produit de traduction d'un gène codant pour MAGUIN-1 humain et/ou MAGUIN-2 humain,

dans un prélèvement provenant d'un sujet ne souffrant pas de la maladie d'Alzheimer.

**6.** Procédé selon l'une quelconque des revendications 1 à 5, dans lequel la modification de la quantité d'un produit de transcription du gène codant pour MAGUIN-1 humain et/ou MAGUIN-2 humain et/ou d'un produit de traduction du gène codant pour MAGUIN-1 humain et/ou MAGUIN-2 humain, dans un prélèvement de cellule ou de tissu provenant dudit sujet par rapport à une valeur de référence représentant un état non pathologique connu, indique un diagnostic de la maladie d'Alzheimer chez ledit sujet.

**7.** Procédé selon l'une quelconque des revendications 1 à 6, comprenant en outre l'étape consistant à comparer une quantité

(i) d'un produit de transcription d'un gène codant pour MAGUIN-1 humain et/ou MAGUIN-2 humain, et/ou
(ii) d'un produit de traduction d'un gène codant pour MAGUIN-1 humain et/ou MAGUIN-2 humain,

dans une série de prélèvements provenant dudit sujet pendant une certaine durée.

**8.** Utilisation d'un kit dans un procédé selon l'une quelconque des revendications 1 à 7, ledit kit comprenant :

(a) au moins un réactif qui est choisi dans le groupe constitué par

(i) des réactifs qui détectent sélectivement un produit de transcription d'un gène codant pour MAGUIN-1 humain et/ou MAGUIN-2 humain, et
(ii) des anticorps qui détectent sélectivement un produit de traduction d'un gène codant pour MAGUIN-1 humain et/ou MAGUIN-2 humain, et

(b) une instruction pour diagnostiquer la maladie d'Alzheimer, en

(i) détectant une quantité dudit produit de transcription et/ou dudit produit de traduction d'un gène codant pour MAGUIN-1 humain et/ou MAGUIN-2 humain dans un prélèvement de cerveau provenant dudit sujet ; et
(ii) diagnostiquant la maladie d'Alzheimer,

dans laquelle une quantité différente dudit produit de transcription et/ou dudit produit de traduction par rapport à une valeur de référence représentant un état non pathologique connu ; ou une quantité dudit produit de transcription et/ou dudit produit de traduction similaire ou égale à une valeur de référence représentant un état pathologique connu, indique un diagnostic de la maladie d'Alzheimer.

**9.** Utilisation d'un animal non humain recombiné comprenant une séquence de gènes non naturelle codant pour MAGUIN-1 humain et/ou MAGUIN-2 humain en vue de dépister, analyser et valider des composés, agents et modulateurs dans l'élaboration de diagnostics et de médicaments pour traiter la maladie d'Alzheimer.

**10.** Procédé *in vitro* de dépistage d'un modulateur de la maladie d'Alzheimer, ledit procédé comprenant les étapes consistant à :

(a) mettre en contact une cellule avec un composé d'essai ;
(b) mesurer la quantité d'une ou plusieurs substances dans le groupe constitué

(i) d'un gène codant pour MAGUIN-1 humain et/ou MAGUIN-2 humain,
(ii) d'un produit de transcription d'un gène codant pour MAGUIN-1 humain et/ou MAGUIN-2 humain, et
(iii) d'un produit de traduction d'un gène codant pour MAGUIN-1 humain et/ou MAGUIN-2 humain ;

(c) mesurer la quantité d'une ou plusieurs substances décrites de (i) à (iii) dans une cellule témoin n'ayant pas été mise en contact avec ledit composé d'essai ; et
(d) comparer les quantités de la substance dans les cellules des étapes (b) et (c), où une modification de la quantité de substances dans les cellules en contact indique que le composé d'essai est un modulateur desdites maladies.

**11.** Molécule protéique représentée dans SEQ ID N° 1 ou SEQ ID N° 2 en vue d'une utilisation en tant que diagnostic cible pour détecter la maladie d'Alzheimer.

**12.** Utilisation d'un anticorps spécifiquement immunoréactif avec une molécule protéique selon SEQ ID N° 1 ou SEQ ID N° 2, pour détecter l'état pathologique d'une cellule dans un prélèvement de cerveau provenant d'un sujet, comprenant les étapes consistant à colorer immunocytochimiquement ladite cellule avec ledit anticorps, où le degré de coloration modifié ou d'un profil de coloration modifié dans ladite cellule par rapport à une cellule représentant un état non pathologique connu, indique un état pathologique de ladite cellule, et où, de surcroît, ledit état pathologique concerne la maladie d'Alzheimer.

Fig. 1: Identification of Genes Involved in Alzheimer's Disease Pathology

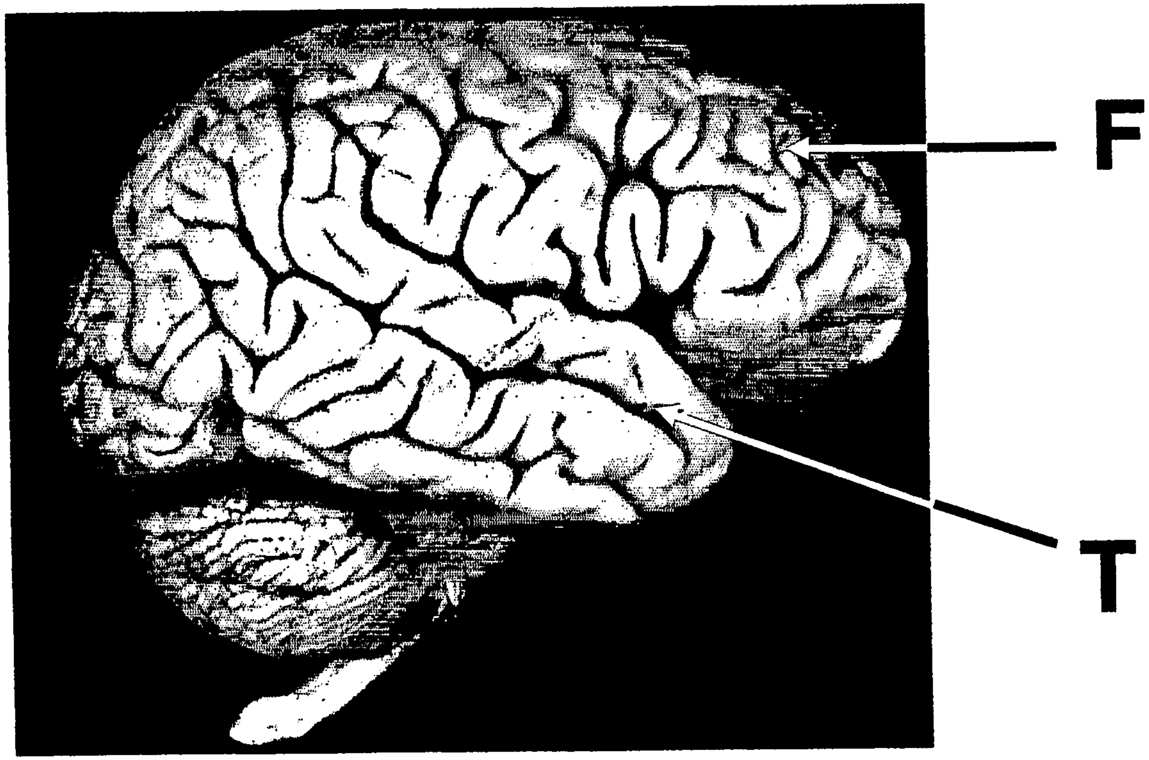

Fig. 2: Identification of differentially expressed genes in a fluorescence differential display screen

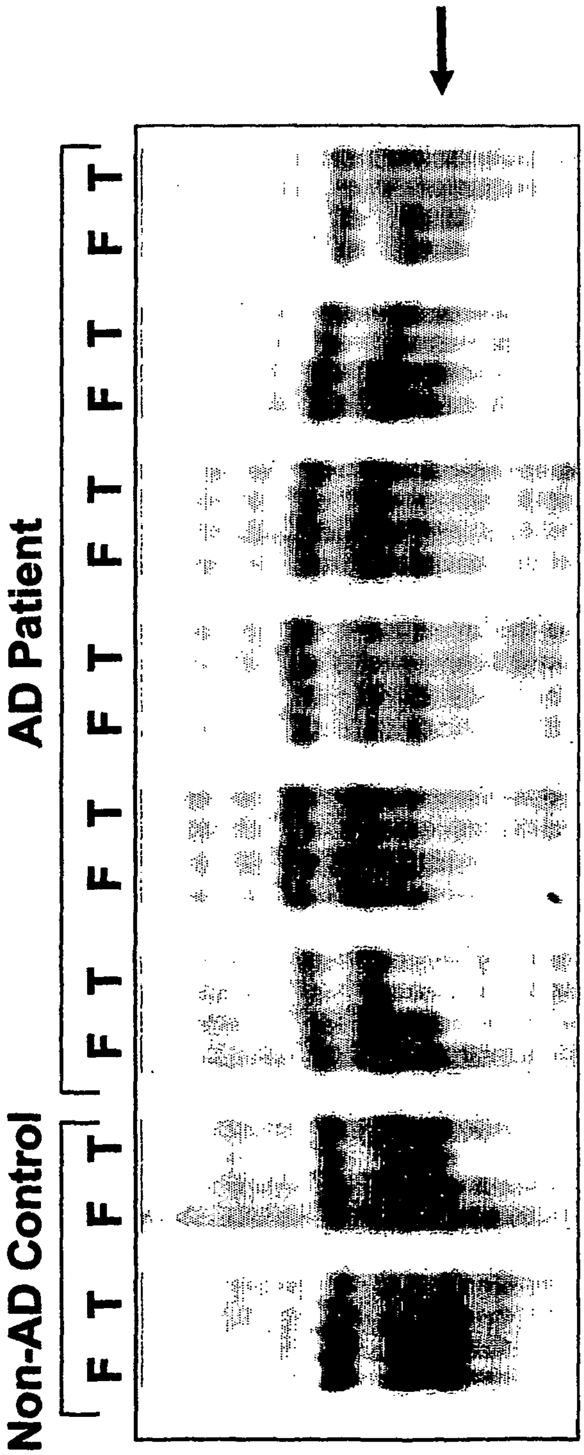

**Fig. 3: Verification of differential expression of human MAGUIN-1 by quantitative RT-PCR**

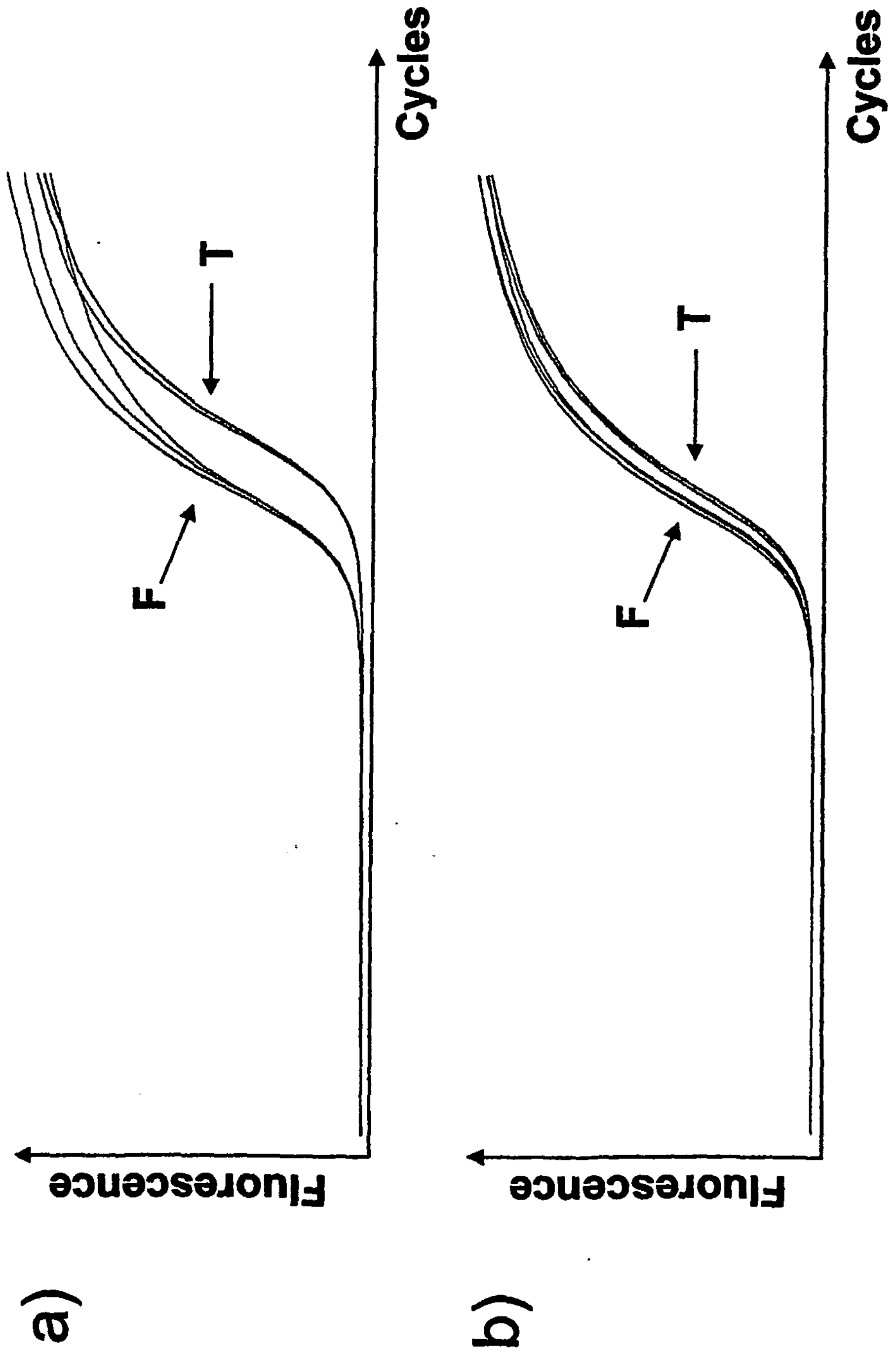

**Fig. 4: Verification of differential expression of Maguin-1 by quantitative RT-PCR**

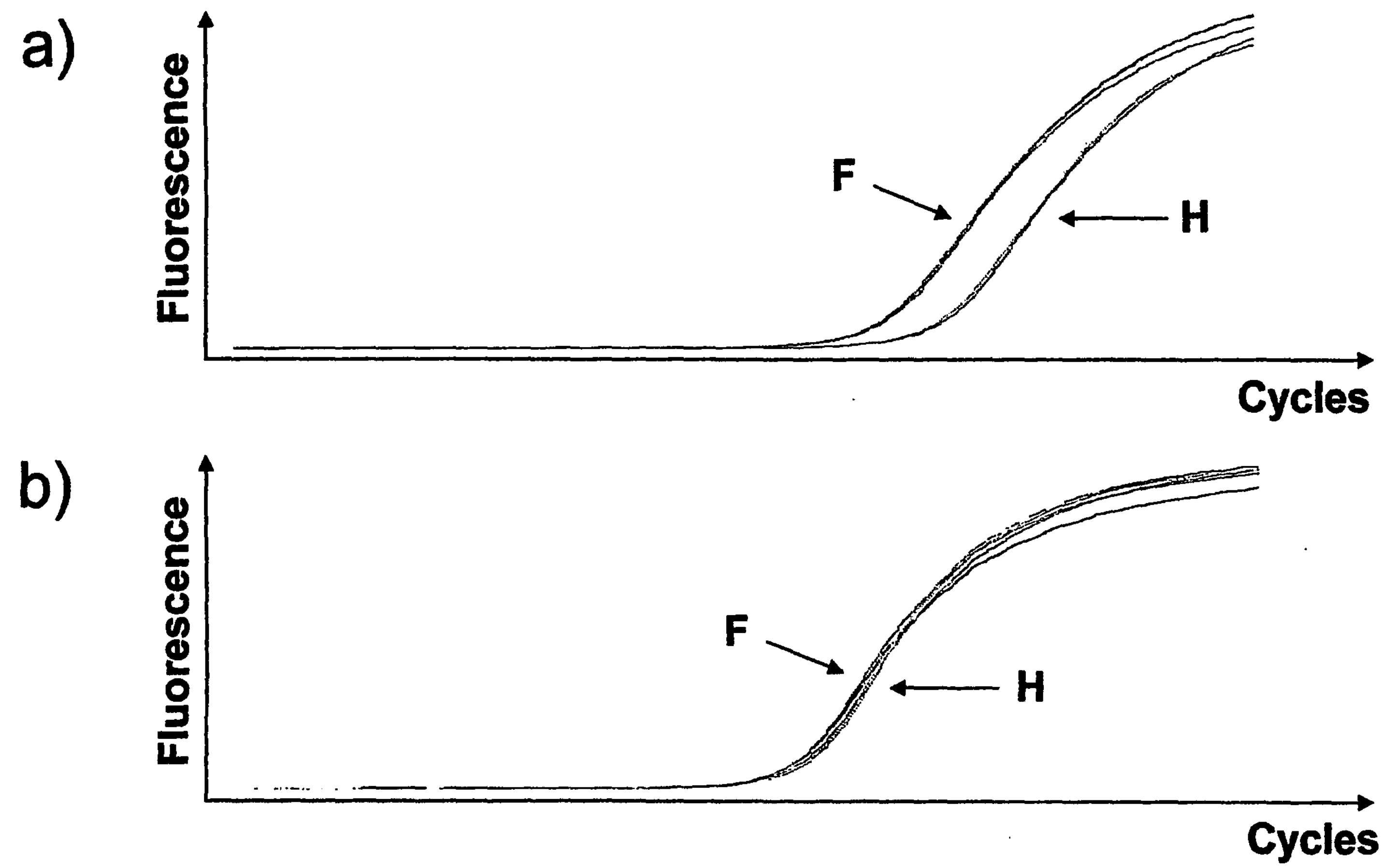

**Fig. 5: Verification of differential expression of human MAGUIN-2 by quantitative RT-PCR**

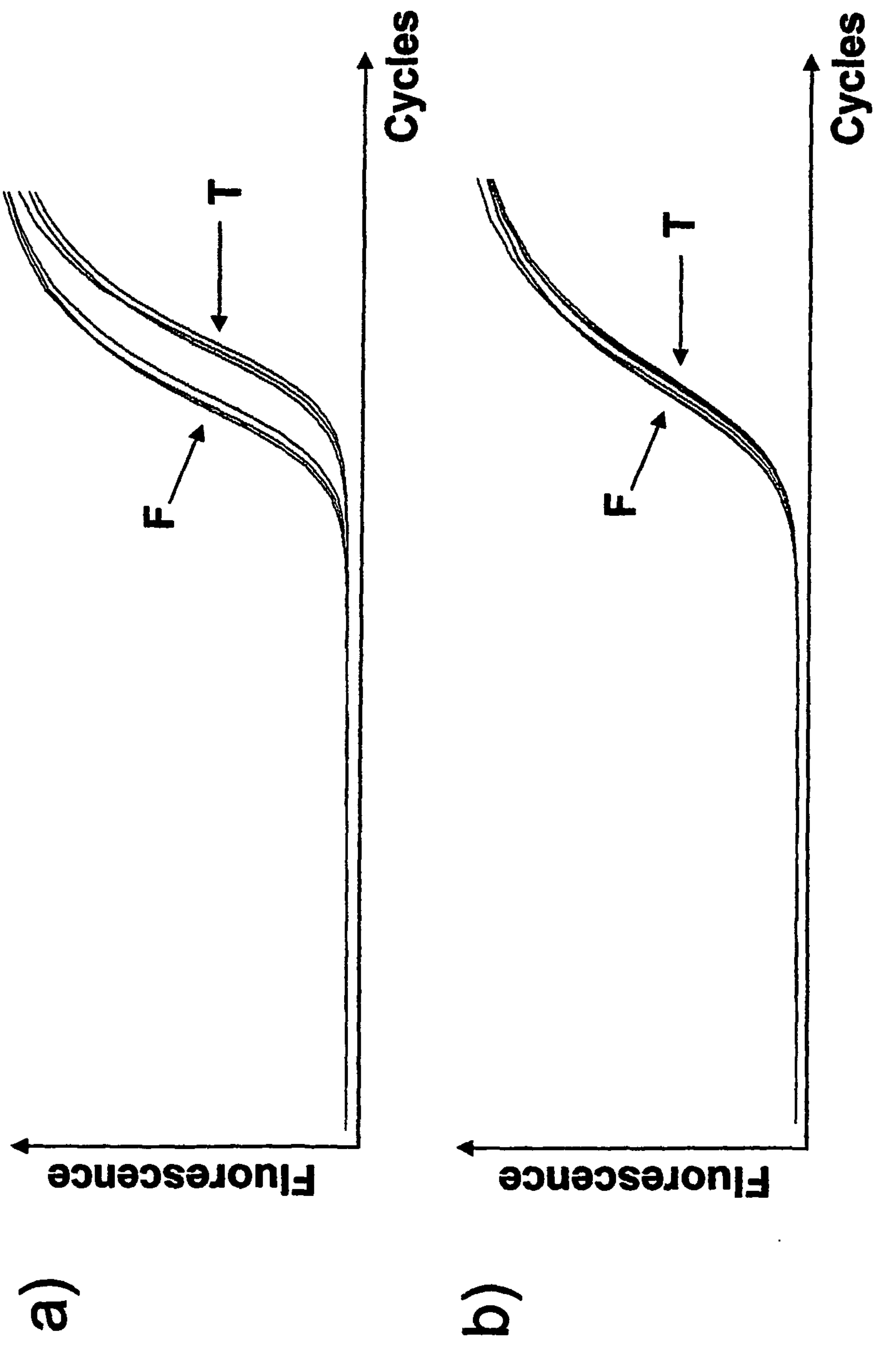

**Fig. 6: Verification of differential expression of Maguin-2 by quantitative RT-PCR**

a)

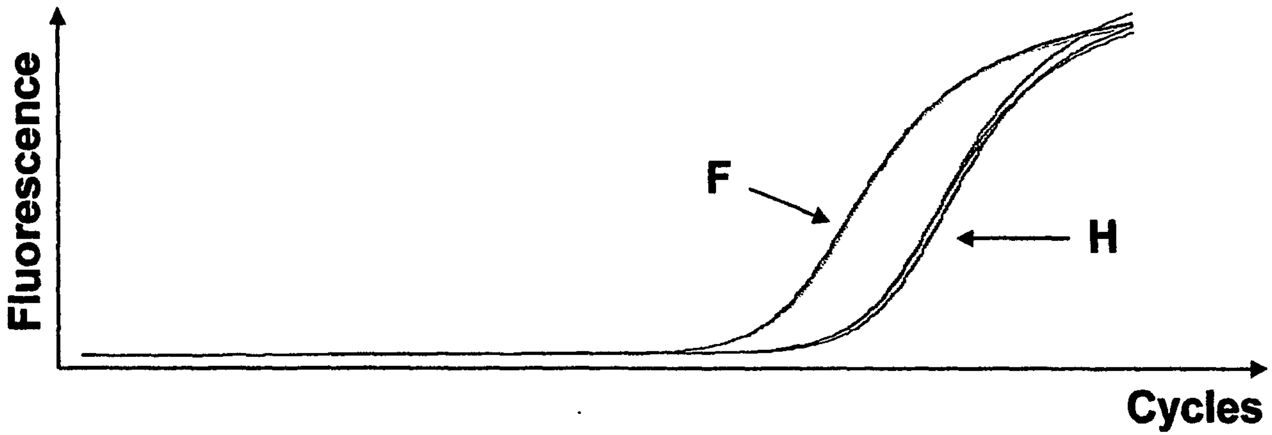


b)

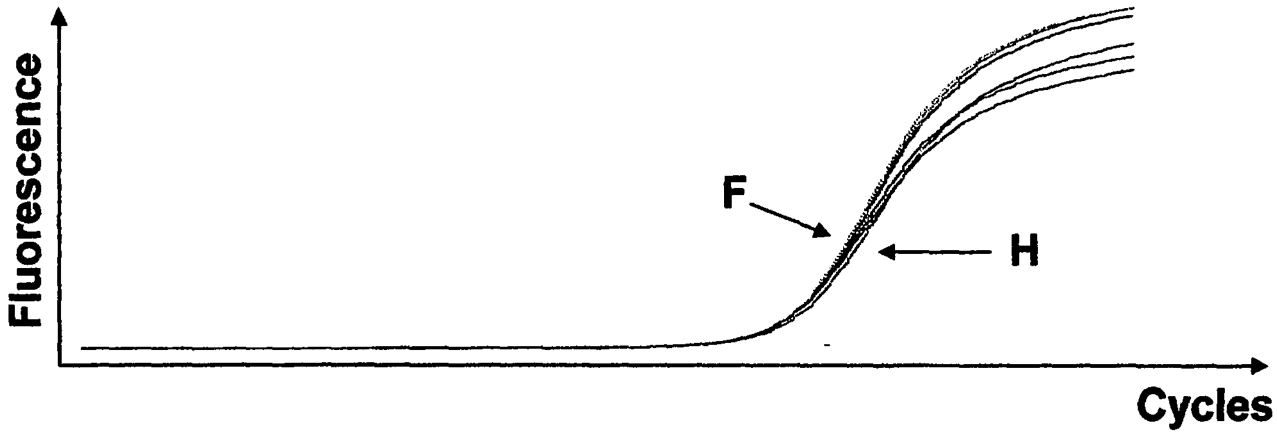

# Fig. 7: SEQ ID NO. 1: amino acid sequence of human MAGUIN-1 protein

**Length: 1034 aa**

1 MALIMEPVSK WSPSQVVDWM KGLDDCLQQY IKNFEREKIS GDQLLRITHQ

51 ELEDLGVSRI GHQELILEAV DLLCALNYGL ETENLKTLSH KLNASAKNLQ

101 NFITGRRRSG HYDGRTSRKL PNDFLTSVVD LIGAAKSLLA WLDRSPFAAV

151 TDYSVTRNNV IQLCLELTTI VQQDCTVYET ENKILHVCKT LSGVCDHIIS

201 LSSDPLVSQS AHLEVIQLAN IKPSEGLGMY IKSTYDGLHV ITGTTENSPA

251 DRCKKIHAGD EVIQVNHQTV VGWQLKNLVN ALREDPSGVI LTLKKRPQSM

301 LTSAPALLKN MRWKPLALQP LIPRSPTSSV ATPSSTISTP TKRDSSALQD

351 LYIPPPPAEP YIPRDEKGNL PCEDLRGHMV GKPVHKGSES PNSFLDQEYR

401 KRFNIVEEDT VLYCYEYEKG RSSSQGRRES TPTYGKLRPI SMPVEYNWVG

451 DYEDPNKMKR DSRRENSLLR YMSNEKIAQE EYMFQRNSKK DTGKKSKKKG

501 DKSNSPTHYS LLPSLQMDAL RQDIMGTPVP ETTLYHTFQQ SSLQHKSKKK

551 NKGPIAGKSK RRISCKDLGR GDCEGWLWKK KDAKSYFSQK WKKYWFVLKD

601 ASLYWYINEE DEKAEGFISL PEFKIDRASE CRKKYAFKAC HPKIKSFYFA

651 AEHLDDMNRW LNRINMLTAG YAERERIKQE QDYWSESDKE EADTPSTPKQ

701 DSPPPPYDTY PRPPSMSCAS PYVEAKHSRL SSTETSQSQS SHEEFRQEVT

751 GSSAVSPIRK TASQRRSWQD LIETPLTSSG LHYLQTLPLE DSVFSDSAAI

801 SPEHRRQSTL PTQKCHLQDH YGPYPLAESE RMQVLNGNGG KPRSFTLPRD

851 SGFNHCCLNA PVSACDPQDD VQPPEVEEEE EEEEEEGEAA GENIGEKSES

901 REEKLGDSLQ DLYRALEQAS LSPLGEHRIS TKMEYKLSFI KRCNDPVMNE

951 KLHRLRILKS TLKAREGEVA IIDKVLDNPD LTSKEFQQWK QMYLDLFLDI

1001 CQNTTSNDPL SISSEVDVIT SSLAHTHSYI ETHV*

# Fig. 8: Alignment of SEQ ID NO. 1, human MAGUIN-1, with rat MAGUIN-1

**Length: 1034 aa**

```
                       .         .         .         .         .
  1 MALIMEPVSKWSPSQVVDWMKGLDDCLQQYIKNFEREKISGDQLLRITHQ 50
    ||||||||||||||||||||||||||||||||||||||||||||||||||
  1 MALIMEPVSKWSPSQVVDWMKGLDDCLQQYIKNFEREKISGDQLLRITHQ 50
                       .         .         .         .         .
 51 ELEDLGVSRIGHQELILEAVDLLCALNYGLETENLKTLSHKLNASAKNLQ 100
    ||||||||||||||||||||||||||||||||||||||||||||||||||
 51 ELEDLGVSRIGHQELILEAVDLLCALNYGLETENLKTLSHKLNASAKNLQ 100
                       .         .         .         .         .
101 NFITGRRRSGHYDGRTSRKLPNDFLTSVVDLIGAAKSLLAWLDRSPFAAV 150
    ||||||||||||||||||||||||||||||||||||||||||||||||||
101 NFITGRRRSGHYDGRTSRKLPNDFLTSVVDLIGAAKSLLAWLDRSPFAAV 150
                       .         .         .         .         .
151 TDYSVTRNNVIQLCLELTTIVQQDCTVYETENKILHVCKTLSGVCDHIIS 200
    ||||||||||||||||||||||||||||||||||||||||||||||||||
151 TDYSVTRNNVIQLCLELTTIVQQDCTVYETENKILHVCKTLSGVCDHIIS 200
                       .         .         .         .         .
201 LSSDPLVSQSAHLEVIQLANIKPSEGLGMYIKSTYDGLHVITGTTENSPA 250
    ||||||||||||||||||||||||||||||||||||||||||||||||||
201 LSSDPLVSQSAHLEVIQLANIKPSEGLGMYIKSTYDGLHVITGTTENSPA 250
                       .         .         .         .         .
251 DRCKKIHAGDEVIQVNHQTVVGWQLKNLVNALREDPSGVILTLKKRPQSM 300
    ||||||||||||||||||||||||||||||||||||||||||||||||||
251 DRCKKIHAGDEVIQVNHQTVVGWQLKNLVNALREDPSGVILTLKKRPQSM 300
                       .         .         .         .         .
301 LTSAPALLKNMRWKPLALQPLIPRSPTSSVATPSSTISTPTKRDSSALQD 350
    ||||||||||||||||||||||||||||||||||||||||||||||||||
301 LTSAPALLKNMRWKPLALQPLIPRSPTSSVATPSSTISTPTKRDSSALQD 350
                       .         .         .         .         .
351 LYIPPPPAEPYIPRDEKGNLPCEDLRGHMVGKPVHKGSESPNSFLDQEYR 400
    ||||||||||||||||||||||||||||||||||||||||||||||||||
351 LYIPPPPAEPYIPRDEKGNLPCEDLRGHMVGKPVHKGSESPNSFLDQEYR 400
                       .         .         .         .         .
401 KRFNIVEEDTVLYCYEYEKGRSSSQGRRESTPTYGKLRPISMPVEYNWVG 450
    ||||||||||||||||||||||||||||||||||||||||||||||||||
401 KRFNIVEEDTVLYCYEYEKGRSSSQGRRESTPTYGKLRPISMPVEYNWVG 450
                       .         .         .         .         .
451 DYEDPNKMKRDSRRENSLLRYMSNEKIAQEEYMFQRNSKKDTGKKSKKKG 500
    ||||||||||||||||||||||||||||||||||||||||||||||||||
451 DYEDPNKMKRDSRRENSLLRYMSNEKIAQEEYMFQRNSKKDTGKKSKKKG 500
```

```
              .         .         .         .         .
 501 DKSNSPTHYSLLPSLQMDALRQDIMGTPVPETTLYHTFQQSSLQHKSKKK 550
     |||:||||||||||||||||||||||||||||||||||||||||||||||
 501 DKSTSPTHYSLLPSLQMDALRQDIMGTPVPETTLYHTFQQSSLQHKSKKK 550
              .         .         .         .         .
 551 NKGPIAGKSKRRISCKDLGRGDCEGWLWKKKDAKSYFSQKWKKYWFVLKD 600
     |||:||||||||||||||||||||||||||||||||||||||||||||||
 551 NKGAIAGKSKRRISCKDLGRGDCEGWLWKKKDAKSYFSQKWKKYWFVLKD 600
              .         .         .         .         .
 601 ASLYWYINEEDEKAEGFISLPEFKIDRASECRKKYAFKACHPKIKSFYFA 650
     ||||||||||||||||||||||||||||||||||||||||||||||||||
 601 ASLYWYINEEDEKAEGFISLPEFKIDRASECRKKYAFKACHPKIKSFYFA 650
              .         .         .         .         .
 651 AEHLDDMNRWLNRINMLTAGYAERERIKQEQDYWSESDKEEADTPSTPKQ 700
     ||||||||||||||||||||||||||||||||||||||||||||||||||
 651 AEHLDDMNRWLNRINMLTAGYAERERIKQEQDYWSESDKEEADTPSTPKQ 700
              .         .         .         .         .
 701 DSPPPPYDTYPRPPSMSCASPYVEAKHSRLSSTETSQSQSSHEEFRQEVT 750
     ||||||||||||||||||||||||||||||||||||||||||||||||||
 701 DSPPPPYDTYPRPPSMSCASPYVEAKHSRLSSTETSQSQSSHEEFRQEVT 750
              .         .         .         .         .
 751 GSSAVSPIRKTASQRRSWQDLIETPLTSSGLHYLQTLPLEDSVFSDSAAI 800
     ||||||||||||||||||||||||||||||||||||||||||||||||||
 751 GSSAVSPIRKTASQRRSWQDLIETPLTSSGLHYLQTLPLEDSVFSDSAAI 800
              .         .         .         .         .
 801 SPEHRRQSTLPTQKCHLQDHYGPYPLAESERMQVLNGNGGKPRSFTLPRD 850
     ||||||||||||||||||||||||||||||||||||||||||||||||||
 801 SPEHRRQSTLPTQKCHLQDHYGPYPLAESERMQVLNGNGGKPRSFTLPRD 850
              .         .         .         .         .
 851 SGFNHCCLNAPVSACDPQDDVQPPEVEEEEEEEEEEGEAAGENIGEKSES 900
     ||||||||||||||||||||:||||||||||||||  ||||||||||:|:
 851 SGFNHCCLNAPVSACDPQDDIQPPEVEEEEEEEEE..EAAGENIGEKNEN 898
              .         .         .         .         .
 901 REEKLGDSLQDLYRALEQASLSPLGEHRISTKMEYKLSFIKRCNDPVMNE 950
     |||||||||||||||||:||||||||||||||:|||||||||||||||||
 899 REEKLGDSLQDLYRALEEASLSPLGEHRISTKIEYKLSFIKRCNDPVMNE 948
              .         .         .         .         .
 951 KLHRLRILKSTLKAREGEVAIIDKVLDNPDLTSKEFQQWKQMYLDLFLDI 1000
     ||||||||||||||||||||||||||||||||||||||||||||||||||
 949 KLHRLRILKSTLKAREGEVAIIDKVLDNPDLTSKEFQQWKQMYLDLFLDI 998
              .         .         .
1001 CQNTTSNDPLSISSEVDVITSSLAHTHSYIETHV 1034
     |||||||||||||||||||||||:||||||||||
 999 CQNTTSNDPLSISSEVDVITSSLTHTHSYIETHV 1032
              .         .         .
```

# Fig. 9: SEQ ID NO. 2: amino acid sequence of human MAGUIN-2 protein

**Length: 898 aa**

1 MALIMEPVSK WSPSQVVDWM KGLDDCLQQY IKNFEREKIS GDQLLRITHQ

51 ELEDLGVSRI GHQELILEAV DLLCALNYGL ETENLKTLSH KLNASAKNLQ

101 NFITGRRRSG HYDGRTSRKL PNDFLTSVVD LIGAAKSLLA WLDRSPFAAV

151 TDYSVTRNNV IQLCLELTTI VQQDCTVYET ENKILHVCKT LSGVCDHIIS

201 LSSDPLVSQS AHLEVIQLAN IKPSEGLGMY IKSTYDGLHV ITGTTENSPA

251 DRCKKIHAGD EVIQVNHQTV VGWQLKNLVN ALREDPSGVI LTLKKRPQSM

301 LTSAPALLKN MRWKPLALQP LIPRSPTSSV ATPSSTISTP TKRDSSALQD

351 LYIPPPPAEP YIPRDEKGNL PCEDLRGHMV GKPVHKGSES PNSFLDQEYR

401 KRFNIVEEDT VLYCYEYEKG RSSSQGRRES TPTYGKLRPI SMPVEYNWVG

451 DYEDPNKMKR DSRRENSLLR YMSNEKIAQE EYMFQRNSKK DTGKKSKKKG

501 DKSNSPTHYS LLPSLQMDAL RQDIMGTPVP ETTLYHTFQQ SSLQHKSKKK

551 NKGPIAGKSK RRISCKDLGR GDCEGWLWKK KDAKSYFSQK WKKYWFVLKD

601 ASLYWYINEE DEKAEGFISL PEFKIDRASE CRKKYAFKAC HPKIKSFYFA

651 AEHLDDMNRW LNRINMLTAG YAERERIKQE QDYWSESDKE EADTPSTPKQ

701 DSPPPPYDTY PRPPSMSCAS PYVEAKHSRL SSTETSQSQS SHEEFRQEVT

751 GSSAVSPIRK TASQRRSWQD LIETPLTSSG LHYLQTLPLE DSVFSDSAAI

801 SPEHRRQSTL PTQKCHLQDH YGPYPLAESE RMQVLNGNGG KPRSFTLPRD

851 SGFNHCCLNA PVSACDPQDD VQPPEVEEEE EEEEEEGEAA GENIGEKS*

# Figure 10: Alignment of SEQ ID NO. 2, human MAGUIN-2, with rat MAGUIN-2

**Length: 898 aa**

```
                  .         .         .         .         .
  1 MALIMEPVSKWSPSQVVDWMKGLDDCLQQYIKNFEREKISGDQLLRITHQ  50
    ||||||||||||||||||||||||||||||||||||||||||||||||||
  1 MALIMEPVSKWSPSQVVDWMKGLDDCLQQYIKNFEREKISGDQLLRITHQ  50
                  .         .         .         .         .
 51 ELEDLGVSRIGHQELILEAVDLLCALNYGLETENLKTLSHKLNASAKNLQ 100
    ||||||||||||||||||||||||||||||||||||||||||||||||||
 51 ELEDLGVSRIGHQELILEAVDLLCALNYGLETENLKTLSHKLNASAKNLQ 100
                  .         .         .         .         .
101 NFITGRRRSGHYDGRTSRKLPNDFLTSVVDLIGAAKSLLAWLDRSPFAAV 150
    ||||||||||||||||||||||||||||||||||||||||||||||||||
101 NFITGRRRSGHYDGRTSRKLPNDFLTSVVDLIGAAKSLLAWLDRSPFAAV 150
                  .         .         .         .         .
151 TDYSVTRNNVIQLCLELTTIVQQDCTVYETENKILHVCKTLSGVCDHIIS 200
    ||||||||||||||||||||||||||||||||||||||||||||||||||
151 TDYSVTRNNVIQLCLELTTIVQQDCTVYETENKILHVCKTLSGVCDHIIS 200
                  .         .         .         .         .
201 LSSDPLVSQSAHLEVIQLANIKPSEGLGMYIKSTYDGLHVITGTTENSPA 250
    ||||||||||||||||||||||||||||||||||||||||||||||||||
201 LSSDPLVSQSAHLEVIQLANIKPSEGLGMYIKSTYDGLHVITGTTENSPA 250
                  .         .         .         .         .
251 DRCKKIHAGDEVIQVNHQTVVGWQLKNLVNALREDPSGVILTLKKRPQSM 300
    ||||||||||||||||||||||||||||||||||||||||||||||||||
251 DRCKKIHAGDEVIQVNHQTVVGWQLKNLVNALREDPSGVILTLKKRPQSM 300
                  .         .         .         .         .
301 LTSAPALLKNMRWKPLALQPLIPRSPTSSVATPSSTISTPTKRDSSALQD 350
    ||||||||||||||||||||||||||||||||||||||||||||||||||
301 LTSAPALLKNMRWKPLALQPLIPRSPTSSVATPSSTISTPTKRDSSALQD 350
                  .         .         .         .         .
351 LYIPPPPAEPYIPRDEKGNLPCEDLRGHMVGKPVHKGSESPNSFLDQEYR 400
    ||||||||||||||||||||||||||||||||||||||||||||||||||
351 LYIPPPPAEPYIPRDEKGNLPCEDLRGHMVGKPVHKGSESPNSFLDQEYR 400
                  .         .         .         .         .
401 KRFNIVEEDTVLYCYEYEKGRSSSQGRRESTPTYGKLRPISMPVEYNWVG 450
    ||||||||||||||||||||||||||||||||||||||||||||||||||
401 KRFNIVEEDTVLYCYEYEKGRSSSQGRRESTPTYGKLRPISMPVEYNWVG 450
                  .         .         .         .         .
451 DYEDPNKMKRDSRRENSLLRYMSNEKIAQEEYMFQRNSKKDTGKKSKKKG 500
    ||||||||||||||||||||||||||||||||||||||||||||||||||
451 DYEDPNKMKRDSRRENSLLRYMSNEKIAQEEYMFQRNSKKDTGKKSKKKG 500
```

```
         .         .         .         .
501 DKSNSPTHYSLLPSLQMDALRQDIMGTPVPETTLYHTFQQSSLQHKSKKK 550
    |||:||||||||||||||||||||||||||||||||||||||||||||||
501 DKSTSPTHYSLLPSLQMDALRQDIMGTPVPETTLYHTFQQSSLQHKSKKK 550
             .         .         .         .         .
551 NKGPIAGKSKRRISCKDLGRGDCEGWLWKKKDAKSYFSQKWKKYWFVLKD 600
    |||:||||||||||||||||||||||||||||||||||||||||||||||
551 NKGAIAGKSKRRISCKDLGRGDCEGWLWKKKDAKSYFSQKWKKYWFVLKD 600
             .         .         .         .         .
601 ASLYWYINEEDEKAEGFISLPEFKIDRASECRKKYAFKACHPKIKSFYFA 650
    ||||||||||||||||||||||||||||||||||||||||||||||||||
601 ASLYWYINEEDEKAEGFISLPEFKIDRASECRKKYAFKACHPKIKSFYFA 650
             .         .         .         .         .
651 AEHLDDMNRWLNRINMLTAGYAERERIKQEQDYWSESDKEEADTPSTPKQ 700
    ||||||||||||||||||||||||||||||||||||||||||||||||||
651 AEHLDDMNRWLNRINMLTAGYAERERIKQEQDYWSESDKEEADTPSTPKQ 700
             .         .         .         .         .
701 DSPPPPYDTYPRPPSMSCASPYVEAKHSRLSSTETSQSQSSHEEFRQEVT 750
    ||||||||||||||||||||||||||||||||||||||||||||||||||
701 DSPPPPYDTYPRPPSMSCASPYVEAKHSRLSSTETSQSQSSHEEFRQEVT 750
             .         .         .         .         .
751 GSSAVSPIRKTASQRRSWQDLIETPLTSSGLHYLQTLPLEDSVFSDSAAI 800
    ||||||||||||||||||||||||||||||||||||||||||||||||||
751 GSSAVSPIRKTASQRRSWQDLIETPLTSSGLHYLQTLPLEDSVFSDSAAI 800
             .         .         .         .         .
801 SPEHRRQSTLPTQKCHLQDHYGPYPLAESERMQVLNGNGGKPRSFTLPRD 850
    ||||||||||||||||||||||||||||||||||||||||||||||||||
801 SPEHRRQSTLPTQKCHLQDHYGPYPLAESERMQVLNGNGGKPRSFTLPRD 850
             .         .         .         .
851 SGFNHCCLNAPVSACDPQDDVQPPEVEEEEEEEEEEGEAAGENIGEKS  898
    ||||||||||||||||||||:||||||||||||||  |||||||||||
851 SGFNHCCLNAPVSACDPQDDIQPPEVEEEEEEEEE..EAAGENIGEKS  896
```

# Fig. 11: SEQ ID NO. 3: nucleotide sequence of human MAGUIN-1 coding sequence

**Length: 3105 bp**

```
   1 ATGGCTCTGA TAATGGAACC GGTGAGCAAA TGGTCTCCGA GTCAAGTAGT GGACTGGATG
  61 AAAGGTCTTG ATGACTGTTT GCAGCAGTAT ATTAAGAACT TTGAGAGGGA GAAGATCAGT
 121 GGGGACCAGC TGCTGCGCAT TACACATCAG GAGCTAGAAG ATCTGGGGGT CAGCCGCATT
 181 GGCCATCAGG AACTGATCTT GGAAGCAGTT GACCTTCTGT GTGCATTGAA TTATGGCTTG
 241 GAAACAGAAA ATCTAAAAAC CCTTTCTCAC AAGTTGAATG CATCTGCCAA AAATCTGCAG
 301 AATTTTATAA CAGGAAGGAG AAGGAGTGGC CATTATGATG GGAGGACCAG CCGAAAATTG
 361 CCAAACGACT TTCTGACCTC AGTTGTGGAT CTGATTGGAG CAGCCAAGAG TCTGCTTGCC
 421 TGGTTGGACA GGTCACCATT TGCTGCTGTG ACAGACTATT CAGTTACAAG AAATAATGTC
 481 ATACAACTCT GCCTGGAGTT AACAACAATT GTGCAACAGG ATTGTACTGT ATATGAAACA
 541 GAGAATAAAA TTCTTCACGT GTGTAAAACT CTTTCTGGAG TCTGTGACCA CATCATATCC
 601 CTGTCGTCAG ATCCTCTGGT TTCACAGTCT GCTCACCTGG AAGTGATTCA ACTGGCAAAC
 661 ATTAAACCAA GCGAAGGGCT GGGTATGTAT ATTAAATCTA CATATGATGG CCTCCATGTA
 721 ATTACTGGAA CCACAGAAAA TTCACCTGCA GATCGGTGCA AGAAAATCCA TGCTGGCGAT
 781 GAAGTGATTC AAGTTAATCA TCAGACTGTG GTGGGGTGGC AGTTGAAAAA TTTGGTGAAT
 841 GCACTACGAG AGGACCCGAG TGGTGTTATC TTAACTTTGA AAAAGCGACC TCAGAGCATG
 901 CTTACCTCAG CACCAGCTTT ACTGAAAAAT ATGAGATGGA AGCCCCTTGC TCTGCAGCCT
 961 CTTATACCTA GAAGTCCCAC AAGCAGCGTT GCCACGCCTT CCAGCACCAT CAGTACACCC
1021 ACCAAAAGAG ACAGTTCTGC CCTCCAGGAT CTCTACATTC CCCCTCCTCC TGCAGAACCA
1081 TATATTCCCA GGGATGAAAA AGGAAACCTT CCTTGTGAAG ACCTCAGAGG ACATATGGTG
1141 GGCAAGCCAG TGCATAAGGG ATCTGAATCA CCAAATTCAT TTCTGGATCA GGAATATCGA
1201 AAGAGATTTA ATATTGTCGA AGAAGATACT GTCTTATATT GCTATGAATA TGAAAAAGGA
1261 AGATCAAGTA GTCAAGGAAG ACGAGAAAGC ACCCCAACTT ATGGCAAGCT ACGACCTATA
1321 TCTATGCCAG TGGAATATAA TTGGGTGGGG GACTATGAAG ATCCAAATAA GATGAAGAGA
1381 GATAGTAGAA GAGAAAACTC TCTACTTCGG TATATGAGCA ATGAAAAGAT TGCTCAAGAA
1441 GAATACATGT TTCAGAGAAA CAGCAAAAAG GACACAGGGA AGAAGTCAAA AAAGAAGGGT
1501 GATAAGAGTA ATAGCCCAAC TCACTATTCA TTGCTACCTA GTTTACAAAT GGATGCACTG
1561 AGACAAGACA TCATGGGCAC TCCTGTGCCA GAGACCACAC TATACCATAC ATTTCAGCAG
1621 TCCTCACTGC AGCACAAATC AAAGAAGAAA AACAAAGGTC CTATAGCAGG CAAGAGCAAA
1681 AGACGAATTT CTTGCAAAGA TCTTGGCCGT GGTGACTGTG AGGGCTGGCT TTGGAAAAAG
1741 AAAGATGCGA AGAGTTACTT TTCACAGAAA TGGAAAAAAT ATTGGTTTGT CCTAAAGGAT
1801 GCATCCCTTT ATTGGTATAT TAATGAGGAG GATGAAAAAG CAGAAGGATT CATTAGCCTG
1861 CCTGAATTTA AAATTGATAG AGCCAGTGAA TGCCGCAAAA AATATGCATT CAAAGCCTGT
1921 CATCCTAAAA TCAAAAGCTT TTATTTTGCT GCTGAACATC TTGATGATAT GAACAGGTGG
1981 CTTAACAGAA TTAATATGCT GACTGCAGGA TATGCAGAAA GAGAGAGGAT TAAGCAGGAA
2041 CAAGATTACT GGAGTGAGAG TGACAAGGAA GAAGCAGATA CTCCATCAAC ACCAAAACAA
2101 GATAGCCCTC CACCCCCATA TGATACATAC CCACGACCTC CCTCGATGAG TTGCGCCAGT
2161 CCTTATGTGG AAGCAAAACA TAGCCGACTT TCCTCCACGG AGACTTCTCA GTCTCAGTCT
2221 TCTCATGAGG AGTTTCGCCA GGAAGTAACT GGGAGCAGTG CAGTGTCTCC CATTCGCAAG
2281 ACAGCCAGTC AGCGCCGCTC CTGGCAGGAT TTAATTGAGA CGCCACTGAC AAGTTCAGGC
2341 TTACACTATC TTCAGACTCT GCCCCTGGAG GATTCTGTCT TCTCTGACTC CGCGGCCATC
2401 TCCCCAGAGC ACAGGCGGCA GTCTACCCTG CCAACTCAGA AATGCCACCT GCAGGATCAC
2461 TATGGGCCAT ACCCCTTAGC TGAGAGTGAG AGGATGCAAG TGCTAAATGG AAATGGGGGC
2521 AAGCCTCGAA GTTTTACTCT GCCTCGAGAT AGCGGGTTCA ACCATTGCTG TCTGAATGCT
2581 CCAGTTAGTG CCTGTGACCC ACAGGATGAC GTGCAACCCC CAGAGGTGGA GGAAGAGGAG
2641 GAGGAGGAGG AGGAGGAAGG GGAGGCAGCA GGGGAAAACA TAGGAGAAAA AAGTGAAAGC
2701 AGAGAAGAAA AGTTAGGAGA CTCATTGCAA GATTTATACA GGGCACTGGA GCAGGCCAGT
2761 CTGTCACCAC TAGGAGAACA TCGTATTTCA ACCAAGATGG AATACAAGCT ATCATTTATA
2821 AAAAGATGTA ATGATCCTGT AATGAATGAA AAACTACACC GGCTGAGAAT TCTCAAAAGC
2881 ACTTTAAAGG CCAGAGAAGG GGAAGTAGCC ATTATCGATA AAGTCCTAGA CAATCCAGAC
2941 TTGACATCTA AAGAATTCCA ACAATGGAAG CAGATGTACC TCGACCTTTT CTTGGATATC
3001 TGTCAAAATA CCACCTCAAA TGACCCACTG AGTATTTCTT CTGAAGTAGA TGTAATCACT
3061 TCCTCTCTAG CACACACTCA TTCATACATT GAAACGCATG TCTAA
```

# Fig. 12: SEQ ID NO. 4: nucleotide sequence of human MAGUIN-2 coding sequence

**Length: 2697 bp**

```
   1 ATGGCTCTGA TAATGGAACC GGTGAGCAAA TGGTCTCCGA GTCAAGTAGT
  51 GGACTGGATG AAAGGTCTTG ATGACTGTTT GCAGCAGTAT ATTAAGAACT
 101 TTGAGAGGGA GAAGATCAGT GGGGACCAGC TGCTGCGCAT TACACATCAG
 151 GAGCTAGAAG ATCTGGGGGT CAGCCGCATT GGCCATCAGG AACTGATCTT
 201 GGAAGCAGTT GACCTTCTGT GTGCATTGAA TTATGGCTTG GAAACAGAAA
 251 ATCTAAAAAC CCTTTCTCAC AAGTTGAATG CATCTGCCAA AAATCTGCAG
 301 AATTTTATAA CAGGAAGGAG AAGGAGTGGC CATTATGATG GGAGGACCAG
 351 CCGAAAATTG CCAAACGACT TTCTGACCTC AGTTGTGGAT CTGATTGGAG
 401 CAGCCAAGAG TCTGCTTGCC TGGTTGGACA GGTCACCATT TGCTGCTGTG
 451 ACAGACTATT CAGTTACAAG AAATAATGTC ATACAACTCT GCCTGGAGTT
 501 AACAACAATT GTGCAACAGG ATTGTACTGT ATATGAAACA GAGAATAAAA
 551 TTCTTCACGT GTGTAAAACT CTTTCTGGAG TCTGTGACCA CATCATATCC
 601 CTGTCGTCAG ATCCTCTGGT TTCACAGTCT GCTCACCTGG AAGTGATTCA
 651 GCTGGCAAAC ATTAAACCAA GCGAAGGGCT GGGTATGTAT ATTAAATCTA
 701 CATATGATGG CCTCCATGTA ATTACTGGAA CCACAGAAAA TTCACCTGCA
 751 GATCGGTGCA AGAAAATCCA TGCTGGCGAT GAAGTGATTC AAGTTAATCA
 801 TCAGACTGTG GTGGGGTGGC AGTTGAAAAA TTTGGTGAAT GCACTACGAG
 851 AGGACCCGAG TGGTGTTATC TTAACTTTGA AAAAGCGACC TCAGAGCATG
 901 CTTACCTCAG CACCAGCTTT ACTGAAAAAT ATGAGATGGA AGCCCCTTGC
 951 TCTGCAGCCT CTTATACCTA GAAGTCCCAC AAGCAGCGTT GCCACGCCTT
1001 CCAGCACCAT CAGTACACCC ACCAAAAGAG ACAGTTCTGC CCTCCAGGAT
1051 CTCTACATTC CCCCTCCTCC TGCAGAACCA TATATTCCCA GGGATGAAAA
1101 AGGAAACCTT CCTTGTGAAG ACCTCAGAGG ACATATGGTG GGCAAGCCAG
1151 TGCATAAGGG ATCTGAATCA CCAAATTCAT TTCTGGATCA GGAATATCGA
1201 AAGAGATTTA ATATTGTCGA AGAAGATACT GTCTTATATT GCTATGAATA
1251 TGAAAAAGGA AGATCAAGTA GTCAAGGAAG ACGAGAAAGC ACCCCAACTT
1301 ATGGCAAGCT ACGACCTATA TCTATGCCAG TGGAATATAA TTGGGTGGGG
1351 GACTATGAAG ATCCAAATAA GATGAAGAGA GATAGTAGAA GAGAAAACTC
1401 TCTACTTCGG TATATGAGCA ATGAAAAGAT TGCTCAAGAA GAATACATGT
1451 TTCAGAGAAA CAGCAAAAAG GACACAGGGA AGAAGTCAAA AAAGAAGGGT
1501 GATAAGAGTA ATAGCCCAAC TCACTATTCA TTGCTACCTA GTTTACAAAT
1551 GGATGCACTG AGACAAGACA TCATGGGCAC TCCTGTGCCA GAGACCACAC
1601 TATACCATAC ATTTCAGCAG TCCTCACTGC AGCACAAATC AAAGAAGAAA
1651 AACAAAGGTC CTATAGCAGG CAAGAGCAAA AGACGAATTT CTTGCAAAGA
1701 TCTTGGCCGT GGTGACTGTG AGGGCTGGCT TTGGAAAAAG AAAGATGCGA
1751 AGAGTTACTT TTCACAGAAA TGGAAAAAAT ATTGGTTTGT CCTAAAGGAT
1801 GCATCCCTTT ATTGGTATAT TAATGAGGAG GATGAAAAAG CAGAAGGATT
1851 CATTAGCCTG CCTGAATTTA AAATTGATAG AGCCAGTGAA TGCCGCAAAA
1901 AATATGCATT CAAAGCCTGT CATCCTAAAA TCAAAAGCTT TTATTTTGCT
1951 GCTGAACATC TTGATGATAT GAACAGGTGG CTTAACAGAA TTAATATGCT
2001 GACTGCAGGA TATGCAGAAA GAGAGAGGAT TAAGCAGGAA CAAGATTACT
2051 GGAGTGAGAG TGACAAGGAA GAAGCAGATA CTCCATCAAC ACCAAAACAA
2101 GATAGCCCTC CACCCCCATA TGATACATAC CCACGACCTC CCTCGATGAG
2151 TTGCGCCAGT CCTTATGTGG AAGCAAAACA TAGCCGACTT TCCTCCACGG
2201 AGACTTCTCA GTCTCAGTCT TCTCATGAGG AGTTTCGCCA GGAAGTAACT
2251 GGGAGCAGTG CAGTGTCTCC CATTCGCAAG ACAGCCAGTC AGCGCCGCTC
2301 CTGGCAGGAT TTAATTGAGA CGCCACTGAC AAGTTCAGGC TTACACTATC
2351 TTCAGACTCT GCCCCTGGAG GATTCTGTCT TCTCTGACTC CGCGGCCATC
2401 TCCCCAGAGC ACAGGCGGCA GTCTACCCTG CCAACTCAGA AATGCCACCT
2451 GCAGGATCAC TATGGGCCAT ACCCCTTAGC TGAGAGTGAG AGGATGCAAG
2501 TGCTAAATGG AAATGGGGGC AAGCCTCGAA GTTTTACTCT GCCTCGAGAT
2551 AGCGGGTTCA ACCATTGCTG TCTGAaTGCT CCAGTTAGTG CCTGTGACCC
2601 ACAGGATGAC GTGCAACCCC CAGAGGTGGA GGAAGAGGAG GAGGAGGAGG
2651 AGGAGGAAGG GGAGGCAGCA GGGGAAAACA TAGGAGAAAA AAGCTAA
```

# Fig. 13: SEQ ID NO. 5: nucleotide sequence of human MAGUIN-1 cDNA

**Length: 5749 bp**

```
   1 CGGGCAGCTA GTCGTGCTCG GGGCTTCACT CCCGCGCGTG AGGCGAGCGG GCAAGTTGGC
  61 TGAGGGCGTG CGGCAGAGGC TGCTTCCCTC GGCGACGCGA CCCCTCAGCA ACTCAAGCTA
 121 TGAACTGAAG CTCCCTAGGG ACGGAGACCG GAGCGGAGCG GCGGAGGCAG CAGCAGCAGC
 181 AGCAGCAGCA GCAGCAGCAG CAGCCGCCGC CGCCGCCGCC TTAGCGGGAA CTGAGCAGAC
 241 CCGGCGCGGA GCCACGACTC CTGCACGTTT ACCTCCCTGT CGCCGTTCCT GCCGGCGGTT
 301 GGCTAAAAGA CGTTACAGCC GCGAGACCCG ACACACAAAA GCCGCTTTCT CCGCGCCGCC
 361 CGCCCAGGGA GGCTGCGGCC AGCAAGGGAC CCCACCTGAG AGCAGCTCGG GCTGCTGAGT
 421 TCGTTTTGTG TCTGAGCTCT GCGCTCTGCA CGGAACCGAC CCCGTACCCA TGGCTCTGAT
 481 AATGGAACCG GTGAGCAAAT GGTCTCCGAG TCAAGTAGTG GACTGGATGA AAGGTCTTGA
 541 TGACTGTTTG CAGCAGTATA TTAAGAACTT TGAGAGGGAG AAGATCAGTG GGGACCAGCT
 601 GCTGCGCATT ACACATCAGG AGCTAGAAGA TCTGGGGGTC AGCCGCATTG GCCATCAGGA
 661 ACTGATCTTG GAAGCAGTTG ACCTTCTGTG TGCATTGAAT TATGGCTTGG AAACAGAAAA
 721 TCTAAAAACC CTTTCTCACA AGTTGAATGC ATCTGCCAAA AATCTGCAGA ATTTTATAAC
 781 AGGAAGGAGA AGGAGTGGCC ATTATGATGG GAGGACCAGC CGAAAATTGC CAAACGACTT
 841 TCTGACCTCA GTTGTGGATC TGATTGGAGC AGCCAAGAGT CTGCTTGCCT GGTTGGACAG
 901 GTCACCATTT GCTGCTGTGA CAGACTATTC AGTTACAAGA AATAATGTCA TACAACTCTG
 961 CCTGGAGTTA ACAACAATTG TGCAACAGGA TTGTACTGTA TATGAAACAG AGAATAAAAT
1021 TCTTCACGTG TGTAAAACTC TTTCTGGAGT CTGTGACCAC ATCATATCCC TGTCGTCAGA
1081 TCCTCTGGTT TCACAGTCTG CTCACCTGGA AGTGATTCAA CTGGCAAACA TTAAACCAAG
1141 CGAAGGGCTG GGTATGTATA TTAAATCTAC ATATGATGGC CTCCATGTAA TTACTGGAAC
1201 CACAGAAAAT TCACCTGCAG ATCGGTGCAA GAAAATCCAT GCTGGCGATG AAGTGATTCA
1261 AGTTAATCAT CAGACTGTGG TGGGGTGGCA GTTGAAAAAT TTGGTGAATG CACTACGAGA
1321 GGACCCGAGT GGTGTTATCT TAACTTTGAA AAAGCGACCT CAGAGCATGC TTACCTCAGC
1381 ACCAGCTTTA CTGAAAAATA TGAGATGGAA GCCCCTTGCT CTGCAGCCTC TTATACCTAG
1441 AAGTCCCACA AGCAGCGTTG CCACGCCTTC CAGCACCATC AGTACACCCA CCAAAAGAGA
1501 CAGTTCTGCC CTCCAGGATC TCTACATTCC CCCTCCTCCT GCAGAACCAT ATATTCCCAG
1561 GGATGAAAAA GGAAACCTTC CTTGTGAAGA CCTCAGAGGA CATATGGTGG GCAAGCCAGT
1621 GCATAAGGGA TCTGAATCAC CAAATTCATT TCTGGATCAG GAATATCGAA AGAGATTTAA
1681 TATTGTCGAA GAAGATACTG TCTTATATTG CTATGAATAT GAAAAAGGAA GATCAAGTAG
1741 TCAAGGAAGA CGAGAAAGCA CCCCAACTTA TGGCAAGCTA CGACCTATAT CTATGCCAGT
1801 GGAATATAAT TGGGTGGGGG ACTATGAAGA TCCAAATAAG ATGAAGAGAG ATAGTAGAAG
1861 AGAAAACTCT CTACTTCGGT ATATGAGCAA TGAAAAGATT GCTCAAGAAG AATACATGTT
1921 TCAGAGAAAC AGCAAAAAGG ACACAGGGAA GAAGTCAAAA AAGAAGGGTG ATAAGAGTAA
1981 TAGCCCAACT CACTATTCAT TGCTACCTAG TTTACAAATG GATGCACTGA GACAAGACAT
2041 CATGGGCACT CCTGTGCCAG AGACCACACT ATACCATACA TTTCAGCAGT CCTCACTGCA
2101 GCACAAATCA AAGAAGAAAA ACAAAGGTCC TATAGCAGGC AAGAGCAAAA GACGAATTTC
2161 TTGCAAAGAT CTTGGCCGTG GTGACTGTGA GGGCTGGCTT TGGAAAAAGA AAGATGCGAA
2221 GAGTTACTTT TCACAGAAAT GGAAAAAATA TTGGTTTGTC CTAAAGGATG CATCCCTTTA
2281 TTGGTATATT AATGAGGAGG ATGAAAAAGC AGAAGGATTC ATTAGCCTGC CTGAATTTAA
2341 AATTGATAGA GCCAGTGAAT GCCGCAAAAA ATATGCATTC AAAGCCTGTC ATCCTAAAAT
2401 CAAAAGCTTT TATTTTGCTG CTGAACATCT TGATGATATG AACAGGTGGC TTAACAGAAT
2461 TAATATGCTG ACTGCAGGAT ATGCAGAAAG AGAGAGGATT AAGCAGGAAC AAGATTACTG
2521 GAGTGAGAGT GACAAGGAAG AAGCAGATAC TCCATCAACA CCAAAACAAG ATAGCCCTCC
2581 ACCCCCATAT GATACATACC CACGACCTCC CTCGATGAGT TGCGCCAGTC CTTATGTGGA
2641 AGCAAAACAT AGCCGACTTT CCTCCACGGA GACTTCTCAG TCTCAGTCTT CTCATGAGGA
2701 GTTTCGCCAG GAAGTAACTG GGAGCAGTGC AGTGTCTCCC ATTCGCAAGA CAGCCAGTCA
2761 GCGCCGCTCC TGGCAGGATT TAATTGAGAC GCCACTGACA AGTTCAGGCT TACACTATCT
2821 TCAGACTCTG CCCCTGGAGG ATTCTGTCTT CTCTGACTCC GCGGCCATCT CCCCAGAGCA
```

2881 CAGGCGGCAG TCTACCCTGC CAACTCAGAA ATGCCACCTG CAGGATCACT ATGGGCCATA
2941 CCCCTTAGCT GAGAGTGAGA GGATGCAAGT GCTAAATGGA AATGGGGGCA AGCCTCGAAG

3001 TTTTACTCTG CCTCGAGATA GCGGGTTCAA CCATTGCTGT CTGAATGCTC CAGTTAGTGC
3061 CTGTGACCCA CAGGATGACG TGCAACCCCC AGAGGTGGAG GAAGAGGAGG AGGAGGAGGA
3121 GGAGGAAGGG GAGGCAGCAG GGGAAAACAT AGGAGAAAAA AGTGAAAGCA GAGAAGAAAA
3181 GTTAGGAGAC TCATTGCAAG ATTTATACAG GGCACTGGAG CAGGCCAGTC TGTCACCACT
3241 AGGAGAACAT CGTATTTCAA CCAAGATGGA ATACAAGCTA TCATTTATAA AAAGATGTAA
3301 TGATCCTGTA ATGAATGAAA AACTACACCG GCTGAGAATT CTCAAAAGCA CTTTAAAGGC
3361 CAGAGAAGGG GAAGTAGCCA TTATCGATAA AGTCCTAGAC AATCCAGACT TGACATCTAA
3421 AGAATTCCAA CAATGGAAGC AGATGTACCT CGACCTTTTC TTGGATATCT GTCAAAATAC
3481 CACCTCAAAT GACCCACTGA GTATTTCTTC TGAAGTAGAT GTAATCACTT CCTCTCTAGC
3541 ACACACTCAT TCATACATTG AAACGCATGT CTAAATGTAT TCTGCCTTCA GACCATCTAG
3601 TACCTGCTGG TACTCTGAAC AAGTATATAA GGTAGTTTTT ATATCAATGT GTGGAACACT
3661 TGACAAGCTA TACTTTAATG TTACCAAACT ATATGAAACA AACCATATAT GGTCACAATA
3721 CCACTATCTT TAATGAGCAT TTGTATATTT TATATGCAAC AGTGCTCAGC TTATGTTTAC
3781 CATGTGCAAA ATCAACTGTC TTTAATGACT TAAAATTAAC TTTTGCAAAC AATTCTAAAT
3841 ACAGGTGGTC TTCAAGTAGT AAAACCACAA AAGGCAGTTT TCTATCTATG GTCATCTTTT
3901 CTCCCTTTAA GTTAATTTTA TATAAACAAG ACTTCAAAAG TAAATCACAT TTTTTCAGGT
3961 GCAGACATCC TTGTGGGTGG GAAAGAATTT AAACCTTTTT TATATTTATT AAAATGTTCT
4021 AAGAATTTTC TTAAACATTG CACAAAGTTT AATGCTGTAG TTTTATTTTT GTGAAATGTA
4081 GATGCGCATA CAAGAGCTAA GCAAAATAGA AGAGCATCGA CATAAGAAAA GTTCAGGTAT
4141 CTAATATTCG TCTTAATAGT CTATTAACTT GTGAAAGCTA AGTTAATGGA AATATTATTC
4201 CAAATCTATG AGAACACTTG GTGTATCAGG GCAAAGCTTT GTAAGATGTT TTTGTAACTA
4261 AGACCAAAAT TGAAGATAGA GCTGCTTTAT TTTCTTGGTT TAAATCTTCC TTTATTTTTG
4321 TAGTGATGAG ATGCTGATTG TGTACAGAAG AATTTGAGAG GGGATTTTTA AAAACTGACT
4381 TAACACACCC AGAAAGGCAG CTAACAGCTA TATATATATA TAAATTTCAG CCCAAACTCA
4441 TGTTTTTAAA CTCCAACTCT TAAAAGACAA CAAGGTATAA ACTGAAATGA ATCAACTTTC
4501 CACTTAGTTT CCAATTTTCC CCTAGTCCAC TAATTAAACT TAGGTAATTA TACTTCAGGT
4561 AGGGAAGTAC AATATGTTTA GTTTCAGGCT GATGTGTGTT ATAAAAAACA ACACTGAAAA
4621 ATAAAAATGT ACTTCCCTTC TAAGGAGCAA GCAGGTGATG GTCATTCAAA GAGATGTCAC
4681 ATTGAATTAT GAGAGAAACA ATTTAGAGGT TTTTTTCCTG GCTTCATGAA TTGTTCTATA
4741 GAGTGGATGA AGTCTAAGGA AAAGTCCTCT TCATATATTT CCATTTATAA GCGTCTTGTT
4801 TTTGAAAGTG ATCACAGCAT GAAAATGACT GTGCTGCTTT TTAGTGTCTG GCTGCATAAT
4861 GTACAAGTCA CAATTTGCTG TTTTTTTCAG GAGGAGAAAG GGAACCTCCT TTACTATTCT
4921 ATATCCTAAA ATCTACTTCT AATCAGCTTT ATACTGTTGC CTGTACAGCT CAGTGAATGT
4981 ACTTTCATCT TTAAGAGTTC AGATATATGC CAGTGAATAT TTTTGCTGTA GAGGAGAAAG
5041 TAAAAACTCC ACAGCGGGGA TCTTTTTCTT TGCTTTTGAA ACCACCATTG AATCACTATC
5101 GTTTTGCAGA CTTTGCACAA CTGTACAGGA GAGTGGCCTT TCTACAGCAC ATTTTCAGTA
5161 ATCCTATATT TAGTCAAAAT GGATGAGAAA TCATGTATTA ATGTTTGTAT GGAATTTTGG
5221 GTCCAGTGTA ATATTTTTAT CATTTAAAAA GAACTCTATT TGTAAAAACA TTTATTTACT
5281 GCATGGATAT TGACGCACAT TAAATTTGTG GGATTTTGTA TATGTAAAAA AAAAAAAAAA
5341 AAAAAAAAAC AAAAAACCTC TTGTCCTAAA ATGAAGTGTG CTTGTTAACA GGTGTTTAGA
5401 CTTATTGATG TTTACTAGAC CAAATGTGTA TGTTCACTTA AAAATATATG TACCTGATGG
5461 ATGTGTCATG TTTACAGTGG CCAGGTTGTG GCCTGTAAAC AGCAAGCAGT TGACGGGAAG
5521 ACTAGCTCTG TTGCTACTAA GCAGCTTTTA CTTTTGTAAA GTCAGCTCTG TTGTTTTAAA
5581 TGGTAAAAAT TAAACTAATG AATTTGACAA GACTCGTGGC TAGCCTAGCA TGAAAGAGAC
5641 CTTTTAACAC TATATAATAT CTGTACATTT TATTGCATTC GTTTCAAATC TAGGAGAGAG
5701 GCAGCACTGT AAACTGAAGT CAAATAAATT CAGCTCTTAA TGAATCCTT

# Fig. 14: SEQ ID NO. 6: nucleotide sequence of human MAGUIN-2 cDNA

**Length: 4350 bp**

```
   1 GTGCTCGGGG CTTCACTCCC GCGCGTGAGG CGAGCGGGCA AGTTGGCTGA
  51 GGGCGTGCGG CAGAGGCTGC TTCCCTCGGC GACGCGACCC CTCAGCAACT
 101 CAAGCTATGA ACTGAAGCTC CCTAGGGACG GAGACCGGAG CGGAGCGGCG
 151 GAGGCAGCAG CAGCAGCAGC AGCAGCAGCA GCAGCAGCAG CCGCCGCCGC
 201 CGCCGCCTTA GCGGGAACTG AGCAGACCCG GCGCGGAGCC ACGACTCCTG
 251 CACGTTTACC TCCCTGTCGC CGTTCCTGCC GGCGGTTGGC TAAAAGACGT
 301 TACAGCCGCG AGACCCGACA CACAAAAGCC GCTTTCTCCG CGCCGCCCGC
 351 CCAGGGAGGC TGCGGCCAGC AAGGGACCCC ACCTGAGAGC AGCTCGGGCT
 401 GCTGAGTTCG TTTTGTGTCT GAGCTCTGCG CTCTGCACGG AACCGACCCC
 451 GTACCCATGG CTCTGATAAT GGAACCGGTG AGCAAATGGT CTCCGAGTCA
 501 AGTAGTGGAC TGGATGAAAG GTCTTGATGA CTGTTTGCAG CAGTATATTA
 551 AGAACTTTGA GAGGGAGAAG ATCAGTGGGG ACCAGCTGCT GCGCATTACA
 601 CATCAGGAGC TAGAAGATCT GGGGGTCAGC CGCATTGGCC ATCAGGAACT
 651 GATCTTGGAA GCAGTTGACC TTCTGTGTGC ATTGAATTAT GGCTTGGAAA
 701 CAGAAAATCT AAAAACCCTT TCTCACAAGT TGAATGCATC TGCCAAAAAT
 751 CTGCAGAATT TTATAACAGG AAGGAGAAGG AGTGGCCATT ATGATGGGAG
 801 GACCAGCCGA AAATTGCCAA ACGACTTTCT GACCTCAGTT GTGGATCTGA
 851 TTGGAGCAGC CAAGAGTCTG CTTGCCTGGT TGGACAGGTC ACCATTTGCT
 901 GCTGTGACAG ACTATTCAGT TACAAGAAAT AATGTCATAC AACTCTGCCT
 951 GGAGTTAACA ACAATTGTGC AACAGGATTG TACTGTATAT GAAACAGAGA
1001 ATAAAATTCT TCACGTGTGT AAAACTCTTT CTGGAGTCTG TGACCACATC
1051 ATATCCCTGT CGTCAGATCC TCTGGTTTCA CAGTCTGCTC ACCTGGAAGT
1101 GATTCAGCTG GCAAACATTA AACCAAGCGA AGGGCTGGGT ATGTATATTA
1151 AATCTACATA TGATGGCCTC CATGTAATTA CTGGAACCAC AGAAAATTCA
1201 CCTGCAGATC GGTGCAAGAA AATCCATGCT GGCGATGAAG TGATTCAAGT
1251 TAATCATCAG ACTGTGGTGG GGTGGCAGTT GAAAAATTTG GTGAATGCAC
1301 TACGAGAGGA CCCGAGTGGT GTTATCTTAA CTTTGAAAAA GCGACCTCAG
1351 AGCATGCTTA CCTCAGCACC AGCTTTACTG AAAAATATGA GATGGAAGCC
1401 CCTTGCTCTG CAGCCTCTTA TACCTAGAAG TCCCACAAGC AGCGTTGCCA
1451 CGCCTTCCAG CACCATCAGT ACACCCACCA AAAGAGACAG TTCTGCCCTC
1501 CAGGATCTCT ACATTCCCCC TCCTCCTGCA GAACCATATA TTCCCAGGGA
1551 TGAAAAAGGA AACCTTCCTT GTGAAGACCT CAGAGGACAT ATGGTGGGCA
1601 AGCCAGTGCA TAAGGGATCT GAATCACCAA ATTCATTTCT GGATCAGGAA
1651 TATCGAAAGA GATTTAATAT TGTCGAAGAA GATACTGTCT TATATTGCTA
1701 TGAATATGAA AAAGGAAGAT CAAGTAGTCA AGGAAGACGA GAAAGCACCC
1751 CAACTTATGG CAAGCTACGA CCTATATCTA TGCCAGTGGA ATATAATTGG
1801 GTGGGGGACT ATGAAGATCC AAATAAGATG AAGAGAGATA GTAGAAGAGA
1851 AAACTCTCTA CTTCGGTATA TGAGCAATGA AAAGATTGCT CAAGAAGAAT
1901 ACATGTTTCA GAGAAACAGC AAAAAGGACA CAGGGAAGAA GTCAAAAAAG
1951 AAGGGTGATA AGAGTAATAG CCCAACTCAC TATTCATTGC TACCTAGTTT
2001 ACAAATGGAT GCACTGAGAC AAGACATCAT GGGCACTCCT GTGCCAGAGA
2051 CCACACTATA CCATACATTT CAGCAGTCCT CACTGCAGCA CAAATCAAAG
2101 AAGAAAAACA AAGGTCCTAT AGCAGGCAAG AGCAAAAGAC GAATTTCTTG
2151 CAAAGATCTT GGCCGTGGTG ACTGTGAGGG CTGGCTTTGG AAAAAGAAAG
2201 ATGCGAAGAG TTACTTTTCA CAGAAATGGA AAAAATATTG GTTTGTCCTA
2251 AAGGATGCAT CCCTTTATTG GTATATTAAT GAGGAGGATG AAAAAGCAGA
```

```
2301  AGGATTCATT AGCCTGCCTG AATTTAAAAT TGATAGAGCC AGTGAATGCC
2351  GCAAAAAATA TGCATTCAAA GCCTGTCATC CTAAAATCAA AAGCTTTTAT

2401  TTTGCTGCTG AACATCTTGA TGATATGAAC AGGTGGCTTA ACAGAATTAA
2451  TATGCTGACT GCAGGATATG CAGAAAGAGA GAGGATTAAG CAGGAACAAG
2501  ATTACTGGAG TGAGAGTGAC AAGGAAGAAG CAGATACTCC ATCAACACCA
2551  AAACAAGATA GCCCTCCACC CCCATATGAT ACATACCCAC GACCTCCCTC
2601  GATGAGTTGC GCCAGTCCTT ATGTGGAAGC AAAACATAGC CGACTTTCCT
2651  CCACGGAGAC TTCTCAGTCT CAGTCTTCTC ATGAGGAGTT TCGCCAGGAA
2701  GTAACTGGGA GCAGTGCAGT GTCTCCCATT CGCAAGACAG CCAGTCAGCG
2751  CCGCTCCTGG CAGGATTTAA TTGAGACGCC ACTGACAAGT TCAGGCTTAC
2801  ACTATCTTCA GACTCTGCCC CTGGAGGATT CTGTCTTCTC TGACTCCGCG
2851  GCCATCTCCC CAGAGCACAG GCGGCAGTCT ACCCTGCCAA CTCAGAAATG
2901  CCACCTGCAG GATCACTATG GGCCATACCC CTTAGCTGAG AGTGAGAGGA
2951  TGCAAGTGCT AAATGGAAAT GGGGGCAAGC CTCGAAGTTT TACTCTGCCT
3001  CGAGATAGCG GGTTCAACCA TTGCTGTCTG AaTGCTCCAG TTAGTGCCTG
3051  TGACCCACAG GATGACGTGC AACCCCCAGA GGTGGAGGAA GAGGAGGAGG
3101  AGGAGGAGGA GGAAGGGGAG GCAGCAGGGG AAAACATAGG AGAAAAAAGC
3151  TAATACACTG CGAGAGTTGG TAGAACCTCT CCATGCCAAA TCGGATCCAC
3201  TTCTGTTGGC ACTCAACCCA TTGGACTCAC AGATTGATAA GCTAATGTTT
3251  AGAGAATTTA GATCGGAGAG AGTCGGTACG GCGCAGACTC AACATCAACC
3301  TCTTGCAAGC AACTAAAATG GCCTCGTCCT TGCTGTTTAT AACAGAAAAC
3351  AGACTTGTAA AAAGCTTAGA TCATCAAGTG TTTTGGATTG GGGGCCTCCC
3401  AAAGGGATAT AAGAGGGGCA GGCCACTCTT AAGAAGAATG CGAGCTTTCT
3451  ACATTGGGAC TAGCATAAGA TCAAAGCCAA TCAAGATGGA GCACAGTAAC
3501  AGAAAACTGC GGTTTCTGTG GGAGAACAGA AGGGGAAAGG GTCTTAACTG
3551  GGAAAGGGCT CTGTGTGGTA ACACCTCAGT TGTGTTCTCC TGACACCAGG
3601  AAAAGAGAGG GATCAGCTTC AATAACTAGA AAATTCTGGC TGTTTAATGG
3651  ACTCTTTGGT GGCCTCTTTA AGGCAAAGCA GAGAAAGCAA ATTATGTATT
3701  AAGTGTATTT TGCATTTTTA AAACTTGACG TGCTGTATTG TACTAAATTA
3751  AGTGTAATCT ATTAAGGCAA GGTATACACA ATTTGCTTTG AAACTTACTA
3801  TGTTTATTCT ATTATAAAGT GTATTCAGGT GCAACACAGA GACTGCTTTC
3851  GGTGACATTA ATGAAGAAAA TTTCTCATGC CAGGCTTTAT TATAGAATCT
3901  TCAGCTAAAA TCCTAACTTT CTCCTTATTT CTTGGCACTT GTATACAAGT
3951  GGTGTTGCCT CTTAGGGCAG GCATGAGCTA TTCTTTTCTG TAAAATATTT
4001  TGAATCTATA GGCTGTGGGT TTCATTTTTG AAAAGTATTT TGTCTGGATG
4051  TCTTTCAAAC TAGCTTCAGA TATTATTTAA TACTATGTAA CTGGGTCCCC
4101  TATGGCTCAA TCAATATTGC TTATTTTTCT TCTGTAGTGG ATGTGAAATT
4151  TCCTTTAGTT GGATAAGATA CACTGTAATA ATTTTAATGC TAATTAATGA
4201  TATTTCATAC TGTGCAATGA ACAGATAATT TAACACTGTA TTTTGAAATG
4251  TTTTTTTCTT CCTGTCACCG CAGTGTGTGG TATTGCATAA TGTGAATACC
4301  TGTAAAAATA TAAATTACTT AAAAATAAAA ATATGACCAA TTGGTATCAG
```

# Figure 15: SEQ ID NO. 7

**Length: 50 bp**

1 GGAGAGAGGCAGCACTGTAAACTGAAGTCAAATAAATTCAGCTCTTAATG

# Fig. 16: Alignment of SEQ ID NO. 7 with human MAGUIN-1 cDNA

**Length: 50 bp**

```
              .         .         .         .         .
   1 GGAGAGAGGCAGCACTGTAAACTGAAGTCAAATAAATTCAGCTCTTAATG 50
     ||||||||||||||||||||||||||||||||||||||||||||||||||
5693 GGAGAGAGGCAGCACTGTAAACTGAAGTCAAATAAATTCAGCTCTTAATG 5742
              .         .         .         .         .
```

## Fig. 17: Schematic alignment of SEQ ID NO. 5, SEQ ID NO. 6 and SEQ ID NO. 7 with Genome Database EST-cluster

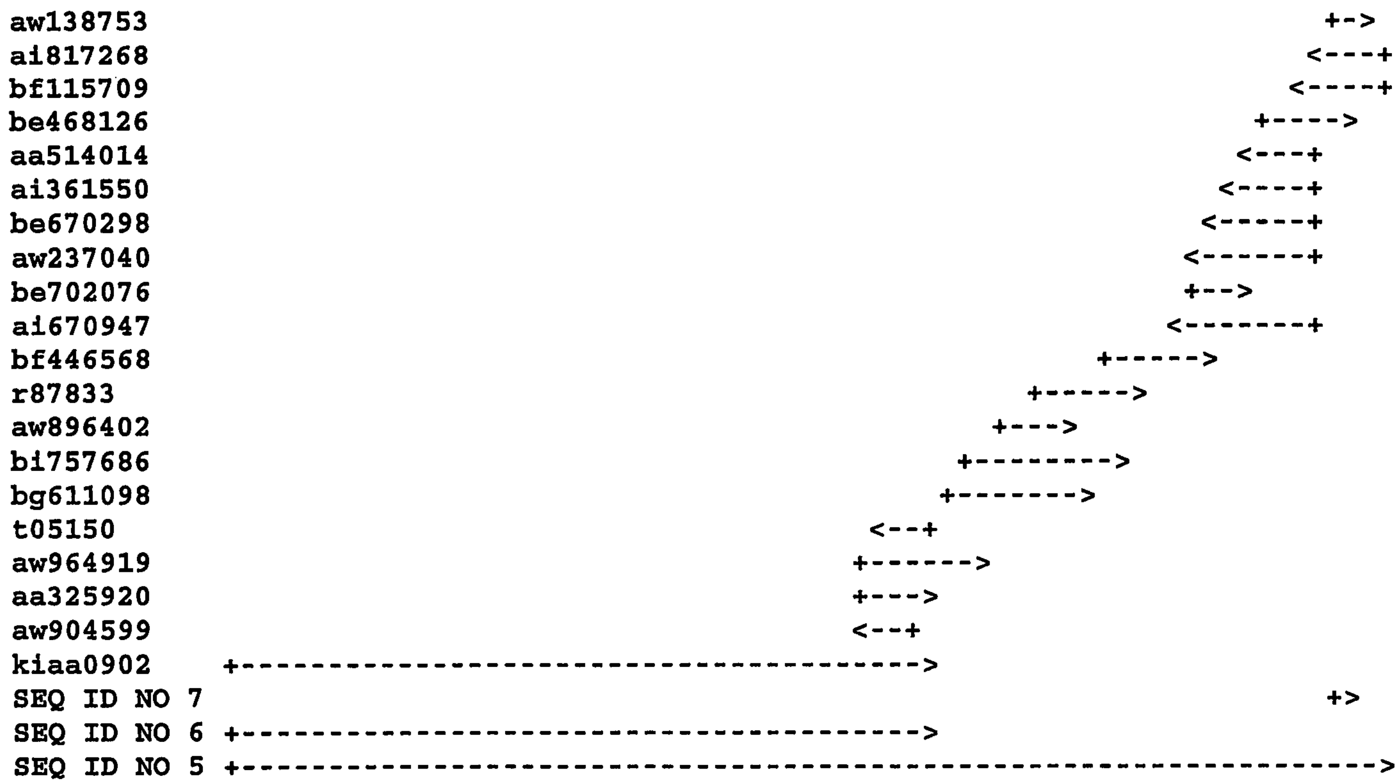

**Fig. 18: Images of the human cerebral cortex labeled with anti-Maguin-1 antiserum and with DAPI**

a b

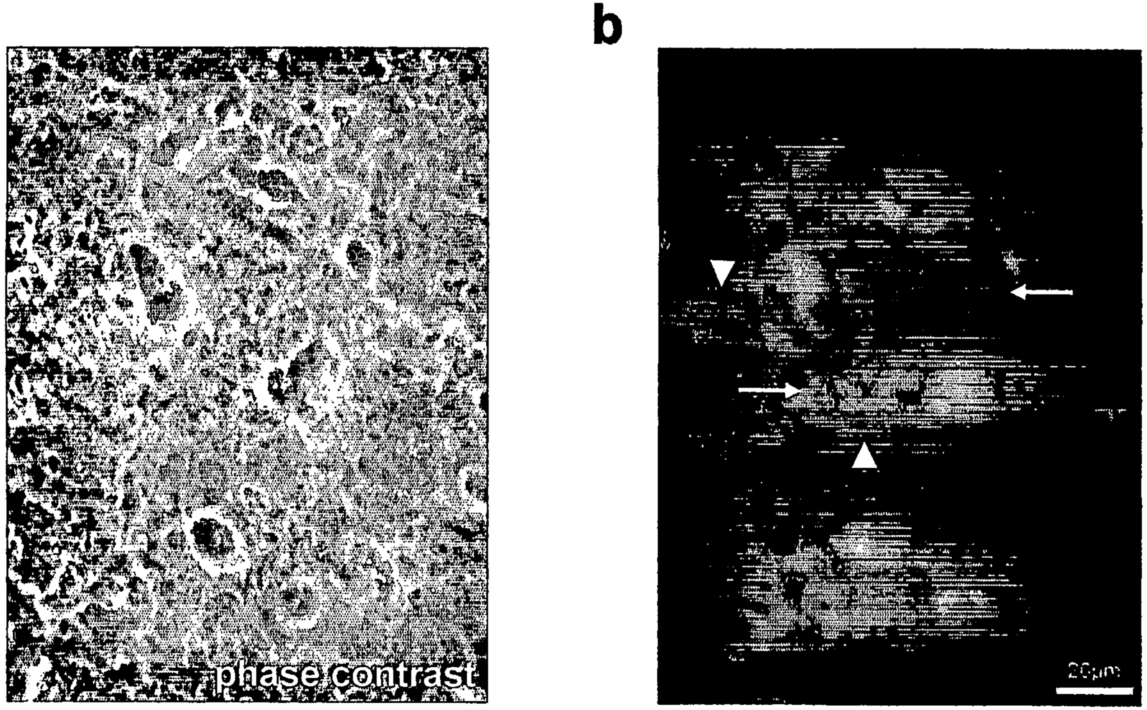

**Table 1:**

| sample | Δ (fold) (frontal / temporal cortex) |
|---|---|
| patient P012 | 2.46 |
| patient P016 | 2.78 |
| patient P010 | 4.14 |
| patient P011 | 2.20 |
| patient P014 | 1.48 |
| patient P017 | 1.42 |
| patient P019 | 1.68 |
| control C011 | 1.28 |
| control C012 | 1.29 |
| control C014 | 0.30 |
| control C005 | 1.36 |
| control C008 | 1.15 |

Table 2:

| sample | Δ (fold) (frontal cortex / hippocampus) |
|---|---|
| patient P012 | 1.37 |
| patient P016 | 3.07 |
| patient P010 | 2.99 |
| patient P011 | 2.28 |
| patient P014 | 1.21 |
| patient P019 | 1.48 |
| control C005 | 1.74 |
| control C008 | 0.39 |
| control C004 | 0.87 |

**Table 3:**

| sample | Δ (fold) (frontal / temporal cortex) |
|---|---|
| patient P012 | 2.68 |
| patient P016 | 2.72 |
| patient P010 | 11.73 |
| patient P011 | 2.44 |
| patient P014 | 1.77 |
| patient P017 | 3.43 |
| patient P019 | 4.02 |
| control C011 | 1.42 |
| control C012 | 1.22 |
| control C014 | 0.30 |
| control C005 | 0.92 |
| control C008 | 0.81 |

**Table 4:**

| sample | Δ (fold) (frontal cortex / hippocampus) |
|---|---|
| patient P012 | 1.57 |
| patient P016 | 4.38 |
| patient P010 | 9.08 |
| patient P011 | 4.53 |
| patient P014 | 0.72 |
| patient P019 | 1.37 |
| control C005 | 1.84 |
| control C008 | 0.46 |
| control C004 | 1.69 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0214543 A
- US 6150173 A


### Non-patent literature cited in the description


- **VICKERS et al.** *Progress in Neurobiology,* 2000, vol. 60, 139-165
- *Physiological Rev,* 2001, vol. 81, 741-66
- **GREENFIELD et al.** *Frontiers Bioscience,* 2000, vol. 5, D72-83
- **BRAAK ; BRAAK.** *Acta Neuropathol,* 1991, vol. 82, 239-259
- **JOHNSON ; JENKINS.** *J Alzheimers Dis,* 1996, vol. 1, 38-58
- **JOHNSON ; HARTIGAN.** *J Alzheimers Dis,* 1999, vol. 1, 329-351
- **SCHMITT et al.** *Neurology,* 2000, vol. 55, 370-376
- **TERRY et al.** *Annals of Neurology,* 1981, vol. 10, 184-92
- **STRITTMATTER et al.** *Proc Natl Acad Sci USA,* 1993, vol. 90, 1977-81
- **ROSES.** *Ann NY Acad Sci,* 1998, vol. 855, 738-43
- **MYERS et al.** *Science,* 2000, vol. 290, 2304-5
- **BERTRAM et al.** *Science,* 2000, vol. 290, 2303
- **SCOTT et al.** *Am J Hum Genet,* 2000, vol. 66, 922-32
- **TESSIER-LAVIGNE ; GOODMAN.** *Science,* 1996, vol. 274, 1123-1133
- **POIRAZI ; MEL.** *Neuron,* 2001, vol. 29, 779-796
- **SHENG ; KIM.** *Current Opinion Neurobiology,* 1996, vol. 6, 602-608
- **KIM ; HUGANIR.** *Current Opinion Cell Biology,* 1999, vol. 11, 248-254
- **HIRAO et al.** *Journal of Biological Chemistry,* 1998, vol. 273, 21105-21110
- **HIRAO et al.** *Journal of Biological Chemistry,* 2000, vol. 275, 2966-2972
- **KIM et al.** *Neuron,* 1996, vol. 17, 103-113
- **YAO et al.** *Journal of Biological Chemistry,* 1999, vol. 274, 11889-11896
- **SCHULTZ et al.** *Protein Science,* 1997, vol. 6, 249-253
- **DOYLE et al.** *Cell,* 1996, vol. 85, 1067-1076
- **LEMMON et al.** *Trends Cell Biology,* 1997, vol. 7, 237-242
- **THERRIEN et al.** *Cell,* 1998, vol. 95, 343-353
- **IQBAL ; SWAAB ; WINBLAD ; WISNIEWSKI.** Alzheimer's Disease and Related Disorders (Etiology, Pathogenesis and Therapeutics. Wiley & Sons, 1999
- **SCINTO ; DAFFNER.** Early Diagnosis of Alzheimer's Disease. Humana Press, 2000
- **MAYEUX ; CHRISTEN.** Epidemiology of Alzheimer's Disease: From Gene to Prevention. Springer Press, 1999
- **YOUNKIN ; TANZI ; CHRISTEN.** Presenilins and Alzheimer's Disease. Springer Press, 1998
- **SAMBROOK ; RUSSELL.** Molecular Cloning: A Laboratory Manual. Cold Spring Harbor Laboratory Press, 2001
- **SCHENA M.** Microarray Biochip Technology. Eaton Publishing, 2000
- **HARLOW ; LANE.** Using Antibodies: A Laboratory Manual. Cold Spring Harbor Laboratory Press, 1999
- **EDWARDS R.** Immunodiagnostics: A Practical Approach. Oxford University Press, 1999
- **CAPECCHI.** *Science,* 1989, vol. 244, 1288-1292
- **HOGAN et al.** Manipulating the Mouse Embryo: A Laboratory Manual. Cold Spring Harbor Laboratory Press, 1994
- **JACKSON ; ABBOTT.** Mouse Genetics and Transgenics: A Practical Approach. Oxford University Press, 1999
- **HARLOW et al.** Antibodies, A Laboratory Manual. Cold Spring Harbor Laboratory Press, 1988
- **DUBEL ; BREITLING.** Recombinant Antibodies. Wiley-Liss, 1999
- **EDWARDS R.** Immunodiagnostics: A Practical Approach. Oxford University Press, 1999
- **TERRY et al.** *Annals of Neurology,* 1981, vol. 10, 184-192
- **STRANGE.** Brain Biochemistry and Brain Disorders. Oxford University Press, 1992, 4
- **LIANG ; PARDEE.** *Science,* 1995, vol. 267, 1186-7
- **KAMMER et al.** *Nucleic Acids Research,* 1999, vol. 27, 2211-2218

- **LIANG et al.** *Nucleic Acids Research,* 1994, vol. 22, 5763-5764
- **ZHAO et al.** *Biotechniques,* 1995, vol. 18, 842-850